(19) Europäisches Patentamt
European Patent Office
Office européen des brevets

(11) EP 0 400 562 B1

(12) **EUROPEAN PATENT SPECIFICATION**

(45) Date of publication and mention of the grant of the patent: **21.08.1996 Bulletin 1996/34**

(51) Int. Cl.$^6$: **C07D 487/04**, A61K 31/505 // (C07D487/04, 239:00, 209:00)

(21) Application number: **90110131.1**

(22) Date of filing: **29.05.1990**

(54) **Pyrrolopyrimidines, their production and use as tumoricidal agents**

Pyrrolopyrimidine, ihre Herstellung und Verwendung als tumorizide Wirkstoffe

Pyrrolopyrimidines, leur préparation et leur utilisation en tant qu'agents antitumoraux

(84) Designated Contracting States:
**AT BE CH DE DK ES FR GB GR IT LI LU NL SE**

(30) Priority: **29.05.1989 JP 135642/89**
**20.09.1989 JP 246209/89**
**09.04.1990 JP 93370/90**

(43) Date of publication of application:
**05.12.1990 Bulletin 1990/49**

(73) Proprietor: **Takeda Chemical Industries, Ltd.**
**Chuo-ku, Osaka (JP)**

(72) Inventors:
- **Akimoto, Hiroshi**
  **Higashinada-ku, Kobe, Hyogo 658 (JP)**
- **Hitaka, Takenori**
  **Takarazuka, Hyogo 665 (JP)**
- **Miwa, Tetsuo**
  **Nada-ku, Kobe, Hyogo 657 (JP)**

(74) Representative: **Lederer, Franz, Dr. et al**
**Lederer, Keller & Riederer**
**Patentanwälte**
**Prinzregentenstrasse 16**
**80538 München (DE)**

(56) References cited:
**EP-A- 0 075 880** **EP-A- 0 075 881**
**EP-A- 0 255 228** **EP-A- 0 334 636**
**US-A- 4 532 241** **US-A- 4 684 653**

- **JOURNAL OF MEDICINAL CHEMISTRY, vol. 28, 1985, pp. 914-921; E.C. TAYLOR et al.: "Synthesis of the Antileukemic Agents 5,10-Dideazaaminopterin and 5,10-Dideaza-5,6,7,8-tetrahydroaminopterin"**

Remarks:
The file contains technical information submitted after the application was filed and not included in this specification

EP 0 400 562 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Rank Xerox (UK) Business Services
2.13.2/3.4

## Description

BACKGROUND OF THE INVENTION

1. Field of the Invention

This invention relates to novel pyrrolopyrimidine derivatives which are useful as antitumor agents.

2. Prior Arts

Folic acid is a carrier of a C1 unit in a living body, derived from formic acid or formaldehyde, acting as a coenzyme in various enzymatic reactions such as those in biosynthesis of nucleic acid, in metabolism of amino acids and peptides and in generation of methane. Particularly in biosynthesis of nucleic acid, folic acid is essential for formylation in the two pathways, i.e. the purine synthetic pathway and the thymidine synthetic pathway. Usually folic acid is required to be transformed into its activated coenzyme form by reduction in two steps before it becomes biologically active.

Amethopterin (methotrexate; MTX) and the related compounds are known to inhibit the reduction from dihydrofolic acid into tetrahydrofolic acid by coupling strongly with the dominant enzyme in the second step (dihydrofolic acid reductase). These drugs have been developed as antitumor drugs because they may disturb the DNA synthesis and consequently cause cell death, and are currently regarded of major clinical importance.

On the other hand, a novel tetrahydroaminopterin antitumor agent (5,10-dideaza-5,6,7,8-tetrahydroaminopterin: DDATHF) has been reported which, unlike the drugs described above, does not inhibit dihydrofolic acid reductase and the main mechanism of which consists in inhibition of glycinamide ribonucleotide transformylase required in the initial stage of purine biosynthesis [Journal of Medicinal Chemistry, 28, 914(1985)].

With regard to the treatment of cancer, it is now strongly expected to develop a new drug which possesses an excellent effect based on a novel mechanism and exhibits a highly selective toxicity against cancer cells. An antitumor agent which mainly antagonizes folic acid, namely, MTX is now widely used in a clinical field, but it is not sufficient due to relatively high toxicity and insufficient effect on solid tumors. And further, increase of resistance against this type of drug is a big problem.

Thus, some new series of compounds as antitumor agents have been proposed [see European Patent Application No. 0 334 636 (pyrrolopyrimidine derivatives), United States Patent Nos. 4,532,241 and 4,684,653, and European Patent Application Nos. 255 228 and 075 880 (Pyridopyrimidine derivatives)]. EP-A-075 881 reports 7-deazapurine derivatives which are selectively taken up by tumor cells.

SUMMARY OF THE INVENTION

As the result of the inventor's study under the circumstances described above, they found that pyrrolo[2,3-d]pyrimidine derivatives, which are not pteridine compounds, exhibit a highly selective toxicity against a variety of tumor cells and also possess an excellent antitumor activity on MTX resistant cells, and completed the present invention.

That is, this invention relates to

(1) a compound of the formula (I)

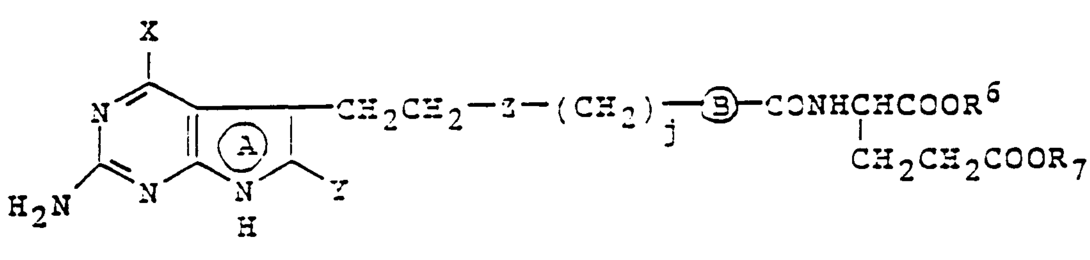

(I)

wherein the ring Ⓐ is a pyrrole ring which may be hydrogenated, X is an amino, hydroxyl or mercapto group, Y is a hydrogen atom or a hydroxyl group, Z is -O-, -S- or

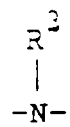

in which $R^3$ is a hydrogen atom, $C_{1-3}$ alkyl group or $C_{2-5}$ alkoxycarbonyl group, -Ⓑ- is phenyl-1,4-ylene, thiazol-2,5-ylene or thiophen-2,5-ylene group, $-COOR^6$ and $-COOR^7$ may be the same or different and are each a carboxyl group which may be esterified, wherein $R^6$ and $R^7$ are the same or different and are each hydrogen, an alkyl group with 1 to 5 carbon atoms or an optionally substituted benzyl or phenyl group and j is 0 or 1, or its salt;

(2) A process for preparing a compound of the formula (I), which comprises reacting a compound of the formula (II)

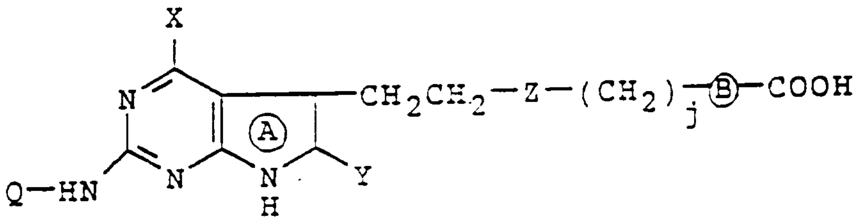 (II)

wherein each of the ring Ⓐ, X, Y, Z, -Ⓑ- and j has the same meaning as above, and Q is a hydrogen atom or an amino protecting group, or its salt or reactive derivative at the carboxyl group, with a compound of the general formula (III)

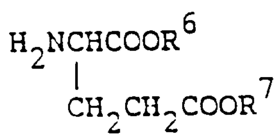 (III)

wherein each of $-COOR^6$ and $-COOR^7$ has the same meaning as above, or its salt and if necessary, subjecting the resultant product to removal reaction of the protecting group;

(3) A process for preparing compound of the formula (I), which comprises reacting a compound of the formula (IV)

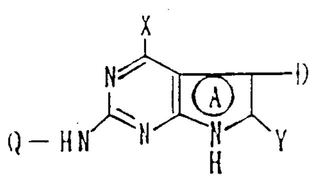 (IV)

wherein each of the ring Ⓐ, X, Y and Q has the same meaning as above, D is a group of the formula $-CH_2CH_2-L$ in which L is a leaving group, a group of the formula
$-CH_2CH_2-ZH$ in which Z has the same meaning as above, a group of the formula

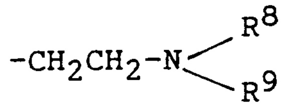

in which $R^8$ and $R^9$ may be the same or different and are each a hydrogen atom or a hydrocarbon radical optionally having substituent(s) or are combined to form a cyclic amino group together with the adjacent nitrogen atom, or a group of the formula $-CH_2CHO$, or its salt, with a compound of the general formula (V)

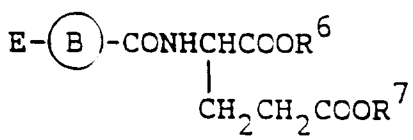
(V)

wherein each of Ⓑ, $-COOR^6$ and $-COOR^7$ has the same meaning as above, E is a group of the formula $L-(CH_2)_{m'}-$ in which L has the same meaning as above, and m' is an integer of 0 or 1, a group of the formula OHC- or a group of the formula $HZ-(CH_2)_{m'}-$ in which Z and m' have the same meaning as above, or its salt and then subjecting the resultant product to removal reaction of the protecting group, if necessary;

(4) a compound of the formula (VI)

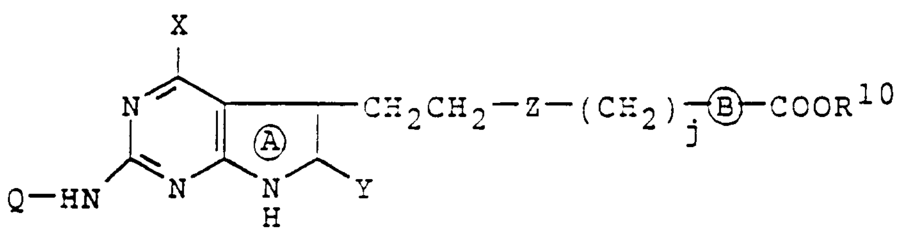
(VI)

wherein each of the ring Ⓐ, X, Y, Q, Z, -Ⓑ- and j has the same meaning as above, and $-COOR^{10}$ is a carboxyl group which may be esterified, wherein $R^{10}$ has the same meaning as $R^6$ and $R^7$, respectively, as defined above, or its salt; and

(5) an antitumor agent containing the compound (I) or its salt.

Each of the above compounds (I), (II), (IV) and (VI) where X is a hydroxyl or mercapto group and Y is a hydroxyl group may exist as an equilibrium mixture of the respective tautomers. The following partial structural formulas show the sites of the structures which are subject to tautomerism, and the equilibrium between the tautomers is illustrated.

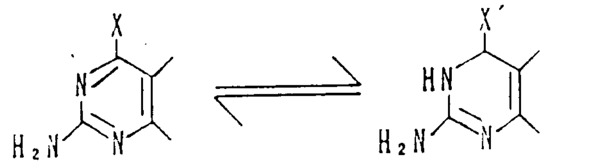

X = OH, SH        X' = O, S

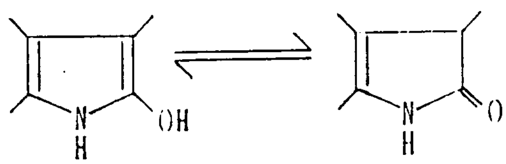

For the convenience of description, only the hydroxyl or mercapto forms, and the corresponding nomenclatures are stated throughout this specification, but it should be noted that their tautomer, namely, the oxo compound and thioxo compound are also included in the scope of this invention.

There may be two or more asymmetric centers in the compounds (I) of this invention, and the absolute configuration at all of the asymmetric carbon atoms may be the S, R or SR mixed form, except that the absolute configuration at the asymmetric carbon atom in the side chain derived from glutamic acid is always S(L). Therefore the compounds (I) may have two or more diastereomers which, if necessary, can easily be separated from each other by a routine method for separation and purification. All of the diastereomers which can be separated by such a method are included in this invention.

In the above formulas, the pyrrole ring which may be hydrogenated for the ring Ⓐ may be a pyrrole or pyrroline ring.

$R^3$ may be a $C_{1-3}$ alkyl group (e.g. methyl, ethyl, propyl or iso-propyl) or an alkoxycarbonyl having 2 to 5 carbon atoms (e.g. methoxycarbonyl, ethoxycarbonyl, n-propoxycarbonyl, iso-propoxycarbonyl, n-butoxycarbonyl, iso-butoxycarbonyl or tert-butoxycarbonyl group).

Preferable examples of Z include

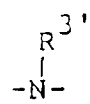

($R^{3'}$ is a $C_{1-3}$ alkyl group such as methyl, ethyl or propyl group).

The carboxyl group which may be esterified for $-COOR^6$, $-COOR^7$ and $-COOR^{10}$ may be carboxyl groups esterified by a $C_{1-5}$lower alkyl group, a benzyl group which may be substituted or a phenyl group which may be substituted.

The above lower alkyl group may be methyl, ethyl, propyl, iso-propyl, n-butyl, isobutyl, sec-butyl, tert-butyl, n-pentyl, iso-pentyl, sec-pentyl, neo-pentyl or tert-pentyl. The substituent on the above benzyl group which may be substituted or on the above phenyl group which may be substituted may be, for example nitro or a $C_{1-3}$ alkoxy (e.g., methoxy, ethoxy, propoxy or iso-propoxy). Therefore, the benzyl group which may be substituted is, for example, benzyl, nitrobenzyl or methoxybenzyl, and the phenyl group which may be substituted is, for example, phenyl, nitrophenyl or methoxyphenyl. Preferably, $-COOR^6$ and $-COOR^7$ are carboxyl groups. Preferably, $-COOR^{10}$ is a carboxyl group which may be esterified by a $C_{1-5}$ lower alkyl or benzyl.

The hydrocarbon radical in the hydrocarbon radical optionally having substituent(s) represented by $R^8$ and $R^9$ may be, for example, a $C_{1-18}$ alkyl group (e.g., methyl, ethyl, propyl, iso-propyl, butyl, iso-butyl, sec-butyl, tert-butyl, pentyl, iso-pentyl, hexyl, iso-hexyl, heptyl, octyl, nonyl, decyl, undecyl, dodecyl, tetradecyl, hexadecyl, octadecyl, 1,2-dimethylpropyl, 1-ethylpropyl, 1,2,2-trimethylpropyl, 1-propylbutyl or 2-ethylhexyl group), a $C_{2-12}$ alkenyl group (e.g., vinyl, allyl, 1-methylvinyl, 2-methylvinyl, 1-octenyl or 1-decenyl group), a $C_{3-12}$ cycloalkyl group (e.g., cyclopropyl, cyclobutyl, cyclopentyl, cyclohexyl, cycloheptyl, cyclooctyl or adamantyl group), a $C_{3-8}$ cycloalkenyl group (e.g., cyclopentenyl, cyclohexenyl, cycloheptenyl, cyclooctenyl, cyclopentadienyl, cyclohexadienyl, cycloheptadienyl or cyclooctadienyl group), a $C_{7-13}$ aralkyl group (e.g., benzyl, α-methylbenzyl, phenethyl or diphenylmethyl group) or a $C_{6-10}$ aryl group (e.g., phenyl, α-naphthyl or β-naphthyl group).

The hydrocarbon radical optionally having substituents represented by $R^8$ and $R^9$ may form a cyclic amino group with the adjacent nitrogen atom. The cyclic amino group formed by $R^8$ and $R^9$ with the adjacent nitrogen atom may be preferably a 4 to 10-membered ring such as azetidinyl, pyrrolidinyl, pyrrolinyl, pyrrolyl, imidazolyl, pyrazolyl, imidazolinyl, piperidino, morpholino, dihydropyridyl, tetrahydropyridyl, N-methylpiperazinyl, N-ethylpiperazinyl, azacycloheptyl, azacyclooctyl, isoindolyl, indolyl, indolinyl, 2-isoindolinyl, azacyclononyl or azacyclodecyl.

These hydrocarbon radical represented by $R^8$ and $R^9$ and the ring formed by $R^8$ and $R^9$ with the adjacent nitrogen atom may have one or two sustituents. These substituents may be, for example, a $C_{1-4}$ alkyl group (e.g., methyl, ethyl, propyl, iso-propyl, butyl, iso-butyl, sec-butyl or tert-butyl group), a $C_{1-4}$ alkoxy group (e.g., methoxy, ethoxy, propoxy, isopropoxy, n-butoxy, iso-butoxy, sec-butoxy or tert-butoxy group), a $C_{1-4}$ alkanoyl group (e.g., formyl, acetyl, propionyl, n-butyryl or iso-butyryl group), a $C_{1-4}$ alkanoyloxy group (e.g., formyloxy, acetyloxy, propionyloxy, n-butyryloxy, iso-butyryloxy group), carboxyl group, a $C_{2-4}$ alkoxycarbonyl group (e.g., methoxycarbonyl, ethoxycarbonyl, n-propoxycarbonyl, iso-propoxycarbonyl, n-butoxycarbonyl, iso-butoxycarbonyl or tert-butoxycarbonyl group), a halogen atom (e.g., fluorine, chlorine, bromine or iodine), hydroxyl group, nitro group, cyano group, trifluoromethyl group, amino group, mono-substituted amino group (e.g., methylamino, ethylamino, propylamino, iso-propylamino or butylamino group), di-substituted amino group(e.g., dimethylamino, diethylamino, dipropylamino, diisopropylamino or dibutylamino group), an alkanoylamido group(e.g., formamido, acetamido, trifluoroacetamido, propionylamido, butyrylamido or isobutyrylamido group), carbamoyl group, a N-substituted carbamoyl group (e.g., N-methylcarbamoyl, N-ethylcarbamoyl, N-propylcarbamoyl, N-isopropylcarbamoyl or N-butylcarbamoyl group), a N,N-disubstituted carbamoyl group (e.g., N,N-dimethylcarbamoyl, N,N-diethylcarbamoyl, N,N-dipropylcarbamoyl, N,N-dibutylcarbamoyl, 1-aziridinylcarbonyl, 1-azetidinylcarbonyl, 1-pyrrolidinylcarbonyl, 1-piperidinylcarbonyl, N-methylpiperazinylcarbonyl or morpholinocarbonyl group), carbamoylamino group, a N-substituted carbamoylamino group (e.g., N-methylcarbamoylamino, N-ethylcarbamoylamino, N-propylcarbamoylamino, N-isopropylcarbamoylamino or N-butylcarbamoylamino group), a N,N-disubstituted carbamoylamino group (e.g., N,N-dimethylcarbamoylamino, N,N-diethylcarbamoylamino, N,N-dipropylcarbamoylamino, N,N-dibutylcarbamoylamino, 1-aziridinylcarbonylamino, 1-azetidinylcarbonylamino, 1-pyrrolidinylcarbonylamino, 1-piperidinylcarbonylamino, N-methylpiperazinylcarbonylamino or morpholinocarbonylamino group), mercapto group, sulfo group, sulfino group, phosphono group, sulfamoyl group, a N-substituted sulfamoyl group (e.g., N-methylsulfamoyl, N-propylsulfamoyl, N-isopropylsulfamoyl or N-butylsulfamoyl group), a N,N-disubsituted sulfamoyl group e.g., N,N-dimethylsulfamoyl, N,N-diethylsulfamoyl, N,N-dipropylsulfamoyl, N,N-dibutylsulfamoyl, N,N-dipropylsulfamoyl, N,N-dibutylsulfamoyl, 1-pyrrolidinylsulfonyl, 1-piperidinylsulfonyl, N-methyl-1-piperazinylsulfonyl or morpholinosulfonyl group), a $C_{1-4}$ alkylthio group (e.g., methylthio, ethylthio, propylthio, isopropylthio, n-butylthio, sec-butylthio or tert-butylthio group), a $C_{1-4}$ alkylsulfinyl group (e.g., methylsulfinyl, ethylsulfinyl, propylsulfinyl or butylsulfinyl group), a $C_{1-4}$ alkylsulfonyl group (e.g., methylsulfonyl, ethylsulfonyl, propylsulfonyl or butylsulfonyl group), or the like.

Preferable examples of -Ⓑ- are phenyl-1,4-ylene or thiophen-2,5-ylene.

The amino protective group represented by Q may be, for example, a $C_{1-18}$ alkanoyl group (e.g., formyl, acetyl, propionyl, butyryl, isobutyryl, valeryl, isovaleryl, pivaloyl, hexanoyl, heptanoyl, octanoyl, 2-ethylhexanoyl, nonanoyl, decanoyl, undecanoyl, tridecanoyl, tetradecanoyl, pentadecanoyl, hexadecanoyl, heptadecanoyl or octadecanoyl group), a $C_{7-12}$ aroyl group (e.g., benzoyl, toluoyl, naphthoyl, phenylacetyl or cinnamoyl group), among which a $C_{1-10}$ alkanoyl group or benzoyl group is preferable.

The leaving group represented by L may be, for example, a halogen atom (chlorine, bromine or iodine atom), methanesulfonyloxy group, benzenesulfonyloxy group, p-toluenesulfonyloxy group, trifluoromethanesulfonyloxy group, a lower ($C_1$ - $C_6$) alkanoyloxy group, a N,N-disubstituted amino group or a N,N,N-trisubstituted ammonium group. The N,N-disubstituted amino group may be, for example, an amino group substituted by the groups exemplified for $R^8$ and $R^9$, and the N,N,N-trisubstituted ammonium group may be, for example, a quaternary salt of the above N,N-disubstituted amino group (e.g., quaternary salt by methyl bromide, methyl iodide, methyl methanesulfonate, methyl benzenesulfonate, methyl p-toluenesulfonate, etc.)

Preferably, X is amino group or hydroxyl group.

Preferable groups of the compounds (I) or their salt are the compounds represented by the following formula or their salts.

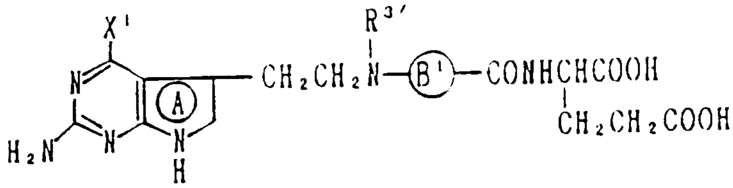

wherein $X^1$ is an amino or hydroxyl group, $R^{3'}$ is a $C_{1-3}$ alkyl group, -Ⓑ- is phenyl-1,4-ylene, thiazol-2,5-ylene or thiophen-2,5-ylene group and Ⓐ is the same meaning as defined above.

Further preferable examples of compound (I) include those wherein

(1) the ring Ⓐ is a pyrrole or pyrroline ring, X is an amino, hydroxyl or mercapto group, Y is a hydrogen atom or a hydroxyl group, the moiety $-(CH_2)_j-$ is a chemical bond, Z is -O-, -S- or

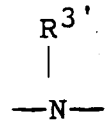

in which $R^{3'}$ is a $C_{1-3}$ alkyl group, -Ⓑ-is a phenyl-1,4-ylene or thiophen-2,5-ylene group, and $-COOR^6$ and $-COOR^7$ are a carboxyl group or a carboxyl group esterified with a $C_{1-5}$ lower alkyl group, or its salt; or

(2) the ring Ⓐ is a pyrroline ring, X is an amino group, Y is a hydrogen atom, the moiety $-(CH_2)_j-$ is a chemical bond, Z is an amino group or an amino group substituted by a $C_{1-3}$ alkyl, -Ⓑ- is a phenyl-1,4-ylene group, and $-COOR^6$ and $-COOR^7$ are a carboxyl group or an ethoxycarbonyl group, or its salt.

The methods for preparing the compounds (I) of this invention are explained in the following.

Process (1)

The compound (I) can be prepared by acylation of a glutamic acid derivative of the formula (III) or its salt, with a carboxylic acid of the formula (II) or its salt or its reactive derivative at the carboxyl group. The acylation can be carried out by reacting a compound (III) or its salt with a compound (II) or its salt in the presence of a carbodiimide, diphenylphosphoryl azide, diethyl phosphorocyanidate, or the like. The amount of the compound (III) or its salt to be used is generally about 1 - 20, preferably about 1 - 5 molar equivalents to the compound (II) or its salt. The carbodiimide is used in about 1 - 25, preferably about 1 - 5 molar equivalents, to the compound (II) or its salt.

The carbodiimide is preferably dicyclohexylcarbodiimide, and the other carbodiimides such as diphenylcarbodiimide, di-o-tolylcarbodiimide, di-p-tolylcarbodiimide, di-tert-butylcarbodiimide, 1-cyclohexyl-3-(2-morpholinoethyl)carbodiimide, 1-cyclohexyl-3-(4-diethylaminocyclohexyl)carbodiimide, 1-ethyl-3-(2-diethylaminopropyl)carbodiimide and 1-ethyl-3-(3-diethylaminopropyl)carbodiimide can also be used. This acylation is preferably conducted in the presence of a proper solvent such as water, alcohols (e.g. methanol or ethanol), ethers (e.g. dimethyl ether, diethyl ether, tetrahydrofuran, dioxane, monoglyme, diglyme), nitriles (e.g. acetonitrile), esters (e.g. ethyl acetate), halogenated hydrocarbons (e.g. dichloromethane, chloroform, carbon tetrachloride), aromatic hydrocarbons (e.g. benzene, toluene or xylene), acetone, nitromethane, pyridine, dimethylsulfoxide, dimethylformamide, hexamethylphosphoramide or sulfolane, or a proper mixture thereof. This reaction is usually carried out at a pH in the range of about pH2 - 14, preferably about pH6 - 9, at temperature in the range of about -10°C to boiling point of the solvent to be employed (to about 100°C), preferably about 0 - 50°C, in a reaction time of about 1 - 100 hours.. The pH value of the reaction mixture can be adjusted, if necessary, by using an acid (e.g., hydrochloric acid, sulfuric acid, phosphoric acid, nitric acid or acetic acid), a base (e.g., sodium methylate, sodium ethylate, sodium hydroxide, potassium hydroxide, barium hydroxide, lithium hydroxide, sodium carbonate, potassium carbonate, barium carbonate, calcium carbonate, sodium bicarbonate, trimethylamine, triethylamine, triethanolamine or pyridine) or a buffer solution (e.g., phosphate buffer solution, borate buffer solution, acetate buffer solution), or the like. This reaction can be advantageously carried out by using a catalyst for accelerating the acylation. The catalyst may be, for example, basic catalysts such as tertiary amines (e.g., aliphatic tertiary amine such as triethylamine; aromatic tertiary amines such as pyridine, α-, β- or γ-picoline, 2,6-lutidine, 4-dimethylaminopyridine, 4-(1-pyrrolidinyl)pyridine, dimethylaniline and diethylaniline) or acidic catalysts such as Lewis acids [e.g., anhydrous zinc chloride, anhydrous aluminium chloride ($AlCl_3$), anhydrous ferric chloride, titanium tetrachloride ($TiCl_4$), tin tetrachloride ($SnCl_4$), antimony pentachloride, cobalt chloride, cupric chloride or boron trifluoride etherate]. Among the above catalysts, 4-dimethylaminopyridine or 4-(1-pyrrolidinyl)pyridine is ordinarily preferable. The catalyst is used in an amount enabling to accelerate the acylation, and the amount is usually about 0.01 - 10, preferably about 0.1 - 1 molar equivalents to the compound (II) or its salt. The reactive derivatives at the carboxyl group of the carboxylic acid (II) may be, for example, the acid halides (e.g., fluoride, chloride, bromide or iodide), mixed acid anhydrides with other acids (e.g., iodoacetic acid or isobutyric acid), mixed acid anhydrides with lower mono-alkyl carbonic esters (e.g., mono-methyl carbonic ester, mono-ethyl carbonic ester, mono-propyl carbonic ester, mono-iso-propyl carbonic ester, mono-butyl carbonic ester, mono-iso-butyl carbonic ester or mono-sec-butyl carbonic ester, mono-tert-butyl carbonic ester), active esters ( e.g., cyanomethyl ester, carbethoxymethyl ester, methoxymethyl ester, phenyl ester, nitrophenyl ester, o- or p-nitrophenyl ester, p-carbomethoxyphenyl ester, p-cyanophenyl ester or thiophenyl ester), acid azide, mixed acid anhydrides with phosphoric diesters (e.g., dimethyl phosphate, diethyl phosphate, dibenzyl phosphate or diphenyl phosphate), mixed acid anhydrides with phosphorous diesters (e.g., dimethyl phosphite, diethyl phosphite, dibenzylphosphite or diphenylphosphite) and the like. In the acylation using the above reactive derivative, the solvent, catalyst and reaction temperature may be similar to those of the aforementioned acylation conducted in the presence of the carbodiimide.

Process (2)

The compound (I) can be prepared by reacting a pyrrolo[2,3-d]pyrimidine derivative of the formula (IV) or its salt with a compound of the formula (V) or its salt, according to which a covalent bond is formed. If necessary, the amino group at the $C_2$ position of the compound (IV) where Q is hydrogen atom is previously protected, and thus protected compound (IV) or its salt is reacted with a compound (V) or its salt, and then the protective group is removed to give a compound (I) or its salt of the present invention. The method for introducing a protective group and the method for removing the protective group can be carried out by a known method described in, for example, [J.F.W. McOmie, Protective Groups in Organic Chemistry, Plenum Press, London and New York (1973)] or analogous methods thereto.

In case where the group D of the compound (IV) is $-CH_2CH_2-L$ and the group E of the compound (V) is $HZ-(CH_2)_{m'}-$, or the group D of the compound (IV) is $-CH_2CH_2-ZH$ and the group E of the compound (V) is $L-(CH_2)_{m'}-$, so-called alkylation-type reaction is applied for forming the covalent bond between the compound (IV) or its salt and the compound (V) or its salt. In case where the group D of the compound (IV) is

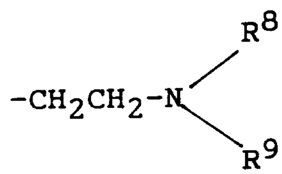

and the group E of the compound (V) is

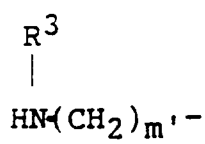

where $R^3$=H or a $C_{1-3}$ alkyl group, so-called amine-exchange-type reaction (Gramine decomposition type reaction) is advantageously applied. And further, in case where the group D of the compound (IV) is

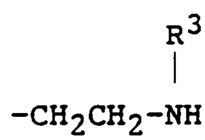

where $R^3$ is a hydrogen atom or a $C_{1-3}$ alkyl group and the group E of the compound (V) is OHC-, or the group D of the compound (IV) is $-CH_2-CHO$ and the group E of the compound (V) is

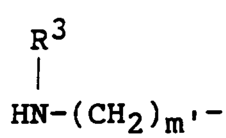

($R^3$ is the same meaning as defined above), a method where Schiff's base is formed and it is reduced if necessary, or a method where the compounds are directly subjected to reductive alkylation is applied. The above-mentioned alkylation-type reaction and amine-exchange-type reaction can be carried out by reacting a compound (IV) or its salt with a compound (V) or its salt without any solvent or in a proper solvent, at a temperature of about -10°C to the boiling point of the solvent to be employed preferably about 10 - 80°C, for 10 minutes to 48 hours. The compound (V) or its salt is used in about 1 - 50 moles, preferably about 1 - 10 moles, to one mole of the compound (IV) or its salt. Examples of the solvents include water, alcohols (e.g., methanol, ethanol, propanol, iso-propanol, butyl alcohol, sec-butyl alcohol, tert-butyl alcohol, ethylene glycol, methoxyethanol or ethoxyethanol), ethers (e.g., diethyl ether, tetrahydrofuran, dioxane, monoglyme or diglyme), halogenated hydrocarbons (e.g., dichloromethane, chloroform or carbon tetrachloride), nitriles (e.g., acetonitrile), aliphatic hydrocarbons (e.g., pentane, hexane, heptane or octane), cyclic aliphatic hydrocarbons (e.g., cyclopentane or cyclohexane), aromatic hydrocarbons (e.g., benzene, toluene, xylene), nitromethane, pyridine, dimethylformamide, dimethylsulfoxide, hexamethylphosphoramide or sulfolane, or a proper mixture thereof. The reaction is occasionally carried out in the presence of a base to give a preferable result. Examples of the bases to be used include metallic hydroxides such as sodium hydroxide, potassium hydroxide, lithium hydroxide, barium hydroxide, etc., metallic alkoxides such as sodium methoxide, sodium ethoxide, potassium tert-butoxide, etc., metallic hydrides such as sodium hydride, potassium hydride, etc., organic metal compounds such as phenyllithium, butyllithium, aliphatic tertiary amines such as triethylamine, etc., aromatic tertiary amines such as pyridine, α-, β- or γ-picoline 2,6-lutidine, 4-dimethylaminopyridine, 4-(1-pyrrolidinyl)pyridine, dimethylaniline, diethylaniline, etc., or the like. Further, the reaction can be advantageously carried out by using a phase-transfer catalyst (e.g., cetyltrimethyl- ammonium chloride, etc.) in an amount of 0.01 - 0.2, preferably 0.02 - 0.05 equivalents to the compound (IV) or compound (V) or its salt. The amine-exchange-type reaction is occasionally carried out under a mild condition by using a quaternary salt of the compound (IV), for example, a salt with methyl bromide, methyl iodide, methyl methanesulfonate, methyl benznensulfonate, methyl p-toluenesulfonate, etc.

The reaction to produce the above -mentioned Schiff's base is carried out by reacting a compound (IV) or its salt with a compound (V) or its salt in a molar ratio (IV)/(V) = 0.1 - 10 : 1 , without any solvent or in a proper solvent, at a temperature from -10°c to the boiling point of the solvent, preferably 0 - 50°C, for about 10 minutes - 48 hours. The compounds (IV) and (V) or their salts where the part of aldehyde or ketone is protected in an acetal or ketal form may be used in this reaction. The reaction solvent is preferably nonaqueous one, for example, alcohols (e.g., methanol, ethanol, propanol, iso-propanol, butyl alcohol, sec-butyl alcohol, tert-butyl alcohol, ethylene glycol, methoxyethanol or ethoxyethanol), ethers (e.g., dimethyl ether, diethyl ether, tetrahydrofuran, dioxane, monoglyme or diglyme), esters(e.g., methyl acetate or ethyl acetate), halogenated hydrocarbons (e.g., dichloromethane, chloroform or carbon tetrachloride), nitriles (e.g., acetonitrile), aliphatic hydrocarbons (e.g., pentane, hexane, heptane or octane ), cyclic aliphatic hydrocarbons (e.g., cyclopentane or cyclohexane), aromatic hydrocarbons (e.g., cyclopentane or cyclohexane), aromatic hydrocarbons (e.g., benzene, toluene or xylene), acetone, nitromethane, pyridine, dimethyl formamide, dimethylsulfoxide, hexamethylphosphoramide or sulfolane, or a proper mixture thereof. Improvement of the reaction speed and yield can be achieved by adding a dehydrating agent such as molecular sieve, calcium chloride, magnesium sulfate, sodium sulfate, calcium sulfate, etc., or by adjusting pH value of the reaction mixture with an acid (e.g., hydrochloric acid, hydrobromic acid, hydriodic acid, sulfuric acid, nitric acid or phosphoric acid), a base (e.g., metallic hydroxide used as sodium hydroxide, potassium hydroxide, lithium hydroxide, barium hydroxide, etc., sodium methoxide, sodium ethoxide, potassium tert-butoxide, sodium carbonate, potassium carbonate, barium carbonate, calcium carbonate, sodium bicarbonate, trimethylamine, triethylamine, triethanolamine or pyridine) or a buffer solution (e.g., phosphate buffer solution, borate buffer solution or acetate buffer solution). The reduction of the Schiff's base and the reductive alkylation can be carried out in a proper solvent and at a temperature from about -40°C to the boiling point of the solvents preferably at about 0 - 50°C, utilizing hydride reduction methods or catalytic reduction methods. Examples of the solvents to be used include water, alcohols (e.g., methanol, ethanol, propanol, iso-propanol, butyl alcohol., sec-butyl alcohol, ter-butyl alcohol, ethylene glycol, methoxyethanol or ethoxyethanol), acetic esters (e.g., methyl acetate or ethyl acetate), ethers (e.g., dimethyl ether, diethyl ether, tetrahydrofuran, dioxane, monoglyme or diglyme), aromatic hydrocarbons (e.g., benzene, toluene or xylene), pyridine or dimethylformamide, or a proper mixture thereof. The catalyst used in the catalytic reduction is, for example, palladium, platinum, rhodium, Raney nickel or the like. Further, a slight amount of acetic acid, trifluoroacetic acid, hydrochloric acid, sulfuric acid or the like may be added to the reaction mixture. The reagents for the hydride reduction are, for example, lithium aluminum hydride, sodium borohydride, lithium borohydride, lithium cyanoborohydride or the like. The amount of the reducing agent to be used is about 1- 100 moles, usually 2 - 20 moles for one mole of the compound to be reduced. The starting compounds (IV) and (V) or their salts used in the above method can be prepared by known methods.

The resultant compound (I) or its salt can be isolated from the reaction mixture by a conventional isolation method, for example, concentration, solvent extraction, chromatography, recrystallization, or the like.

Among the compounds(I) or their salts, a compound (I-1) where the groups $-COOR^6$ and $-COOR^7$ are carboxyl groups can preferably be prepared by reacting a compound (III) or its salt or a compound (V) or its salt where the groups $-COOR^6$ and $-COOR^7$ are esterified carboxyl groups with a compound (II) or its salt or a compound (IV) or its salt respectively, and then de-esterifying the resultant product by a cleavage reaction or catalytic reduction. The cleavage reaction can be carried out, for example, by a hydrolysis under basic condition (Method A), a hydrolysis under acidic

condition (Method B-1) or a cleavage reaction under acidic and nonaqueous condition (Method B-2). The base to be used in the above Method A is, for example, metallic alkoxide such as sodium methoxide, sodium ethoxide, sodium butoxide and potassium butoxide, metallic hydroxide such as sodium hydroxide, potassium hydroxide, lithium hydroxide or barium hydroxide, amines such as ammonia, triethylamine and pyridine, or the like. The acid to be used in the above method B-1 is, for example, mineral acids such as hydrochloric acid, hydrobromic acid, sulfuric acid, nitric acid, and phosphric acid, organic acids such as trifluoroacetic acid, trichloroacetic acid, methanesulfonic acid, benzenesulfonic acid, p-toluenesulfonic acid and camphor sulfonic acid, or the like. The catalyst to be used in the above Method B-2 is, for example, mineral acids such as hydrogen chloride, hydrogen bromide, perchloric acid, sulfuric acid, nitric acid and phosphoric acid, organic acids such as trifluoroacetic acid, trichloroacetic acid, methanesulfonic acid, benzenesulfonic acid, p-toluenesulfonic acid and camphor sulfonic acid, Lewis acids such as anhydrous zinc chloride, anhydrous aluminum chloride ($AlCl_3$), anhydrous ferric chloride, tin tetrachloride ($SnCl_4$), antimony pentachloride, cobalt chloride, cupric chloride and boron trifluoride etherate, or the like. The cleavage reaction is carried out in a proper solvent, at a temperature from 0°C to the boiling point of the solvent, preferably at 10 - 80°C, for 30 minutes - 2 days, respectively. The solvent used in Method A and Method B-1 is, for example, water, methanol, ethanol, propanol, butanol, ethylene glycol, methoxyethanol, ethoxyethanol, tetrahydrofuran, dioxane, monoglyme, diglyme, pyridine, dimethyl formamide, dimethylsulfoxide or sulfolane, or a proper mixture thereof, and the solvent used in the Method B-2 is, for example, ethyl acetate, dimethyl ether, diethyl ether, tetrahydrofuran, dioxane, monoglyme, diglyme, dichloromethane, chloroform, carbon tetrachloride, acetonitrile, benzene, toluene, xylene, nitromethane or pyridine, or a proper mixture thereof.

The above catalytic reduction (Method C) is carried out in a proper solvent, at a temperature from -40°C to the boiling point of the solvent, preferably at about 0 - 50°C. The solvent to be used may be water, alcohols (e.g., methanol, ethanol, propanol, iso-propanol, butyl alcohol, sec-butyl alcohol, tert-butyl alcohol, ethylene glycol, methoxyethanol or ethoxyethanol), acetic esters (e.g., methyl acetate or ethyl acetate), ethers (e.g., dimethyl ether, diethyl ether, tetrahydrofuran, dioxane, monoglyme or diglyme), aromatic hydrocarbons (e.g., benzene, toluene or xylene), pyridine or dimethylformamide, or a proper mixture thereof. The catalyst to be used in the catalytic reduction is, for example, palladium, platinum, rhodium, Raney nickel, or the like. The addition of acetic acid, trifluoroacetic acid, hydrochloric acid, sulfuric acid or the like in a slight amount may proceed the reaction advantageously.

The type of reaction for preparing the compound (I-1) or its salt is selected in accordance with the nature of the groups $-COOR^6$ and $-COOR^7$. For instance, Method A or Method B-1 is usually applied when the groups $-COOR^6$ and $-COOR^7$ are carboxyl groups esterified by methyl, ethyl, propyl, butyl, sec-butyl, phenyl or a substituted phenyl group, and Method B-2 is applied when the groups $-COOR^6$ and $-COOR^7$ are carboxyl groups esterified by iso-propyl or tert-butyl group, and Method B-1 or Method C is advantageously applied when those groups are carboxyl groups esterified by benzyl group or a substituted benzyl group. In case where the groups $-COOR^6$ and $-COOR^7$ are different each other, the above Method A, Method B-1, Method B-2 and Method C may be applied in an optional combination.

The method for preparing the starting compounds (II) and (VI) or their salts is explained in the following.

The compounds (II) and (VI) can be prepared, for example, by the following reaction steps.

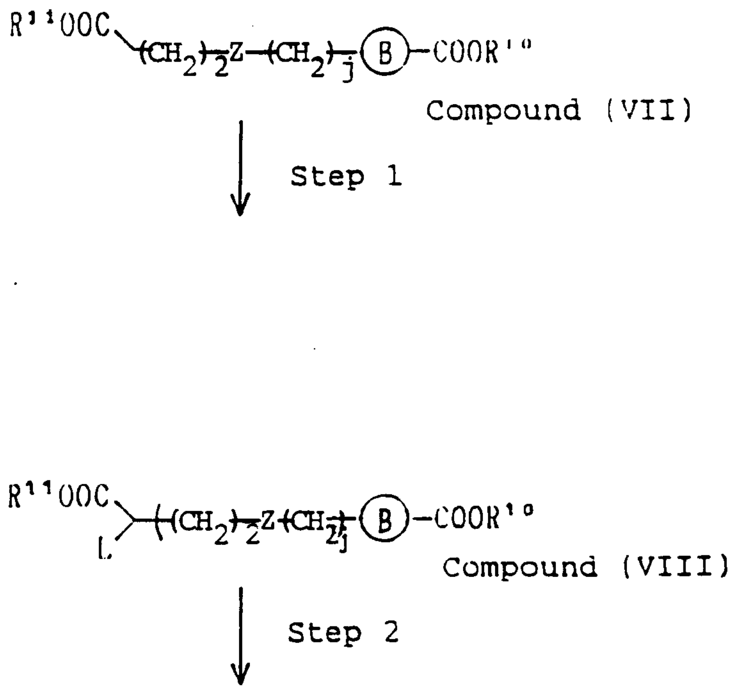

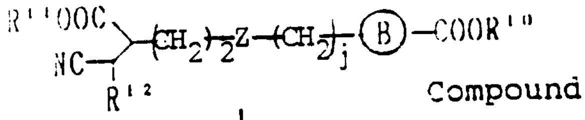

Compound (IX)

Step 3

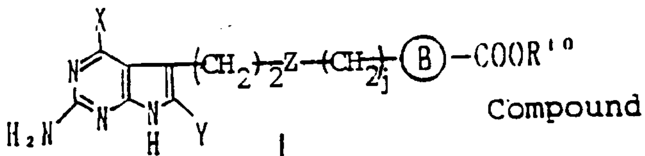

Compound (VI,Q=H)

Step 4

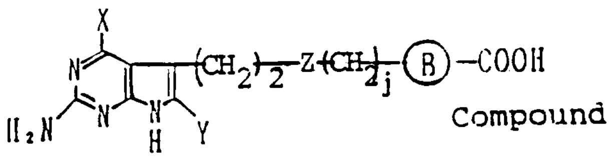

Compound (II,Q=H)

Compound (II-1;Y=OH)

Step 5

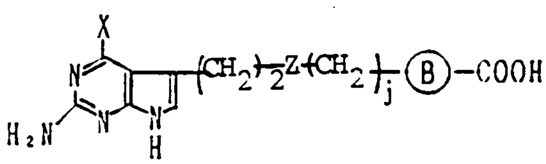

+

(II-2:Y=H)

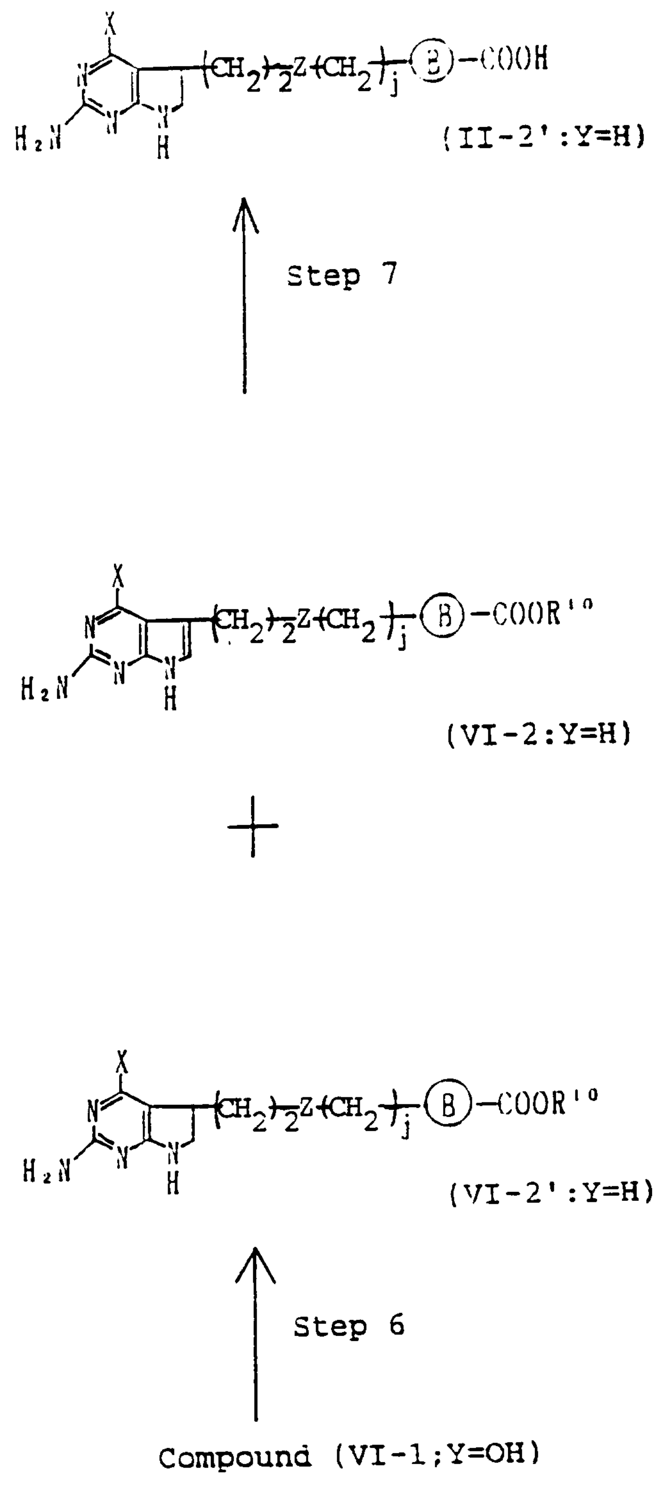


(II-2':Y=H)

(VI-2:Y=H)

(VI-2':Y=H)

Compound (VI-1;Y=OH)

In the above steps, each of X, Y, Z, Q, $R^{10}$, j and -Ⓑ- has the same meaning mentioned before, respectively. $-COOR^{11}$ is a carboxyl group which may be esterified with a group similar to those as exemplified for $-COOR^6$, $-COOR^7$ and $-COOR^{10}$.

$R^{12}$ is cyano group or a group of the formula $-COOR^{13}$, $-CSOR^{13}$ or $-CSSR^{13}$, L is a halogen atom (e.g., chlorine atom, bromine atom or iodine atom) or a removable group which can be easily derived from a hydroxyl group (e.g., methanesulfonyloxy group, benzenesulfonyloxy group, p-toluenesulfonyloxy group or trifluoromethane sulfonyloxy group). The group $R^{13}$ in the group $-COOR^{13}$, $-CSOR^{13}$ or $-CSSR^{13}$ is hydrogen atom, a $C_{1-5}$ lower alkyl group, a benzyl group optionally having substitutent(s) or a phenyl group optionally having substitutent(s).

The lower alkyl group may be, for example, methyl, ethyl, propyl, iso-propyl, n-butyl, iso-butyl, sec-pentyl, neo-pentyl, tert-pentyl, or the like. The above-mentioned substituent may be, for example, nitro, a $C_{1-3}$ alkoxy (e.g., methoxy, ethoxy, propoxy or iso-propoxy), or the like. The benzyl group optionally having substitutent(s) may be, for example, benzyl, nitrobenzyl, methoxybenzyl, or the like, and the phenyl optionally having substituent(s)may be, for example, phenyl, nitrophenyl, methoxyphenyl, or the like.

The above reaction steps are explained in detail in the following.

Step 1

This step is to introduce a removable functional group L to the active methylene part (α-position of the carboxylic ester) of the compound (VII). It can be easily carried out by a known method using a conventional reagent.

Step 2

The compound (VIII) obtained in the Step 1 is subjected under a basic condition to the condensation reaction with malononitrile, cyanoacetic ester [$NC\text{-}CH_2COOR^{13}$; $R^{13}$ is the same as above] or its thio-analogue [$NC\text{-}CH_2CSOR^{13}$, $NC\text{-}CH_2CSSR^{13}$; $R^{13}$ is the same as above] to give a compound (IX). The bases and solvents to be used and the reaction conditions may be conventional ones applied in known methods.

Step 3

The compound (IX) is treated with guanidine, where the cyano group is reacted with the ester residue or thioester residue, followed by ring closure/cyclization to form a pyrrolo[2,3-d]pyrimidine ring. The ring closure may be advantageously carried out under a basic condition. The base to be used is, for example, metallic alkoxides such as sodium methoxide, sodium ethoxide and potassium tert-butoxide. The reaction solvent is, for example, methanol, ethanol, propanol, tert-butyl alcohol, dimethylsulfoxide, hexamethylphosphramide, or the like. The reaction temperature is 0 - 150°C, preferably 20 - 100°C, and the reaction time is 1 - 48 hours.

Step 4

The compound (VI-1; Y=OH) obtained by the Step 3 can be converted to a compound(II-1 ; Y =OH) by deesterifying the ester residue [$-COOR^{10}$] in a similar manner to the preparation of the compound [I-1].

Step 5

The compound (II-1 ; Y = OH) obtained by the Step 4 is subjected to a reduction to give a compound (II-2 : Y = H). The reaction condition for the reduction may be conventional, and for instance reduction using metallic hydride (e.g., borane or alane, or its lower ($C_{1-5}$) alkyl substituted compound or an ate complex thereof) can be applied. The above Step 4 and Step 5 may be carried out in a reverse order. That is, a compound (VI-1 : Y = OH) is reduced in the Step 6 in a similar manner to the Step 5 to give a compound (VI-2 : Y = H), and the resultant product is deesterified in the Step 7 in a similar manner to the Step 4 to give a compound (II-2 : Y = H). The order of the deesterification and reduction may be selected in accordance with the nature of the substituent of the compound (VI-1 : Y = OH). In the above Step 5 and Step 6, the compounds (II-2) and (II-2') or compounds (VI-2) and (VI-2') are produced respectively, and these compounds may be isolated by a conventional method, or each of the compounds (II-2) and (II-2') or compounds (VI-2) and (VI-2') may be prepared by selective reduction.

In case where the group Z of the compound (II-2) and (VI-2) is

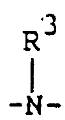

and $R^3$ is a hydrogen atom, the group -NH- may be occasionally condensed with α-position of the pyrrole ring to form a tricyclic compound (pyrrolo[3', 2' : 4, 4]pyrrolo[2,3-d]pyrimidine compound ), which can be easily converted to a bicyclic compound (II-2) and (VI-2) by treating with an acid or base.

The base to be used may be metallic hydoxides (e.g., sodium hydroxide, potassium hydroxide, lithium hydroxide or barium hydroxide), metallic alkoxides (e.g., sodium methoxide, sodium ethoxide or potassium tert-butoxide) or the like, and the acid to be used may be mineral acid (e.g., hydrochloric acid, hydrobromic acid, hydriodic acid, sulfuric acid,

nitric acid, phosphoric acid or boric acid), organic acids (e.g., oxalic acid, tartaric acid, lactic acid, citric acid, acetic acid, trifluoroacetic acid, methanesulfonic acid, ethanesulfonic acid, benzenesulfonic acid, p-toluenesulfonic acid or camphor sulfonic acid), or the like.

Among the compounds (I) of this invention and the starting compounds (VI) or their salts, a compound where Y is hydrogen atom or its salt can be prepared, for example, by the following reaction steps.

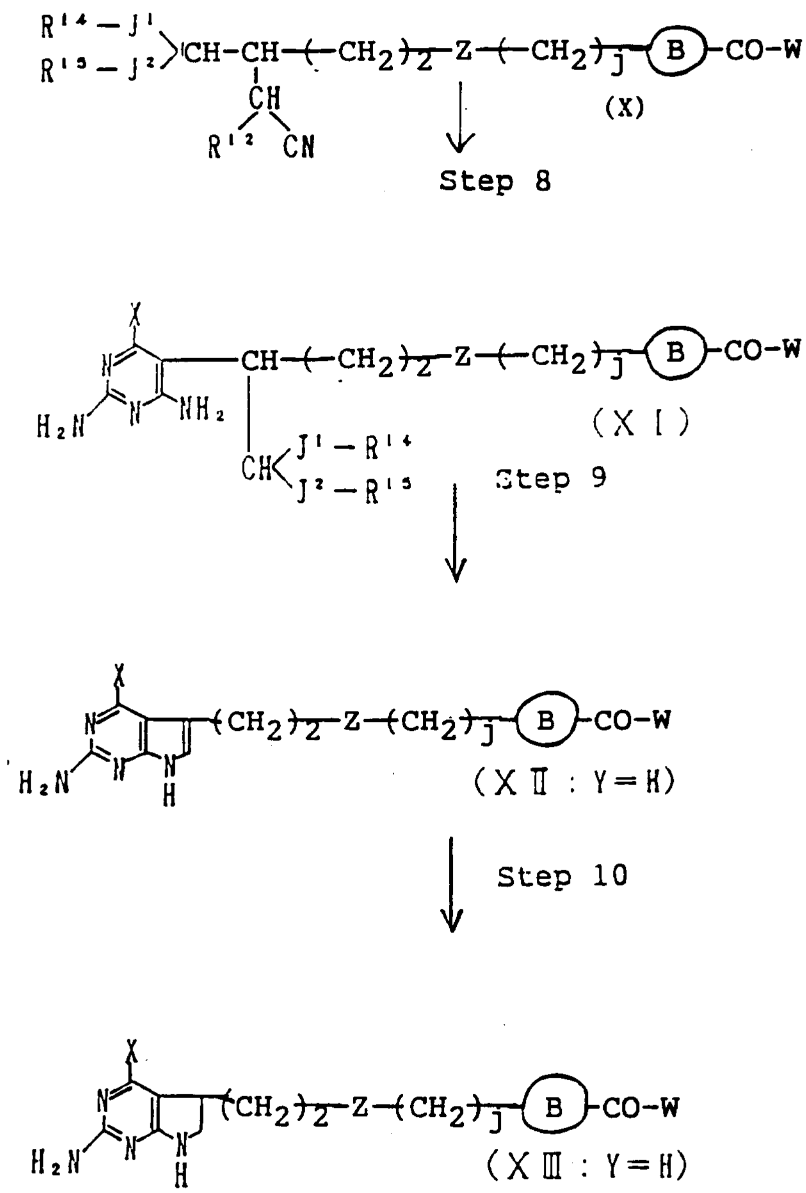


(X)

(XI)

(XII : Y=H)

(XIII : Y=H)

In the above steps, each of X, Z, j, $R^{12}$ and -Ⓑ- has the same meaning as above, W is a group of the formula $OR^{10}$ or

$$\begin{array}{l} NHCHCOOR^{6} \\ \quad | \\ \quad CH_2CH_2COOR^{7} \end{array}$$

(wherein each of $R^{10}$, $R^6$ and $R^7$ has the same meaning as above), $J^1$ and $J^2$ may be the same or different and each of which is oxygen or sulfur atom, and $R^{14}$ and $R^{15}$ may be the same or different and are a hydrocarbon residue optionally having substituent(s).

The hydrocarbon residue represented by $R^{14}$ and $R^{15}$ may be a $C_{1-5}$ lower alkyl group (e.g., methyl, ethyl, propyl, iso-propyl, n-butyl, iso-butyl, sec-butyl, tert-butyl, n-pentyl, iso-pentyl, sec-pentyl, neo-pentyl or tert-pentyl), benzyl group, phenyl group, or the like. These lower alkyl group, benzyl group and phenyl group may have one to three substituent(s). The substituent may be a halogen atom (e.g., fluorine, chlorine, bromine or iodine), nitro group, cyano group, a $C_{1-4}$ alkoxy group (e.g., methoxy, ethoxy, propoxy, iso-propoxy, n-butoxy, iso-butoxy, sec-butoxy or tert-butoxy group), a $C_{1-4}$ alkyl group (e.g., methyl, ethyl, propyl, iso-propyl, butyl, iso-butyl, sec-butyl, tert-butyl group), a $C_{1-4}$ alkanoyl group (e.g., formyl, acetyl, propionyl, n-butyryl or iso-butyryl group), trifluoromethyl group, or the like.

The above reaction steps are explained in detail in the following.

Step 8

A compound (X) is treated with guanidine in a similar manner to the aforementioned Step 3 to give a compound (XI) by forming a pyrimidine ring.

Step 9

The group

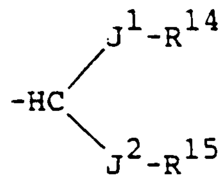

of the compound (XI) is restored to a carbonyl group (>C=O), and intramolecular ring closure reaction is spontaneously proceeded to give a compound (XII) by this step. The restoration reaction to a carbonyl group can be carried out, for example, by subjecting a compound (XI) to cleavage reaction without any solvent or in a proper solvent, at a temperature from about -10°C to the boiling point (about 100°C) of the solvent used, preferably about 0 - 50°C, for about 10 minutes to 100, hours. The cleavage reaction may be carried out by the hydrolysis under acidic condition (Method B-1), the cleavage reaction under acidic and nonaqueous condition (Method B-2), the catalytic reduction (Method C), the cleavage reaction using a metal salt (Method D) or the cleavage reaction using an oxidizing agent (Method E), or the like. The Method B-1, Method B-2 and Method C can be carried out in the same way as the methods explained for deesterification reaction of the groups $-COOR^6$ and $-COOR^7$. The metal salt to be used in the Method D is, for example, cupric chloride, silver nitrate, silver oxide, mercuric chloride, tellurium salt (e.g., tellurium nitrate, tellurium trifluoroacetate), or the like. The oxidizing agent to be used in the Method E may be oxygen light, hydrogen peroxide, perbenzoic acid, m-chloroperbenzoic acid, perchlorates (e.g., lithium perchlorate, silver perchlorate, mercuric perchlorate or tetrabutyl ammonium perchlorate), nitrosyl sulfuric acid, alkyl nitrites (e.g., iso-amyl nitrite), iodine, bromine, chlorine, N-bromosuccinimide, sulfuryl chloride, chloramine-T, or the like. The method for restoring the carbonyl group (>C = 0) may be selected in accordance with the chemical nature of the groups $-J^1-R^{14}$ and $-J^2-R^{15}$. The reaction solvent to be used in the Method D and Method E is, for example, water, alcohols (e.g., methanol, ethanol, propanol, iso-propanol, butyl alcohol, sec-butyl alcohol, tert-butyl alcohol, ethylene glycol or methoxyethanol, ethoxyethanol), ethers (e.g., dimethyl ether, diethylether, tetrahydrofuran, dioxane, monoglyme or diglyme), aromatic hydrocarbons (e.g., benzene, toluene or xylene), halogenated hydrocarbons (e.g., dichloromethane, chloroform or carbon tetrachloride), acetone or acetonitrile, or an optional mixture thereof. Pyrrolo[2,3-d]pyrimidine ring is formed by spontaneous condensation with the amino group or the pyrimidine ring in the course of the restoration to carbonyl group (>C = 0) or after the restoration in the intramolecular ring closure reaction in the step for preparing the compound (XII). The use of an acidic catalyst can accelerate the ring closure reaction and achieve a high yield. The acidic catalyst may be the mineral acids, organic acids or Lewis acids as exemplified in the aforementioned Method B-1 and Method B-2.

Step 10

The compound (XII) where the ring A is a pyrrole ring obtained by the above Step 9 can be easily converted to a compound (XIII) where the ring A is pyrroline ring through a catalytic reduction, if necessary. The catalytic reduction can be advantageously carried out in the same way as the aforementioned Method C.

Further, an ester of the compound (XII) or (XIII) can be converted to the corresponding carboxylic acid (I-1; Y = H) or (II; Q = Y = H) by deesterification which is carried out in a similar manner to the Step 4.

The substituent X on the pyrimine ring of thus obtained compound (I), (II) or (VI) or its salt can be converted into another X, if necessary, by a known method described in literature [Protein, Nucleic Acid and Enzyme, Extra issue : Chemical Synthesis of Nucleic Acid, Kyoritsu Shuppan, Japan (1968)].

And further, the reaction, reagent, reaction conditions and protective group optionally applied to each functional group in the aforementioned Step 1 to Step 10 and in the preparation of the starting compounds (III), (IV), (V), (VII), and (X) are known and explained in detail in the following literature.
[J.F.W. McOmine; Protective Groups in Organic Chemistry; Plenum Press, London and New York (1973)],
[Pyne, Hendrickson, Hamond; Organic Chemistry, 4th Edition [I]-[II]; Organic Chemistry, 4th Edition [I]-[II]; Hirokawa Shoten, Japan (1982)], [M.Fieser and L. Fieser; Reagents for Organic Synthesis vol. 1 - 13); Wiley-Interscience, New York, London, Sydney and Toronto (1969-1988)], [T. Kondo et al.; Chemistry Letters 419 (1983)] and [H. Akimoto et al; J. Chem. Soc.; Perkin Trans. I, 1637(1988)].

The intermediates (II)-(XI) and object compounds (I) of this invention prepared by those steps can be isolated from the reaction mixture by a conventional method for isolation and purification, for example, concentration, solvent extraction, chromatography, recrystallization, or the like.

The compounds (I) of this invention may be in the salt form. The salts may be the salt with a pharmaceutically acceptable base or acid or the quaternary salt. The salts with base may be the alkali metal salts, alkali earth metal salt, nontoxic metal salt, ammonium salt or substituted ammonium salt, for example, sodium, potassium, lithium, calcium, magnesium, aluminium, zinc, ammonium, trimethylammonium, triethylammonium, triethanolammonium, pyridinium, substituted pyridinium salt, or the like. The salts with acid may be the salt with a mineral acid such as hydrochloric acid, sulfuric acid, nitric acid, phosphoric acid or boric acid, the salt with an organic acid such as oxalic acid, tartaric acid, acetic acid, trifluoroacetic acid, methanesulfonic acid, benzenesulfonic acid, p-toluenesulfonic acid or camphor sulfonic acid. The quarternary salt may be the salt with methyl bromide, methyl iodide, methyl methanesulfonate, methyl benzenesulfonate or methyl p-toluenesulfonate. The starting compounds (II), (III), (IV) and (V) may be in the similar salt form as mentioned above in the salt of the compound (I).

The above salt or quaternary salt of the compound (I) of this invention can be easily prepared, for example, by mixing or reacting the compound (I) with a pharmaceutically acceptable base, acid or a reagent for quaternary salt in the proportion of 1 : 1 - 10 moles, preferably 1 : 1 - 4 moles, without any solvent or in a proper solvent, at -20°C to the boiling point of the solvent used, preferably 0 - 100°C, for 5 minutes to 24 hours. The reaction solvent is, for example, water, alcohols (e.g., methanol, ethanol, propanol, iso-propanol, butyl alcohol, sec-butyl alcohol, tert-butyl alcohol, ethylene glycol, methoxyethanol or ethoxyethanol), esters (e.g., ethyl acetate), ethers (e.g., dimethyl ether, diethyl ether, tetrahydrofuran, dioxane, monoglyme or diglyme), halogenated hydrocarbons (e.g., dichloromethane, chloroform or carbon tetrachloride), nitriles (e.g., acetonitrile), aliphatic hydrocarbons (e.g., pentane, hexane, heptane or octane), cyclic aliphatic hydrocarbons (e.g., cyclopentane or cyclohexane), aromatic hydrocarbons (e.g., benzene, toluene or xylene), nitromethane, dimethylformamide, dimethylsulfoxide, hexamethylphosphoramide or sulfolane, or an optioanl mixture thereof.

Further, the compound (I) may be converted to its intramolecular salt by a known method.

## Activity

## Effects

The compounds (I) of this invention show excellent antitumor effects in mouse tumor cell strains (P388, L1210, L5178Y, B16 melanoma, MethA, Lewis Lung Carcinoma, S180 sarcoma, Ehrlich Carcinoma, Colon 38) and human tumor cell strains (HL60, KB), decrease the tumors carried by warm-blooded animals [e.g.,melanoma, sarcoma, mastocytoma, carcinoma, neoplasia, etc.] and prolong the life-span of tumor-carrying warm-blooded animals.

In the following are described the results indicating the pharmacological effects of the compounds (I) of this invention.

The cell growth inhibiting effect ($IC_{50}$) of the compounds obtained in the Examples described below in KB cells was determined by the following method.

Human epidermoid carcinoma KB cells (1 x $10^4$ cells/ml) prepared according to a conventional method were inoculated into each well of the 96-microwell plate (0.1ml in a well) and subjected to standing culture at 37°C under 5% $CO_2$ for 24 hours. To this was added a solution of one of the compounds obtained in the Examples in 10% MEM (Nissui Pharmaceutical Co. Ltd. Japan), and subjected again to standing culture at 37°C under 5% $CO_2$ for 72 hours. Then the culture was pipetted out, and another 0.1ml of the solution of MTT (Dojin Laboratories Japan) in 10 % MEM (1.0mg/ml) was added and incubated at 37°C for 4 hours. Then 0.1ml of the 100% SDS solution (Wako Pure Chemicals, Japan) was added and incubated at 37°C for further 24 hours. The absorbance at 590nm was measured and the $IC_{50}$ value of the compound was defined as the concentration of the compound required to decrease the number of cells in the untreated control group by 50%. The results obtained are shown in Table 1.

Table 1

| Test Compound | $IC_{50}$(μg/ml) |
|---|---|
| Compound of Example 4 | 0.0025 |
| Compound of Example 6 | 0.0013 |
| Compound of Example 10 | 0.0025 |

As shown in the above-mentioned results, the compounds (I) and salts thereof are excellent in inhibition of growth of KB cells of human epidermoid carcinoma. The compounds (I) of this invention and salts thereof are of low toxicity, having remarkable antitumor effect. Therefore, the preparations containing the compounds (I) or salts thereof can be employed as antitumor agents for the treatment of tumors in warm-blooded animals, particularly mammals (e.g. mouse, rat,cat,dog, rabbit, etc.).

The compounds (I) and salts thereof, when used as antitumor agents, can be administered orally and parenterally as they are or in the form of powders, granules, tablets, capsules, suppositories and injections, which are prepared according to the conventional methods using pharmaceutically acceptable carriers, excipients, diluents, etc.

The dosage varies in accordance with object animals, diseases, conditions, kind of the compounds, routes of administration, and the like. For instance, the dosage of a compound of this invention is about 2.0 - 150mg, preferably 5 - 100mg/Kg weight per day for oral aministration to warm-blooded animals, and about 1.0 - 100mg, preferably 2.5 - 50mg/Kg per day for parenteral administration. The administration route as an injection may be intramuscular injection, abdominal injection, subscutaneous injection, intravenous injection or the like.

The preparations can be produced by the per se known methods. The above-mentioned oral preparations, for example, tablets are produced by suitable combination with a binder (e.g., hydroxypropylcellulose, hydroxypropylmethylcellulose, macrogol, etc.), a disintegrator (e.g. starch, calcium carboxylmethylcellulose, etc.) and a lubricant (e.g. magnesium stearate, talc, etc.).

The parenteral preparations, for example, injections are produced by suitable combination with an agent to provide isotonicity (e.g. glucose, D-sorbitol, D-mannitol, sodium chloride, etc.), an antiseptic (e.g. benzyl alcohol, chlorobutanol, methyl p-hyrdoxybenzoate, propyl p-hydroxybenzoate, etc.) and a buffer (e.g. phosphate buffer, sodium acetate buffer, etc.).

An example of method for the production of tablets comprises mixing about 1.0 - 25mg of a compound of this invention, about 100 - 500mg of lactose, about 50 to 100mg of corn starch and about 5 to 20mg of hydroxypropylcellulose for prepartion of a tablet by a conventional means, granulating, mixing with corn starch and magnesium stearate and tabletting, so that tablets each weighing about 100 to 500mg with the diameter of about 3 to 10mm are obtained. The tablets may be coated with a mixture of acetone and ethanol, the mixture containing hydroxypropylmethylcellulose phthalate (about 10 to 20mg per tablet) and castor oil (0.5 to 2mg) at a concentration of about 5 to 100%, to give enteric coated tablets.

An example of method for an injectable preparation comprises dissolving about 2.0 to 50mg of a sodium salt of the compound of this invention in about 2ml of physiological saline for preparation of an ampoule, sealing the resultant solution in an ampoule and sterilizing the ampoule at 110°C for about 30 minutes or adding about 2ml of sterile distilled water containing about 10 to 40mg of mannitol or sorbitol into the ampoule, freeze-drying and sealing the ampoule. For use of the freeze-dried compound for subcutaneous, intraveous or intramuscular injection, the ampoule is opened and the content is dissolved in, for example, physiological saline so that the concentration of the compound may be about 1.0 to 25mg/ml.

This invention is explained concretely by the following Reference Examples and Examples.

Besides, room temperature means 10 - 30°C and % means % (w/w) unless specific attention is given.

Reference Example 1

Preparation of methyl 4-[N-(4-ethoxycarbonylphenyl)-N-methylamino]-2-iodobutyrate

A solution of butyllithium (38.16 mmol) in hexane (24.62 ml) was added to a solution of diisopropylamine (3.86g) in tetrahydrofuran (35ml) at 0°C under an atmosphere of argon gas, and the mixture was stirred for 10 minutes. After cooling to -78°C, a solution of methyl 4-[N-(4-ethoxycarbonylphenyl)-N-methylamino]butyrate (8.9g) in tetrahydrofuran (35ml) was added dropwise to the reaction mixture in 15 minutes. After stirring for 30 minutes, a solution of iodine (8.08g) in tetrahydrofuran (47.7ml) was added to the mixture and stirred for 20 minutes. After raising the temperature to 0°C in about 30 minutes, the mixture was adjusted to pH7 by adding 1N potassium hydrogen sulfate solution and

extracted with ether. The ether layer was washed with a saturated saline solution and dried over anhydrous magnesium sulfate. The solvent was distilled off under reduced pressure, and the residue was purified by flash column chromatography on silica gel [developing solvent : ethyl acetate : hexane = 1 : 19] to give the captioned compound (4.78g).

| | |
|---|---|
| IR(KBr): | 2990, 2910, 1720, 1695,1600, 1520, 1430, 1370, 1275, 1180, 1140, 1020, 950, 835cm$^{-1}$ |
| $^1$H-NMR($CDCl_3$)δ: | 1.35(3H,t,J=7Hz), 2.13-2.43(2H,m), 3.0(3H,s), 3.37-3.57(2H,m), 3.73(3H,s), 4.31(2H,q,J=7Hz), 4.33(1H,t,J=7Hz), 6.63(2H,d,J=9Hz), 7.88(2H,d,J=9Hz) |

Reference Examples 2

Preparation of methyl 4-[N-(4-ethoxycarbonylphenyl)-N-methylamino]-2-dicyanomethylbutyrate

A suspension of sodium hydride (822mg) in dimethyl sulfoxide (13.7ml) was stirred at 70°C for 1 hour. A solution of malononitrile (2.26g) in dimethyl sulfoxide (5ml) was added to the suspension under cooling with water with stirring, and the mixture was stirred for 5 minutes. To the solution was added dropwise a solution of the compound (5.58g) obtained in the above Reference Example 1 in dimethyl sulfoxide (50ml). The mixture was stirred at room temperature for 1.5 hours, poured into ice-water (400ml) at 0°C, adjusted to pH8 with 1N hydrochloric acid and then extracted with ether. The ether layer was washed with water and dried over anhydrous magnesium sulfate. The solvent was distilled off under reduced pressure and the resultant residue was purified by flash column chromatography on silica gel [developing solvent : ethyl acetate : hexane = 1 : 8] to give the captioned compound (3.68g).

| | |
|---|---|
| IR(KBr): | 2980, 2920, 2250, 1745, 1730, 1690 1525, 1435, 1380, 1280, 1240, 1180, 1120, 1020, 950cm$^{-1}$ |
| $^1$H-NMR($CDCl_3$)δ: | 1.37(3H,t,J=7Hz), 2.10-2.34(2H,m), 2.95-3.17(1H,m), 3.03(3H,s), 3.60(2H,t,J=7Hz), 3.85(3H,s), 4.17(1H,d,J=7Hz), 4.33(2H,q,J=7Hz), 6.67(2H,d,J=9Hz), 7.93(2H,d,J=9Hz) |

Reference Example 3

Preparation of ethyl 4-[N-(tert-butoxycarbonyl)-N-(4-ethoxycarbonylphenyl)amino]-2-iodobutyrate

The captioned compound (0.58g) was obtained from ethyl 4-[N-(tert-butoxycarbonyl)-N-(4-ethoxycarbonylphenyl)amino]butyrate (1.14 g) by the same method as in Reference Example 1.

| | |
|---|---|
| IR(Neat): | 2980, 2930, 1725, 1710, 1602, 1510, 1445, 1365, 1270, 1170, 1100, 1010, 860cm$^{-1}$ |
| $^1$H-NMR($CDCl_3$)δ: | 1.25(3H,t,J=7Hz), 1.40(3H,t,J=7Hz), 1.45(9H,s), 2.13-2.37(2H,m), 3.60-3.93(2H,m), 4.17(2H,q,J=7Hz), 4.31(1H,t,J=7.6Hz), 4.38(2H,q,J=7Hz), 7.28(2H,d,J,=8.8Hz), 8.03(2H,d,J=8.8Hz) |

Reference Example 4

Preparation of ethyl 4-[N-(tert-butoxycarbonyl)-N-(4-ethoxycarbonylphenyl)amino]-2-dicyanomethyl-butyrate

The captioned compound (3.47g) was obtained from the compound (4.84g) of the above Reference Example 3 by the same method as in Reference Example 2.

| | |
|---|---|
| IR(Neat): | 2980, 2930, 2250, 1740, 1710, 1602, 1515, 1450, 1365, 1270, 1160, 1100, 1015, 860cm$^{-1}$ |

$^1$H-NMR($CDCl_3$)δ: 1.30(3H,t,J=7.2Hz), 1.40(3H,t,J=7.2Hz), 1.43(9H,s), 1.95-2.29(2H,m), 3.07(1H,q,J=5.6Hz), 3.69-4.05(2H,m), 4.28(2H,q,J=7.2Hz), 4.39(2H,q,J=7.2Hz), 4.58(1H,d,J=5.6Hz), 7.26(2H,d,J=8.8Hz), 8.06(2H,d,J=8.8Hz)

Reference Example 5

Preparation of ethyl 4-[N-(tert-butoxycarbonyl)-N-[2-(2,4-diamino-6-hydroxy-7H-pyrrolo[2,3-d]pyrimidin-5-yl)ethyl]amino]benzoate

The captioned compound (3.0g) was obtained from the compound (3.45g) of the above Preparation 4 and guanidine hydrochloride (0.892g) by the same method as in Reference Example 1.

IR(KBr): 3360, 3230, 2980, 1715, 1690, 1635, 1605, 1585, 1440, 1370, 1275, 1160, 1105, 1020cm$^{-1}$

$^1$H-NMR($Me_2SO$-$d_6$)δ: 1.32(3H,t,J=7Hz), 1.39(9H,s), 1.90-2.30(2H,m), 3.30(1H,t,J=6.2Hz), 3.39-3.70(2H,m), 4.31(2H,q,J=7Hz), 5.68(2H,s), 6.03(2H,s), 7.39(2H,d,J=8.6Hz), 7.90(2H,d,J=8.6Hz)

Reference Example 6

Preparation of ethyl 4-[(4-ethoxycarbonylphenyl)oxy]butyrate

60% Sodium hydride (6.69g) was washed with hexane under an atmosphere of argon gas and suspended in dimethylformamide (100ml). To the suspension was added dropwise a solution of ethyl p-hydroxybenzoate (25.3g) in dimethylformamide (200 ml). After stirring at 0°C for 1 hour, potassium iodide (2.52 g) and ethyl 4-bromobutyrate (32.6ml) were added to the mixture. The reaction temperature was raised to room temperature, and the mixture was stirred for 15 hours. The mixture was poured into ice-water and extracted with ether. The extract was washed with a saturated sodium bicarbonate solution and then with a saturated saline solution and dried over anhydrous magnesium sulfate. The solvent was distilled off under reduced pressure, and the resultant residue was purified by distillation under reduced pressure to give the captioned compound (37.6g).

IR(Neat): 2950, 1740, 1710, 1605, 1515, 770cm$^{-1}$

$^1$H-NMR)$CDCl_3$)δ: 1.26(3H,t,J=7Hz), 1.38(3H,t,J=7Hz), 2.12-2.20(2H,m), 2.52(2H,t,J=7Hz), 4.06(2H,t,J=6Hz), 4.15(2H,q,7Hz), 4.34(2H,q,7Hz), 6.89(2H,d,J=9Hz), 7.98(2H,d,J=9Hz)

Reference Example 7

Preparation of ethyl 4-[(4-ethoxycarbonylphenyl)oxy]-2-iodobutyrate

A solution of n-butyllithium (1.5 moles) in hexane (12.5ml) was added to a solution of diisopropylamine (2.6ml) in tetrahydrofuran (15ml) at 0°C under an atmosphere of argon gas and stirred for 10 minutes. After cooling to -78°C, a solution of the compound (5.54g) of Reference Example 6 in tetrahydrofuran (50ml) was added dropwise to the mixture over 10 minutes. After stirring for 30 minutes, a solution of iodine (5.52g) in tetrahydrofuran (50ml) was added to the

mixture and stirred for 20 minutes. The reaction temperature was raised to 0°C in 30 minutes, and the mixture was adjusted to pH7 with 1N potassium hydrogen sulfate solution and extracted with ether. The extract was washed with saturated saline solution and dried over anhydrous magnesium sulfate. The solvent was distilled off under reduced pressure, and the resultant residue was purified by column chromatography on silica gel [carrier : 250g, developing solvent; hexane : ethyl acetate = 7 : 1] to give the captioned compound (1.76g).

| | |
|---|---|
| IR(Neat): | 2975, 1730, 1710, 1600, 1525, 765cm$^{-1}$ |
| $^1$H-NMR($CDCl_3$)δ: | 1.29(3H,t,J=7Hz), 1.38(3H,t,J=7Hz), 2.32-2.59(2H,m), 4.00-4.14(2H,m), 4.24(2H,q,J=7Hz), 4.35(2H,t,J=7Hz), 4.66(1H,t,J=7Hz), 6.91(2H,d,J=9Hz), 8.00(2H,d,J=9Hz) |

Reference Example 8

Preparation of ethyl 4-[(4-ethoxycarbonylphenyl)-oxy]-2-dicyanomethylbutyrate

60% Sodium hydride (305mg) was washed with hexane under an atmosphere of argon gas and suspended in dimethyl sulfoxide (10ml). The suspension was stirred at 70°C for 1 hour, and a solution of malononitrile (915mg) in dimethyl sulfoxide (5ml) was added at room temperature and stirred for 5 minutes. To the solution was added dropwise a solution of the compound (2.81g) of Reference Example 7 in dimethyl sulfoxide (20ml), and the mixture was stirred at room temperature for 1.5 hours and poured into ice-water (150ml). The aqueous layer was adjusted to pH7 with 1N hydrochloric acid and then extracted with ether. The extract was washed with water and dried over anhydrous magnesium sulfate. The solvent was distilled off under reduced pressure, and the residue was purified by column chromatography on silica gel [carrier : 120g, developing solvent; hexane : ethyl acetate = 3 : 1] to give the captioned compound (2.15g).

| | |
|---|---|
| IR(Neat): | 2995, 2900, 2200, 1740, 1700, 1600, 1510, 770cm$^{-1}$ |
| $^1$H-NMR($CDCl_3$)δ: | 1.30(3H,t,J=7Hz), 1.39(3H,t,J=7Hz), 2.30-2.58(2H,m), 3.36(1H,q,J=7Hz), 4.19(2H,t,J=6Hz), 4.27-4.43(5H,m), 6.91(2H,d,J=9Hz), 8.02(2H,d,J=9Hz) |

Reference Example 9

Preparation of ethyl 4-(4-ethoxycarbonylphenyl)-thiobutyrate

A solution of diethyl 4,4'-dithiobenzoate (3.41g) in anhydrous ethanol (100ml) was cooled to 0°C under an atmosphere of argon gas, and sodium borohydride (712mg) was added thereto and stirred at room temperature for 1 hour. Ethyl 4-bromobutyrate (5.51g) was added to the mixture and stirred at room temperature for 17 hours. The solvent was distilled off under reduced pressure, and ether was added to the residue. The mixture was washed with a saturated ammonium chloride solution and a saturated saline solution. The ether layer was separated and dried over anhydrous magnesium sulfate. The solvent was distilled off under reduced pressure, and the residue was purified by column chromatography or silica gel [carrier : 150g, developing solvent; hexane : ethyl acetate = 7 : 1] to give the captioned compound (6.00g).

| | |
|---|---|
| IR(Neat): | 2970, 1740, 1720, 1600, 760cm$^{-1}$ |
| $^1$H-NMR($CDCl_3$)δ: | 1.26(3H,t,J=7Hz), 1.38(3H,t,J=7Hz), 1.92-2.08(2H,m), 2.48(2H,t,J=7Hz), 3.04(2H,t,J=7Hz), 4.14(2H,q,7Hz), 4.36(2H,q,7Hz), 7.31(2H,d,J=9Hz), 7.94(2H,d,J=9Hz) |

Reference Example 10

Preparation of ethyl 4-(4-ethoxycarbonylphenyl)thio-2-dicyanomethylbutyrate

A solution of n-butyllithium (1.6 moles) in hexane (18ml) was added to a solution of diisopropylamine (4.1ml) in tetrahydrofuran (40ml) at 0°C under an atmosphere of argon gas and stirred for 10 minutes. The reaction mixture was cooled to -78°C, and a solution of the compound (7.79g) of Reference Example 9 in tetrahydrofuran (80ml) was added dropwise thereto. The mixture was stirred at the same temperature for 30 minutes, and a solution of iodine (6.66g) in tetrahydrofuran (70ml) was added to the mixture and stirred for 20 minutes. The temperature was raised to 0°C over 30 minutes, and the reaction mixture was adjusted to pH7 with 1N potassium hydrogen sulfate solution and extracted with ether. The extract was washed with saturated saline solution and dried over anhydrous magnesium sulfate. The solvent was distilled off under reduced pressure and the resultant residue was purified by column chromatography on silica gel [carrier : 150g, developing solvent; hexane : ethyl acetate = 7 : 1] to give crude ethyl 4-(4-ethoxycarbonylphenyl)thio-2-iodobutyrate (2.69g). This product was dissolved in dimethyl sulfoxide (30ml) and the solution was added dropwise to a solution of sodio malononitrile in dimethyl sulfoxide [prepared from 60% sodium hydride (141mg) and malononitrile (422mg) in a similar manner to Reference Example 8] under an atmosphere of argon gas. The reaction mixture was stirred at room temperature for 1.5 hours, poured into ice-water (150ml), adjusted to pH7 with 1N-hydrochloric acid and then extracted with ether. The ether layer was separated, washed with water and dried over anhydrous magnesium sulfate. The solvent was distilled off under reduced pressure, and the resultant residue was purified by column chromatography on silica gel [carrier : 120g, developing solvent; hexane : ethyl acetate = 3 : 1] to give the captioned compound (670mg).

| | |
|---|---|
| IR(Neat): | 2990, 2920, 2260, 1740, 1710, 1600, 760cm$^{-1}$ |
| $^1$H-NMR($CDCL_3$)δ: | 1.33(3H,t,J=7Hz), 1.39(3H,t,J=7Hz), 2.05-2.40(2H,m), 3.02-3.33(3H,m), 4.17(1H,d,J=6Hz), 4.30(2H,q,J=7Hz), 4.37(2H,q,J=7Hz), 7.34(2H,d,J=9Hz), 7.97(2H,d,J=9Hz) |

Reference Example 11

Preparation of ethyl 5-(tert-butoxycarbonylamino)-2-thiophenecarboxylate

A solution of di-tert-butyl carbonate (20.91g) and ethyl 5-amino-2-thiophenecarboxylate (14.87g) in dry tetrahydrofuran (130.5ml) was refluxed for 28 hours. The solvent was distilled off under reduced pressure, and the residue was recrystallized from ethanol to obtain the captioned compound (15.87g).

| | |
|---|---|
| IR(KBr): | 3260, 2970, 1720, 1710, 1660, 1560, 1505, 1450, 1365, 1260, 1150, 1090, 870cm$^{-1}$ |
| $^1$HNMR($CDCl_3$)δ: | 1.34(3H,t,J=7Hz), 1.53(9H,s), 4.30(2H,q,J=7Hz), 6.44(1H,d,J=4.2Hz), 7.57(1H,d,J=4.2Hz) |

Reference Example 12

Preparation of ethyl 5-[N-(tert-butoxycarbonyl)-N-(3-hydroxypropyl)amino]-2-thiophenecarboxylate

To solution of the compound (14.38g) of Reference Example 11 in dry 1,3-dimethyl-3,4,5,6-tetrahydro-2(1H)-pyrimidinone (79.5ml) was added 60% oily sodium hydride (2.55g) in three portions at below 30°C under an argon atomsphere. The reaction mixture was stirred at room temperature for an hour, and sodium iodide (9.53g) was added to the mixture and stirred for 10 minutes. Then, a solution of 3-bromopropanol (8.84g) in dry 1,3-dimethyl-3,4,5,6-tetrahydro-2(1H)-pyrimidinone (14ml) was dropwise added to the mixture and stirred at room temperature for 14 hours. After adding water (500ml), the reaction mixture was extracted with ethyl ether (500ml x 2). The extracts were washed with water and then saturated saline solution, and dried over sodium sulfate. The solvent was distilled off under reduced pressure, and the residue was purified by column chromatography on flash silica gel [developing solvent : ethyl acetate : hexane = 15 : 85 → 25 : 75) to obtain the captioned compound (10.57g).

| | |
|---|---|
| IR(Neat): | 3470, 2980, 1710, 1680, 1540, 1450, 1400, 1365, 1330, 1280, 1250, 1150, 1095, 840cm$^{-1}$ |

| | |
|---|---|
| $^1$H-NMR($CDCl_3$)δ: | 1.35(3H,t,J=7.2Hz), 1.57(9H,s), 1.90(2H,m), 3.68(2H,dt,J=5.8Hz,J=5.8Hz), 3.97(2H,t,J=6.4Hz), 4.31(2H,q,J=7.2Hz), 6.59(1H,d,J=4.2Hz), 7.59(1H,d,J=4.2Hz) |

Reference Example 13

Preparation of ethyl 5-[N-(tert-butoxycarbonyl)-N-(3-oxopropyl)amino]-2-thiophenecarboxylate

A solution of dimethylsulfoxide (3.81g) in dichloromethane (10ml) was added to a solution of oxalyl chloride (3.09g) in dichloromethane (30.9ml) at -60°C and stirred for 2 minutes. A solution of the compound (6.68g) of Reference Example 12 in dichloromethane (20ml) was added to the above reaction mixture within 5 minutes and stirred for 15 minutes. Triethylamine (10.27g) was added dropwise to the reaction mixture and stirred for 5 minutes. After raising the temperature to 0°C in 30 minutes, the reaction mixture was poured into water (250ml) and extracted with dichloromethane. The solvent was distilled off under reduced pressure, and the residue was purified by flash column chromatography [silica gel 100g, developing solvent : ethyl acetate-n-hexane = 5 : 95 → 10 : 90] to afford the captioned compound (4.90g).

| | |
|---|---|
| IR(Neat): | 2980, 1705, 1540, 1455, 1370, 1280, 1250, 1155, 1130, 1095, 850cm$^{-1}$ |
| $^1$H-NMR($CDCl_3$)δ: | 1.35(2H,q,J=7.2Hz), 1.55(9H,s), 2.86(2H,dt,J=7Hz,J=1.2Hz), 4.15(2H,t,J=7Hz), 4.31(2H,q,J=7.2Hz), 6.49(1H,d,J=4.4Hz), 7.59(1H,d,J=4.4Hz), 9.84(1H,s) |

Reference Example 14

Preparation of ethyl 5-[N-(tert-butoxycarbonyl)-N-(4-methoxy-3-butenyl)amino]-2-thiophenecarboxylate

A solution of potassium tert-butoxide (1 mol) in tetrahydrofuran (14.7ml) was added to a solution of (methoxymethyl)triphenylphosphonium chloride (7.56g) in toluene (22ml) at 0°C and stirred for 30 minutes. A solution of the compound (4.80g) of Reference Example 13 in toluene (14.7ml) was added dropwise to the above mixture at the same temperature and stirred for 1.5 hours. After adding ether (150ml), the reaction mixture was washed successively with water and a saturated sodium chloride solution and dried over anhydrous sodium sulfate. After removing the solvent under reduced pressure, the resulting residue was purified by flash column chromatography [developing solvent : ethyl acetate-n-hexane = 7 : 93] to afford the captioned compound (2.62g).

| | |
|---|---|
| IR(Neat): | 2990, 1710, 1655, 1540, 1450, 1405, 1370, 1285, 1255, 1100, 855cm$^{-1}$ |
| $^1$H-NMR($CDCl_3$)δ: | 1.34(3H,t,J=7.2Hz), 1.56(9H,s), 2.23-2.48(2H,m), 3.50(2H,s), 3.59(1H,s), 3.79(2H,t,J=7.4Hz), 4.31(2H,q,J=7.2Hz), 4.35(0.33H,t,J=6.4Hz), 4.67(0.67H,dt,J=12.6Hz,J=7.6Hz), 5.97(0.33H,d,J=6.4Hz), 6.31(0.67H,d,J=12.6Hz), 6.50(0.67H,d,J=4.4Hz), 6.58(0.33H,d,J=4.4Hz), 7.60(1H,d,J=4.4Hz) |

Reference Example 15

Preparation of ethyl 5-[N-(tert-butoxycarbonyl)-N-(4,4-dicyano-3-dimethoxymethylbutyl)amino]-2-thiophenecarboxylate

The compound (1.08g) of Reference Example 14 and bromonalononitrile (0.53g) were dissolved in dichloromethane (28ml) under an argon atmosphere, to which molecular sieves 3A (0.56g) and anhydrous potassium carbonate

(0.252g) were added. The mixture was irradiated for 2.5 hours with ultra-violet beam using analytical ultra-violet lamp without filter. After adding methanol (1.05ml), the reaction mixture was stirred for 15 minutes and poured into ice-water containing 2N-potassium carbonate solution (4.2ml). The mixture was extracted with dichloromethane, and the extract was washed with water and dried over anhydrous sodium sulfate. After removing the solvent under reduced pressure, the resulting residue was purified by flash column chromatography [developing solvent : ethyl acetate-n-hexane = 1 : 9 → 15 : 85] to afford the captioned compound (0.654g)

IR(Neat): 2980, 2940, 2250, 1700, 1545, 1450, 1400, 1370, 1250, 1150, 1095, 855cm$^{-1}$
$^1$H-NMR($CDCl_3$)δ: 1.35(3H,t,J=7Hz), 1.58(9H,s), 1.90-2.22(2H,m), 2.26-2.40(1H,m), 3.46(3H,s), 3.49(3H,s), 4.0(2H,t,J=7.8Hz), 4.17(1H,d,J=3.6Hz), 4.31(2H,q,J=7Hz), 4.44(1H,d,J=5.6Hz), 6.58(1H,d,J=4.2Hz), 7.61(1H,d,J=4.2Hz)

Reference Example 16

Preparation of ethyl 5-[N-(tert-butoxycarbonyl)-N-[3-(2,4,6-triaminopyrimidin-5-yl)-4,4-dimethoxybutyl]-amino]-2-thiophenecarboxylate

A solution of potassium tert-butoxide (1 mol) in tetrahydrofuran (0.825ml) was added to a solution of guanidine hydrochloride (78.9mg) in ethyl alcohol (2.0ml) under an argon atmosphere, followed by stirring for 10 minutes. A solution of the compound (340mg) of Reference Example 15 in ethyl alcohol (3.0ml) was added to the above mixture and refluxed for 12 hours. The reaction mixture was poured into ice-water (15ml) and extracted with dichloromethane. The extract was dried over anhydrous sodium sulfate and distilled under reduced pressure to remove the solvent. The residue was purified by flash column chromatography [silica gel 15g; developing solvent : dichloromethaneethyl alcohol = 30 : 1 → 19: 1] to obtain the captioned compound (289mg).

IR(KBr): 3350, 3200, 2980, 2940, 1710, 1610, 1570, 1445, 1400, 1365, 1280, 1250, 1140, 1095, 850cm$^{-1}$
$^1$H-NMR($CDCl_3$)δ: 1.34(3H,t,J=7Hz), 1.54(9H,s), 1.91-2.13(1H,m), 2.27-2.48(1H,m), 2.82-2.93(1H,m), 3.49(3H,s), 3.51(3H,s), 3.63-3.77(2H,m), 4.30(2H,q,J=7Hz), 4.39(1H,d,J=3Hz), 4.44(2H,s), 4.51(2H,s), 5.18(2H,s), 6.57(1H,d,J=4.2Hz), 7.58(1H,d,J=4.2Hz)

Example 1

Preparation of ethyl 4-[N-[2-(2,4-diamino-6-hydroxy-7H-pyrrolo[2,3-d]pyrimidin-5-yl)ethyl]-N-methylamino]benzoate

Metallic sodium (0.32g) was dissolved in anhydrous ethanol (30ml) in an atmosphere of argon gas. To this solution were added guanidine hydrochloride (1.23g) and a solution of the compound (3.68g) of Reference Example 2 in ethanol-tetrahydrofuran (2 : 1, 45ml), and the mixture was refluxed for 3 hours. The reaction mixture was cooled, poured into ice-water and adjusted to pH7.5 with 1N-aqueous solution of potassium hydrogen sulfate. The precipitating crystals were collected by filtration and washed with methanol and then with ether to give the captioned compound (2.91g).

IR(KBr): 3420, 3350, 3200, 1685, 1630, 1600, 1580, 1520, 1440, 1360, 1275, 1180, 1105cm$^{-1}$
$^1$H-NMR($Me_2SO$-$d_6$)δ: 1.27(3H,t,J=7Hz), 1.90-2.23(2H,m), 2.89(3H,s), 3.10-3.47(3H,m), 4.20(2H,q,J=7Hz), 5.87(2H,s),

6.08(2H,s), 6.63(2H,d,J=9Hz), 7.72(2H,d,J=9Hz), 10.5(1H,s)

Example 2

Preparation of ethyl 4-[N-[2-(2,4-diamino-6,7-dihydro-5H-pyrrolo[2,3-d]pyrimidin-5-yl)ethyl]-N-methylamino]benzoate (A) and ethyl 4-[N-[2-(2,4-diamino-7H-pyrrolo[2,3-d]pyrimidin-5-yl)ethyl]-N-methylamino]benzoate (B)

A solution of borane-tetrahydrofuran complex (59.36 mmol) in tetrahydrofuran (59.36ml) was added to a suspension of the compound (2.75g) of Example 1 in tetrahydrofuran (40.7ml) at 0°C, and the mixture was stirred for 10 minutes and further stirred at 10 - 15°C for 6.5 hours. After cooling, a mixture (90ml) of acetic acid and ethanol (1 : 2) was added to the reaction mixture and stirred at room temperature for 18 hours. The solvent was distilled off under reduced pressure and the resultant residue was purified by flash column chromatography on silica gel [developing solvent; dichloromethane : ethanol containing 8% ammonia = 33 : 1 → 25 : 1] to give the captioned compound (A; 1.02g) and the captioned compound (B; 0.663g).

Captioned compound (A):

IR(KBr): 3350, 3150, 2980, 1675, 1620, 1590, 1520, 1480, 1440, 1360, 1275, 1180, 1020cm$^{-1}$

$^1$H-NMR($Me_2SO-d_6$)δ: 1.28(3H,t,J=7Hz), 1.45-1.68(1H,m), 1.71-1.92(1H,m), 2.95(3H,s), 3.05-3.22(2H,m), 3.32-3.54(3H,m), 4.22(2H,q,J=7Hz), 5.38(2H,s), 5.56(2H,s), 6.0(1H,s), 6.72(2H,d,J=9Hz), 7.74(2H,d,J=9Hz)

Captioned compound (B):

IR(KBr): 3400, 3150, 1695, 1640, 1605, 1570, 1530, 1435, 1365, 1280, 1180, 1110, 1020cm$^{-1}$

$^1$H-NMR($Me_2SO-d_6$)δ: 1.28(3H,t,J=7Hz), 2.90(2H,t,J=7Hz), 2.93(3H,s), 3.61(2H,t,J=7Hz), 4.22(2H,q,J=7Hz), 5.40(2H,s), 6.02(2H,s), 6.45(1H,d,J=1.4Hz), 6.70(2H,d,J=9Hz), 7.74(2H,d,J=9Hz), 10.46(1H,s)

Example 3

Preparation of diethyl N-[4-[N-[2-(2,4-diamino-6,7-dihydro-5H-pyrrolo[2,3-d]pyrimidin-5-yl)ethyl]-N-methylamino]benzoyl]-L-glutamate

The compound (A; 891mg) of Example 2 was dissolved in a mixture (30ml) of methanol and water (1 : 1) in an atmosphere of argon gas. 1N-sodium hydroxide (10ml) was added to the solution and the mixture was stirred at 60°C for 12 hours. After addition of 1N-hydrochloric acid (10ml), the solvent was distilled off under reduced pressure, and the residue was dried at 80 °C under reduced pressure. The resultant residue and diethyl L-glutamate hydrochloride (899mg) were dissolved in dimethylformamide (15ml). A solution of diphenyl phosphoryl azide (826mg) in dimethylformamide (5ml) was added to the above solution at 0°C and stirred for 15 minutes. A solution of triethylamine (759mg) in dimethylformamide (5ml) was added dropwise to the above mixture at the same temperature. The reaction mixture was stirred at 0°C for 30 minutes, at room temperature for 24 hours and further at 35°C for 72 hours. After removing the precipitate by filtration, the solvent was distilled off under reduced pressure, and the resultant residue was purified by flash column chromatography on silica gel [developing solvent; dichloromethane : ethanol containing 8% ammonia = 33 : 1 → 25 : 1] to give the captioned compound (737mg).

IR(KBr): 3370, 3200, 2980, 2930, 1730, 1630, 1600, 1510, 1430, 1375, 1290, 1190, 1020cm$^{-1}$

$^1$H-NMR($CDCl_3$-$CD_3OD$)δ: 1.23(3H,t,J=7H,z), 1.31(3H,t,J=7Hz), 1.75-1.97(2H,m), 2.06-2.39(2H,m), 2.43-2.54(2H,m), 2.99(3H,s), 3.03-3.15(2H,m), 3.20-3.48(2H,m), 3.64-3.80(1H,m), 4.12(2H,q,J=7Hz), 4.23(2H,q,J=7Hz), 4.71-4.78(1H,m), 6.67(2H,d,J=9Hz), 7.71(2H,d,J=9Hz)

Example 4

Preparation of N-[4-[N-[2-(2,4-diamino-6,7-dihydro-5H-pyrrolo[2,3-d]pyrimidin-5-yl)ethyl]-N-methylamino]benzoyl]-L-glutamic acid

1N-Sodium hydroxide solution (2.7ml) was added to a solution of the compound (463mg) of Example 3 in tetrahydrofuran-water (3 : 2, 15ml), and the mixture was stirred at room temperature for 3 hours. The solvent was distilled off under reduced pressure to a volume of 5ml, and the solution was filtered with Millipore filter. Acetic acid (0.5ml) was added to the filtrate, and the precipitating crystals were crushed with ultrasonic wave, collected by filtration, washed with ice-water, methanol and ethanol and then dried at 60°C under reduced pressure to give the captioned compound (373mg).

IR(KBr): 3350, 3200, 2930, 1690, 1675, 1630, 1600, 1510, 1450, 1385, 1300, 1195, 820cm$^{-1}$

$^1$H-NMR($Me_2SO$-$d_6$-$D_2O$)δ: 1.42-1.66(1H,m), 1.75-2.09(3H,m), 2.29(2H,t,J=7Hz), 2.93(3H,s), 3.13-3.46(4H,m), 3.52-3.65(1H,m), 4.24-4.37(1H,m), 6.69, 6.70(2H,dd,J=9Hz), 7.69(2H,d,J=9Hz)

Example 5

Preparation of diethyl N-[4-[N-[2-(2,4-diamino-7H-pyrrolo[2,3-d]pyrimidin-5-yl)ethyl]-N-methylamino]-benzoyl]-L-glutamate

The compound (B) (621mg) of Example 2 was dissolved in methanol-water (1 : 1, 30ml). To the solution was added 1N-sodium hydroxide solution (8.75ml), and the mixture was stirred at 60°C for 12 hours. After addition of 1N-hydrochloric acid (8.75ml), the solvent was distilled off under reduced pressure, and the residue was dried at 70°C under reduced pressure. The resultant residue and diethyl L-glutamate hydrochloride (630 mg) were suspended in dimethylformamide (10 ml). To the suspension was added a solution of diphenyl phosphoryl azide (578 mg) in dimethylformamide (5 ml) at 0°C, and the mixture was stirred for 15 minutes. A solution of triethylamine (532 mg) in dimethylformamide (5 ml) was added dropwise to the above mixture at the same temperature, and the reaction mixture was stirred at 0°C for 30 minutes, at room temperature for 24 hours and further at 35°C for 72 hours. After removing the precipitate by filtration, the filtrate was concentrated under reduced pressure, and the residue was purified by flash column chromatography on silica gel [developing solvent; dichloromethane : ethanol containing 8% ammonia = 33 : 1 → 25 : 1] to give the captioned compound (485mg).

IR(KBr): 3380, 2930, 1735, 1635, 1600, 1575, 1510, 1425, 1375, 1190, 1095, 1020cm$^{-1}$

$^1$H-NMR($CDCl_3$-$CD_3OD$)δ: 1.24(3H,t,J=7Hz), 1.31(3H,t,J=7Hz), 2.05-2.41(2H,m), 2.43-2.58(2H,m), 2.92(3H,s),

2.95(2H,t,J=7Hz),
3.71(2H,t,J=7Hz),
4.13(2H,q,J=7Hz),
4.24(2H,t,J=7Hz),
4.71-4.80(1H,m), 6.53(1H,s),
6.70(2H,d,J=9Hz),
7.73(2H,d,J=9Hz)

Example 6

Preparation of N-[4-[N-[2-(2,4-diamino-7H-pyrrolo[2,3-d]pyrimidin-5-yl)ethyl]-N-methylamino]benzoyl]-L-glutamic acid

The compound (389mg) of Example 5 was dissolved in tetrahydrofuran-water (3 : 2, 15ml). To this solution was added 1N-sodium hydroxide solution (2.28ml), and the mixture was stirred at room temperature for 3 hours. The solvent was distilled off under reduced pressure to a volume of 5ml, and the concentrate was filtered with Millipore filter. Acetic acid (0.5ml) was added to the filtrate, and the precipitating crystals were crushed with ultrasonic wave, collected by filtration, washed with ice-water, methanol and ether and then dried at 60°C under reduced pressure to give the captioned compound (311mg).

| | |
|---|---|
| IR(KBr): | 3330, 3200, 2930, 1690, 1670, 1630, 1600, 1540, 1500, 1380, 1200, 1090, 950, 820cm$^{-1}$ |
| $^1$H-NMR($Me_2SO$-$d_6$)δ: | 1.87-2.17(2H,m),<br>2.34(2H,t,J=7Hz),<br>2.89(2H,t,J=7Hz), 2.92(3H,s),<br>3.60(2H,t,J=7Hz),<br>4.31-4.44(1H,m), 5.51(2H,bs),<br>6.13(2H,s), 6.47(1H,s),<br>6.68(2H,d,J=9Hz),<br>7.74(2H,d,J=9Hz),<br>8.17(1H,d,J=7Hz), 10.53(1H,s) |

Example 7

Preparation of ethyl 4-[N-[2-(2,4-diamino-6-hydroxy-7H-pyrrolo[2,3-d]pyrimidin-5-yl)ethyl]amino]benzoate

The compound (2.93g) of Reference Example 5 was dissolved in trifluoroacetic acid (30ml) and stirred at room temperature for 1 hour. The solvent was distilled off under reduced pressure. Water (60ml) was added to the residue, and the mixture was adjusted to pH7.5 with 0.1N-sodium hydroxide solution. The precipitating crystalls were collected by filtration, washed with water, ethanol and then with ether and dried to give the captioned compound (2.0g).

| | |
|---|---|
| IR(KBr): | 3470, 3380, 3170, 2980, 1690, 1650, 1600, 1580, 1530, 1440, 1360, 1280, 1175, 1120, 770cm$^{-1}$ |
| $^1$H-NMR($Me_2SO$-$d_6$+$D_2O$)δ: | 1.27(3H,t,J=7Hz),<br>2.02-2.18(2H,m),<br>2.92-3.13(2H,m),<br>3.40(1H,t),<br>4.21(2H,q,J=7Hz),<br>6.54(2H,d,J=8.8Hz),<br>7.68(2H,d,J=8.8Hz) |

Example 8

Preparation of ethyl 4-[N-[2-(2,4-diamino-6,7-dihydro-5H-pyrrolo[2,3-d]pyrimidin-5-yl)ethyl]amino]benzoate (A) and ethyl 4-[2,4-diamino-4b,5,6,7,7a,8-hexahydropyrrolo[3',2' : 4,5]pyrrolo[2,3-d]pyrimidin-7-yl]benzoate (B)

The captioned compound (A; 0.23g) and the captioned compound (B; 0.536g) were obtained from the compound (1.0g) of Example 7 by the same method as in Example 2.

Captioned compound (A):

IR(KBr): 3390, 3200, 2940, 1680, 1600, 1585, 1535, 1450, 1370, 1345, 1290, 1175, 1125, 1030$cm^{-1}$

$^1$H-NMR($Me_2SO-d_6$)δ: 1.27(3H,t,J=7Hz), 1.47-1.68(1H,m), 1.77-1.97(1H,m), 3.0-3.23(4H,m), 3.44(1H,t,J=9.8Hz), 4.20(2H,q,J=7Hz), 5.36(2H,s), 5.5(2H,s), 5.97(1H,s), 6.48(1H,t,J=4.8Hz), 6.58(2H,d,J=8.8Hz), 7.69(2H,d,J=8.8Hz)

Captioned compound (B):

IR(KBr): 3400, 3200, 1695, 1630, 1610, 1520, 1430, 1385, 1370, 1285, 1265, 1180, 1110, 1020, 770$cm^{-1}$

$^1$H-NMR($Me_2SO-d_6$)δ: 1.30(3H,t,J=7.2Hz), 1.89-2.06(1H,m), 2.14-2.35(1H,m), 3.08-3.27(1H,m), 3.42-3.57(1H,m), 3.76-3.89(1H,m), 4.24(2H,q,J=7.2Hz), 5.45(2H,s), 5.62(1H,d,J=8Hz), 5.69(2H,s), 6.79(2H,d,J=8.8Hz), 7.14(1H,s), 7.75(2H,d,J=8.8Hz)

Example 9

Preparation of diethyl N-[4-[N-[2-(2,4-diamino-6,7-dihydro-5H-pyrrolo[2,3-d]pyrimidin-5yl)ethyl]amino]benzoyl]-L-glutamate

The compound (A; 100mg) of Example 8 was dissolved in methanol-tetrahydrofuran-water 16 : 5 : 3, 14ml) in an atmosphere of argon gas. After addition of 1N-sodium hydroxide (0.87ml), the mixture was stirred at 60°C for 40 hours. To the mixture was added 1N-hydrochloric acid (0.87ml). The solvent was distilled off under reduced pressure, and the residue was dried at 80°C under reduced pressure. The resultant residue and diethyl L-glutamate hydrochloride (105 mg) were dissolved in dimethylformamide (2 ml). To the solution was added a solution of diethyl phosphorocyanidate (57 mg) in dimethylformamide (0.5 ml) at 0°C, and the mixture was stirred for 15 minutes. A solution of triethylamine (88 mg) in dimethylformamide (0.5 ml) was added dropwise to the above mixture at the same temperature, and the reaction mixture was stirred at 0°C for 30 minutes and then at room temperature for 5 hours. Precipitates were filtered off, and the solvent was distilled off from the filtrate under reduced pressure. The resultant residue was purified by flash column chromatography on silica gel [developing solvent; dichloromethane : ethanol containing 10% ammonia = 33 : 1 → 19 : 1] to give the captioned compound (61mg).

IR(KBr): 3380, 2980, 1740, 1735, 1605, 1510, 1430, 1270, 1190, 1020$cm^{-1}$

$^1$H-NMR($CDCl_3$+$CD_3OD$)δ: 1.23(3H,t,J=7.2Hz), 1.30(3H,t,J=7.2Hz), 1.71-2.05(2H,m), 2.06-2.34(2H,m), 2.35-2.55(2H,m), 3.06-3.46(4H,m), 3.72(1H,t,J=8.6Hz), 4.11(2H,q,J=7.2Hz), 4.23(2H,q,J=7.2Hz), 4.72-4.83(1H,m), 6.59,

6.60(2H,dd,J=8.8Hz),
7.66, 7.67(2H,dd,J=8.8Hz)

Example 10

Preparation of N-[4-[N-[2-(2,4-diamino-6,7-dihydro-5H-pyrrolo[2,3-d]pyrimidin-5-yl)ethyl]amino]benzoyl]-L-glutamic acid

The captioned compound (23mg) was obtained from the compound (57mg) of Example 9 in a similar manner to Example 4.

IR(KBr): 3360, 3150, 1675, 1605, 1575, 1510, 1455, 1305, 1265, 1185, 770cm$^{-1}$

$^1$H-NMR($Me_2SO$-$d_6$+$D_2O$)δ:
1.50-1.72(1H,m),
1.78-2.12(1H,m),
2.30(2H,t,J=7Hz),
3.0-3.15(2H,m),
3.17-3.33(2H,m),
3.48-3.61(1H,m),
4.23-4.34(1H,m),
6.58(2H,d,J=8.8Hz),
7.65(2H,d,J=8.8Hz)

Example 11

Preparation of diethyl N-[4-[N-[2-(2,4-diamino-7H-pyrrolo[2,3-d]pyrimidin-5-yl)ethyl]amino]benzoyl]-L-glutamate

The compound (B; 200mg) of Example 8 was dissolved in methanol-tetrahydrofuran-water (4 : 4 : 3, 22ml), and 1N-sodium hydroxide (1.76ml) was added to the solution and stirred at 60°C for 20 hours. 1N-Hydrochloric acid (1.76ml) was added to the mixture, and the solvent was distilled off under reduced pressure. The residue was dried at 70°C under reduced pressure. The resultant residue and diethyl L-glutamate hydrochloride (211mg) were dissolved in dimethylformamide (5 ml). A solution of diethyl phosphrocyanidate (115mg) in dimethyl formamide (1.0ml) was added to the above solution at 0°C and stirred for 15 minutes. A solution of triethylamine (178mg) in dimethylformamide (1.0ml) was added to the above mixture at the same temperature. The reaction mixture was stirred at 0°C for 30 minutes and at room temperature for 14 hours, and the precipitates were filtered off. The solvent was distilled off from the filtrate under reduced pressure, and the resultant residue was purified by flash column chromatography on silica gel [developing solvent; dichloromethane : ethanol containing 10% ammonia = 33 : 1 → 19 : 1] to give the captioned compound (253mg).

IR(KBr): 3400, 1740, 1605, 1575, 1545, 1515, 1425, 1270, 1190, 1100, 1020cm$^{-1}$

$^1$H-NMR($Me_2SO$-$d_6$)δ:
1.17(3H,t,J=7.2Hz),
1.18(3H,t,J=7.2Hz),
1.87-2.20(2H,m),
2.41(2H,t,J=7.7Hz),
2.92(2H,t,J=7.6Hz),
3.29(2H,t,J=7.6Hz),
4.05(2H,q,J=7.2Hz),
4.09(2H,q,J=7.2Hz),
4.32-4.46(1H,m), 5.36(2H,s),
5.97(2H,s), 6.12-6.22(1H,bs),
6.51(2=1H,d,J=1.2Hz),
6.58(2H,d,J=8.6Hz),
7.67(2H,d,J=8.6Hz),
8.23(1H,d,J=7.2Hz),
10.45(1H,d,J=1.2Hz)

Example 12

Preparation of N-[4-[N-[2-(2,4-diamino-7H-pyrrolo[2,3-d]pyrimidin-5-yl)ethyl]amino]benzoyl]-L-glutamic acid

The captioned compound (220mg) was obtained from the compound (286mg) of Example 11 in a similar manner to Example 4.

IR(KBr): 3360, 3210, 1690, 1640, 1605, 1545, 1510, 1400, 1340, 1240, 1190, 830cm$^{-1}$
$^1$H-NMR($Me_2SO$-$d_6$+$D_2O$)δ: 1.86-2.14(2H,m), 2.34(2H,t,J=7.6Hz), 2.93(2H,t,J=7Hz), 3.28(2H,t,J=7Hz), 4.30-4.38(1H,m), 6.55(1H,s), 6.58(2H,d,J=8.8Hz), 7.67(2H,d,J=8.8Hz)

Example 13

Preparation of ethyl 4-[2-(2,4-diamino-6-hydroxy-7H-pyrrolo[2,3-d]pyrimidin-5-yl)ethyloxy]benzoate

Sodium metal (263mg) was dissolved in anhydrous ethanol (50ml) in an atmosphere of argon gas. A solution of guanidine hydrochloride (1.00g) and the compound (3.03g) of Reference Example 8 in anhydrous ethanol (50ml) was added to the above solution and refluxed for 3 hours. The reaction mixture was cooled, poured into ice-water and adjusted to pH 7 with 1N-potassium hydrogen sulfate solution. The precipitating crystals were collected by filtration and washed with methanol and ether to give the captioned compound (1.96g).

IR(KBr): 3470, 3350, 3150, 3000, 2950, 2700, 1720, 1660, 770cm$^{-1}$
$^1$H-NMR($Me_2SO$-$d_6$)δ: 1.30(3H,t,J=7Hz), 2.25-2.50(2H,m), 3.33-3.43(1H,m), 3.78-3.98(1H,m), 4.02-4.19(1H,m), 4.26(2H,q,J=7Hz), 5.84(2H,s), 6.02(2H,s), 6.93(2H,d,J=9Hz), 7.86(2H,d,J=9Hz), 10.5(1H,s)

Example 14

Preparation of ethyl 4-[2-(2,4-diamino-6,7-dihydro-5H-pyrrolo[2,3-d]pyrimidin-5-yl)ethyloxy]-benzoate (A) and ethyl 4-[2-(2,4-diamino-7H-pyrrolo [2,3-d]pyrimidin-5-yl)ethyloxy]benzoate (B)

A solution of borane-tetrahydrofuran complex in tetrahydrofuran (1 mole solution, 51ml) was added to a suspension of the compound (1.90g) of Example 13 in tetrahydrofuran (35ml) at 0°C, and the mixture was stirred for 10 minutes and then at 10 - 15°C for 19 hours. After cooling, a mixture (90ml) of acetic acid and ethanol(1 : 2) was added to the reaction mixture and stirred at 40°C for 8 hours. The solvent was distilled off under reduced pressure, and the resultant residue was purified by column chromatography or silica gel[carrier : 80g, developing solvent; chloroform : methanol = 9 : 1] to give the captioned compound (A : 530mg) and the captioned compound (3 : 501mg).

Captioned compound (A):

IR(KBr): 3400, 3180, 1710, 1660, 1620, 1590, 760cm$^{-1}$
$^1$H-NMR($CDCl_3$)δ: 1.39(3H,t,J=7Hz), 2.08(2H,m), 3.39(1H,dd,J=10,2Hz), 3.40-3.54(1H,m), 3.81(1H,d,J=10Hz), 4.12(2H,t,J=5Hz), 4.35(2H,q,J=7Hz),

5.41(3H,m), 5.91(2H,m),
6.92(2H,d,9Hz), 7.98(2H,d,J=9Hz)

Captioned compound (B):

| | |
|---|---|
| IR(KBr): | 3390, 3140, 1700, 1620, 1605, 1580, 760cm$^{-1}$ |
| $^1$H-NMR($CDCl_3$)δ: | 1.37(3H,t,J=7Hz), 3.16(2H,t,J=6Hz), 4.25(2H,t,J=6Hz), 4.34(2H,q,J=7Hz), 4.90(2H,m), 5.91(2H,m), 6.64(1H, s), 6.77(1H,m), 6.89(2H,d,J=9Hz), 7.98(2H,d,J=9Hz) |

Example 15

Preparation of diethyl N-[4-[2-(2,4-diamino-6,7-dihydro-5H-pyrrolo[2,3-d]pyrimidin-5-yl)ethyloxy]benzoyl]-L-glutamate

The compound (A; 475mg) of Example 14 was dissolved in methanol-water (1 : 1, 70ml). Tetrahydrofuran (10ml) and 1N-sodium hydroxide solution (4.2ml) were added to the solution, and the mixture was stirred at 60°C for 12 hours. After addition of 1N-hydrochloric acid (4.2ml), the solvent was distilled off under reduced pressure, and the residue was dried at 80°C under reduced pressure. The resultant residue and diethyl L-glutamate hydrochloride (497mg) were dissolved in dimethylformamide (15ml). A solution of diethyl phosphorocyanidate (0.25ml) in dimethylformamide (3ml) was added to the above solution at 0°C and stirred for 15 minutes. A solution of triethylamine (0.58ml) in dimethylformamide (3ml) was added dropwise to the above mixture at the same temperature. The reaction mixture was stirred at 0°C for 30 minutes and at room temperature for 5 hours, and the precipitates were filtered off. The solvent was distilled off under reduced pressure, and the resultant residue was purified by column chromatography on silica gel [carrier : 20g, developing solvent; dichloromethane : ethanol containing 8% ammonia = 25 : 1] to give the Captioned compound (522mg).

| | |
|---|---|
| IR(KBr): | 3450, 3370, 3180, 2975, 2920, 1730, 1600, 1440, 1250, 1170cm$^{-1}$ |
| $^1$H-NMR($CDCl_3$)δ: | 1.24(3H,t,J=7Hz), 1.31(3H,t,J=7Hz), 2.00-2.41(4H,m), 2.42-2.58(2H,m), 3.31(1H,dd,J=9,2Hz), 3.72(1H,dm, J=9Hz), 3.74(1H,tm,J=7Hz), 4.10(2H,t,J=7Hz), 4.11(2H,q,J=7Hz), 4.24(2H,q,J=7H), 4.41-4.68(5H,m), 4.75-4.86(1H,m), 6.88(0.5Hx2,d,J=9Hz), 6.92(0.5Hx2,d,J=9Hz), 7.12(0.5H,d,J=9Hz), 7.50(0.5H,d,J=9Hz), 7.74(0.5Hx2,d,J=9Hz), 7.77(0.5Hx2,d,J=9Hz) |

Example 16

Preparation of N-[4-[2-(2,4-diamino-6,7-dihydro-5H-pyrrolo[2,3-d]pyrimidin-5-yl)ethyloxy]benzoyl]-L-glutamic acid

1N-Sodium hydroxide solution (1.52ml) was added to a solution of the compound (254 mg) of Example 15 in tetrahydrofuran-water (3:2, 10 ml) and stirred at room temperature for 2 hours. The solvent was distilled off under reduced pressure to a volume of 5 ml and filtered with Millipore filter. Acetic acid (0.1ml) was added to the filtrate, and the precipitating crystals were crushed with ultrasonic wave, collected by filtration, washed successively with ice-water, methanol and ether, and dried at 60°C under reduced pressure to give the captioned compound (163mg) as colorless crystals.

IR(KBr): 3350, 3200, 2950, 1700-1600, 1250, 770cm$^{-1}$

$^1$H-NMR($Me_2SO-d_6$)δ: 1.72-2.18(4H,m), 2.20-2.35(2H,m), 3.20-3.60(3H,m), 4.07(2H,t,J=7Hz), 4.33(1H,dm,J=8Hz), 5.92(2H,m), 6.01(2H,m), 6.54(1H,bs), 6.98(2H,d,9Hz), 7.83(2H,d,J=9Hz), 8.31(1H,d,J=8Hz)

Example 17

Preparation of diethyl N-[4-[2-(2,4-diamino-7H-pyrrolo[2,3-d]pyrimidin-5-yl)ethyloxy]benzoyl]-L-glutamate

The captioned compound (43mg) was obtained from the compound (B; 476mg) of Example 14 in a similar manner to Example 15.

$^1$H-NMR($CDCL_3$)δ: 1.22(3H,t,J=7Hz), 1.28(3H,t,J=7Hz), 2.07-2.39(2H,m), 2.40-2.52(2H,m), 3.13(2H,t,J=6Hz), 4.10(2H,q,J=7Hz), 4.18(2H,t,J=6Hz), 4.23(2H,q,J=7Hz), 4.70-4.86(3H,m), 5.60(2H,bs), 6.59(1H,s), 6.82(2H,d,J=9Hz), 7.20(1H,d,J=8Hz), 7.69(2H,d,J=9Hz), 9.00(1H,bs)

Example 18

Preparation of N-[4-[2-(2,4-diamino-7H-pyrrolo[2,3-d]pyrimidin-5-yl)ethyloxy]benzoyl]-L-glutamic acid

The captioned compound (13mg) was obtained as colorless crystals from the compound (43mg) of Example 17 in a similar manner to Example 16.

IR(KBr): 3340, 3200, 2930, 1670, 1640, 1600, 1500, 1250, 765cm$^{-1}$

$^1$H-NMR($Me_2SO-d_6$)δ: 1.87-2.15(2H,m), 2.28-2.42(2H,m), 3.06-3.20(2H,m), 4.20(2H,t,J=7Hz), 4.30-4.45(1H,m), 5.45(2H,m), 6.14(2H,m), 6.57(1H,d,J=2Hz), 7.01(2H,d,J=9Hz), 7.86(2H,d,J=9Hz), 8.44(1H,d,J=8Hz), 10.54(1H,d,J=2Hz)

Example 19

Preparation of ethyl 4-[2-(2,4-diamino-6-hydroxy-7H-pyrrolo[2,3-d]pyrimidin-5-yl)ethylthio]benzoate

The captioned compound (361mg) was obtained from the compound (744mg) of Reference Example 10 and guanidine hydrochloride (237mg) in a similar manner to Example 13.

IR(KBr): 3500, 3450, 3340, 3200, 2950, 2680, 1720, 1655, 1630, 770cm$^{-1}$

$^1$H-NMR($Me_2SO-d_6$)δ: 1.31(3H,t,J=7Hz), 2.06-2.15(2H,m), 2.31-2.42(1H,m), 2.43-2.57(1H,m),

3.34-3.75(1H,m),
4.29(2H,q,J=7Hz), 5.91(2H,s),
6.08(2H,s), 7.34(2H,d,J=9Hz),
7.85(2H,d,J=9Hz), 10.55(1H,s)

Example 20

Preparation of ethyl 4-[2-(2,4-diamino-6,7-dihydro-5H-pyrrolo[2,3-d]pyrimidin-5-yl)ethylthio]benzoate (A) and ethyl 4-[2-(2,4-diamino-7H-pyrrolo[2,3-d]pyrimidin-5-yl)ethylthio]benzoate (B)

The captioned compound (A; 120mg) and the captioned compound (B; 351mg) were obtained from the compound (613mg) of Example 19 in a similar manner to Example 14.

Captioned compound (A):

IR(KBr): 3400, 3350, 3170, 2940, 1710, 1660, 1620, 1580, 1280, 1100, 760cm$^{-1}$

$^1$H-NMR($Me_2SO$-$d_6$)δ: 1.39(3H,t,J=7Hz),
1.90-2.07(2H,m),
3.00-3.18(2H,m),
3.26-3.48(2H,m),
3.67-3.80(1H,m),
4.37(2H,q,J=7Hz), 4.66(2H,m),
4.91(2H,m), 7.29(2H,d,J=9Hz),
7.95(2H,d,J=9Hz)

Captioned compound (B)

IR(KBr): 3460, 3380, 3130, 2930, 1700, 1620, 1580, 1280, 1100, 760cm$^{-1}$

$^1$H-NMR($Me_2SO$-$d_6$)δ: 1.31(3H,t,J=7Hz),
3.03(2H,t,J=7Hz),
3.26(2H,t,J=7Hz),
4.29(2H,q,J=7Hz), 5.39(2H,bs),
6.00(1H,s), 6.55(2H,d,J=1Hz),
7.38(2H,d,J=9Hz),
7.86(2H,d,J=9Hz)

Example 21

Preparation of diethyl N-[4-[2-(2,4-diamino-6,7-dihydro-5H-pyrrolo[2,3-d]pyrimidin-5-yl)ethylthio]benzoyl]-L-glutamate

The captioned compound (81mg) was obtained from the compound (97mg) of Example 20 and diethyl L-glutamate hydrochloride (97 mg) in a similar manner to Example 15.

$^1$H-NMR($CDCl_3$)δ: 1.23(3H,t,J=7Hz),
1.31(3H,t,J=7Hz), 1.80-2.00(2H,m),
2.05-2.38(2H,m), 2.44-2.56(2H,m),
2.92-3.09(2H,m), 3.20-3.41(2H,m),
3.60-3.72(1H,m),
4.11(2H,q,J=7Hz),
4.24(2H,q,J=7Hz),
4.53-4.69(2H,m), 4.70-4.90(3H,m),
7.26(0.5Hx2,d,J=9Hz),
7.27(0.5Hx2,d,J=9Hz),
7.47(0.5H,d,J=8Hz),
7.55(0.5H,d,J=8Hz),
7.85(0.5Hx2,d,J=9Hz),
7.91(0.5x2H,d,J=9Hz)

Example 22

Preparation of N-[4-[2-(2,4-diamino-6,7-dihydro-5H-pyrrolo[2,3-d]pyrimidin-5-yl)ethylthio]benzoyl]-L-glutamic acid

The captioned compound (13mg) was obtained as colorless crystals from the compound (28mg) of Example 21 in a similar manner to Example 16.

IR(KBr): 3340, 3180, 2920, 1700-1580, 1400, 1300, $750cm^{-1}$

$^1$H-NMR($Me_2SO-d_6$)δ: 1.60-1.78(1H,m), 1.80-2.18(3H,m), 2.20-2.37(2H,m), 2.90-3.03(2H,m), 3.20-3.36(2H,m), 3.44-3.60(1H,m), 4.20-4.35(1H,m), 6.21(0.5Hx2,bs), 6.29(0.5Hx2,bs), 6.44(0.5Hx2,bs), 6.52(0.5Hx2,bs), 6.88(1H,m), 7.29(0.5Hx2,d,J=9Hz), 7.30(0.5Hx2,d,J=9Hz), 7.76(0.5Hx2,d,J=9Hz), 7.78(0.5Hx2,d,J=9Hz), 8.60(0.5H,d,J=8Hz), 8.68(0.5H,d,J=8Hz)

Example 23

Preparation of diethyl 4-[N-[2-(2,4-diamino-7H-pyrrolo[2,3-d]pyrimidin-5-yl)ethylthio]benzoyl]-L-glutamate

The captioned compound (164mg) was obtained from the compound (B; 238mg) and diethyl L-glutamate hydrochloride (239mg) in a similar manner to Example 17.

1H-NMR($Me_2SO-D_6$)δ: 1.17(3H,t,J=7Hz), 1.19(3H,t,J=7Hz), 1.97-2.20(2H,m), 2.38-2.46(2H,m), 2.96-3.09(2h,m), 3.19-3.27(2H,m), 4.05(2H,q,J=7Hz), 4.11(2H,q,J=7Hz), 4.32-4.45(1H,m), 5.37(2H,bs), 5.96(2H,bs), 6.54(1H,bs), 7.37(2H,d,J=9Hz), 7.82(2H,d,J=9Hz), 8.76(1H,d,J=8Hz)

Example 24

Preparation of N-[4-[2-(2,4-diamino-7H-pyrrolo[2,3-d]pyrimidin-5-yl)ethylthio]benzoyl]-L-glutamic acid

The captioned compound (101mg) was obtained as colorless crystals from the compound (157mg) of Example 23 in a similar manner to Example 18.

$^1$H-NMR($Me_2SO-d_6$)δ: 1.84(2H,m), 2.20-2.40(2H,m), 3.01(2H,t,J=8Hz), 3.29(2H,t,J=7Hz), 4.34-4.39(1H,m), 5.44(2H,m), 6.04(2H,bs), 6.55(1H,d,J=2Hz),

7.36(2H,d,J=9Hz),
7.81(2H,d,J=9Hz),
8.52(1H,d,J=8Hz),
10.52(1H,d,J=2Hz)

Example 25

Preparation of diethyl N-[4-[N-[2-(2,4-diamino-7H-pyrrolo[2,3-d]pyrimidin-5-yl)ethyl]-N-methylamino]benzoyl]-L-glutamate

Sodium metal (263mg) was dissolved in anhydrous ethanol (50ml) in an atmosphere of argon gas. To this solution was added a solution of guanidine hydrochloride (1.0 g) and ethyl 4-[N-[4,4-dicyano-3-(dimethoxymethyl)butyl]-N-methylamino]benzoate (3.0 g) in anhydrous ethanol (50ml), and the mixture was refluxed under heating for 3 hours. The reaction mixture was cooled, poured into ice-water and adjusted to pH7 with 1N-potassium hydrogen sulfate. The aqueous layer was extracted with ethyl acetate, and the extract was dried over anhydrous sodium sulfate. The solvent was distilled off under reduced pressure to give crude ethyl 4-[N-[3-(2,4,6-triaminopyrimidin-5-yl)-4,4-dimethoxybutyl]-N-methylamino]benzoate. This product was dissolved in a mixture of trifluoroacetic acid (15ml) and water (300mg) and stirred at room temperature for 2 hours. Trifluoroacetic acid was distilled off under reduced pressure, and the residue was dried at 70°C in vacuo. The resultant residue and diethyl L-glutamate hydrochloride (2.58g) were suspended in dimethylformamide (30ml). To the suspension was added at 0°C a solution of diethyl phosphorocyanidate (1.23g) in dimethylformamide (30ml), and the mixture was stirred for 15 minutes. A solution of triethylamine (3.27g) in dimethylformamide (30ml) was added dropwise to the above mixture at the same temperature, and the mixture was stirred at 0°C for 30 minutes and at room temperature for 2 hours. The solvent was distilled off under reduced pressure, and the resultant residue was purified by column chromatography (silica gel 225g, dichloromethane separated from conc. aqueous ammonia dichloromethane-ethanol containing 10% ammonia = 40:1 → 30:1) to give the captioned compound (2.25 g). This compound was identified with the compound of Example 5 by their physical data.

Example 26

Preparation of diethyl N-[4-[N-[2-(2,4-diamino-6,7-dihydro-5H-pyrrolo[2,3-d]pyrimidin-5-yl)ethyl]-N-methylamino]benzoyl]-L-glutamate

The compound (200mg) of Example 29 was dissolved in ethanol (200ml) containing 2% hydrochloric acid. Platinum oxide (30mg) was added to the solution and the catalytic reduction was carried out for 12 hours in an atmosphere of hydrogen gas. After removing the catalyst by filtration, the filtrate was concentrated. The resultant residue was purified by column chromatography (silica gel 20g, dichloromethane separated from conc. aqueous ammonia, dichloromethane separated from conc. aqueous ammonia-ethanol = 40 : 1 → 30 : 1) to give the captioned compound (52mg). This compound was identified with the compound of Example 5 by their physical data.

Example 27

Preparation of ethyl 5-[N-(tert-butoxycarbonyl)-N-[2-(2,4-diamino-7H-pyrrolo[2,3-d]pyrimidin-5-yl)ethyl]-amino]-2-thiophenecarboxylate

To a solution of the compound (204.5mg) of Reference Example 16 in a mixture of ethyl alcohol and water (10 : 1, 2.2ml) was added 20% hydrogen chloride-ethyl alcohol(2.0ml). The mixture was stirred at room temperature for 24 hours. After adding ice-water (10ml), the reaction mixture was neutralized with conc.-ammonia and then distilled to remove almost of ethyl alcohol. The aqueous layer was extracted with chloroform, and the extract was washed with a saturated sodium chloride solution and dried over anhydrous sodium sulfate. The solvent was distilled off under reduced pressure, and the residue was solidified with tetrahydrofuran to obtain the captioned compound (146mg).

IR(KBr): 3360, 2930, 1700, 1610, 1575, 1450, 1400, 1370, 1290, 1260, 1150, 1100, 1055, 850$cm^{-1}$
$^1$H-NMR($CDCl_3$)δ: 1.37(3H,t,J=7.2Hz), 1.51(9H,s),
3.03(2H,t,J=7.8Hz),
3.85-4.02(2H,broad),
4.33(2H,q,J=7.2Hz), 6.54(1H,s),
6.62(1H,d,J=4.4Hz),
7.61(1H,d,J=4.4Hz)

Example 28

Preparation of diethyl N-[5-[N-(tert-butoxycarbonyl)-N-[2-(2,4-diamino-7H-pyrrolo[2,3-d]pyrimidin-5-yl)ethyl]amino]-2-thenoyl]-L-glutamate.

The compound (126mg) of Example 27 was dissolved in a mixture of tetrahydrofuran and methyl alcohol (1 : 1, 4.0ml), followed by addition of 1N-sodium hydroxide solution (1.12ml). The mixture was stirred at room temperature for 25 hours, neutralized with 1N-hydrochloric acid (1.12ml) and then distilled under reduced pressure to remove the solvent. The residue was solidified with methyl alcohol and toluene and dried at 90°C, thereby affording crude 5-[N-(tert-butoxycarboxy)-N-[2-(2,4-diamino-7H-pyrrolo[2,3-d]pyrimidin-5-yl)ethyl]amino]-2-thiophenecarboxylate as powders. To a solution of all this product and diethyl L-glutamate hydrochloride (88 mg) in dimethylformamide (2.6ml) was added a solution of diethyl phosphorocyanidate (47.9mg) in dimethylformamide (0.5ml) and then dropwise a solution of triethylamine (99.2mg) in dimethylformamide (0.5ml). The mixture was stirred at 0°C for 30 minutes and at room temperature for 4 hours, and distilled under reduced pressure to remove the solvent. The resulting residue was purified by flash column chromatography [silica gel 20g, developing solvent : dichloromethane separated from conc. aqueous ammonia : ethanol containing 10% ammonia-dichloromethane = 1 : 29 → 1 : 19] to give the captioned compound (110mg).

| | |
|---|---|
| IR(KBr): | 3370, 2980, 1730, 1700, 1610, 1580, 1550, 1460, 1395, 1305, 1250, 1155, 1020, 850cm$^{-1}$ |
| $^1$H-NMR($CDCl_3$/$CD_3OD$)δ: | 1.25(3H,t,J=7.2Hz), 1.31(3H,t,J=7.2Hz), 1.51(9H,s), 2.05-2.30(2H,m), 2.35-2.53(2H,m), 3.02(2H,t,J=7.8Hz), 3.76-3.97(2H,broad), 4.13(2H,q,J=7.2Hz), 4.24(2H,q,J=7.2Hz), 4.69-4.76(1H,m), 6.54(1H,s), 6.63(1H,d,J=4.2Hz), 7.39(1H,d,J=4.2Hz) |

Example 29

Preparation of disodium N-[5-[N-(tert-butoxycarbonyl)-N-[2-(2,4-diamino-7H-pyrrolo[2,3-d]pyrimidin-5-yl)ethyl]amino]-2-thenoyl]-L-glutamate

To a solution of the compound (24.15mg) of Example 28 in tetrahydrofuran (0.8ml) was added 0.1N-sodium hydroxide aqueous solution. The mixture was stirred at room temperature for 15 hours and distilled to remove almost of tetrahydrofuran. The remaining aqueous solution was freeze-dried to obtain the captioned compound (28mg).

| | |
|---|---|
| IR(KBr): | 3370, 2970, 1695, 1605, 1580, 1455, 1400, 1310, 1245, 1150, 840cm$^{-1}$ |
| $^1$H-NMR($D_2O$)δ: | 1.04(9H,s), 1.83-2.12(2H,m), 2.15-2.32(2H,m), 2.90-3.04(2H,broad,s), 4.04-4.16(2H,broad,s), 4.21-4.27(1H,m), 6.40(1H,s), 6.72(1H,d,J=4.2Hz), 7.53(1H,d,J=4.2Hz) |

Example 30

Preparation of N-[5-[N-[2-(2,4-diamino-7H-pyrrolo[2,3-d]pyrimidin-5-yl)ethyl]amino]-2-thenoyl]-L-glutamic acid

Trifluoroacetic acid (0.2ml) was added to a solution of the compound (45 mg) of Example 28 in dichloromethane (2.0 ml) under an argon atmosphere and stirred at room temperature for 14 hours. After adding toluene, the reaction mixture was distilled to remove the solvent, and the residue was dissolved in a mixture of tetrahydrofuran and water (1 : 1, 2.8ml). After adding 1N-sodium hydroxide aqueous solution (0.375ml), the mixture was stirred at room temperature for 3.5 hours and concentrated under reduced pressure to become a volume of 1.5ml. Insoluble material in a little amount were filtered off using Millipore filter. The filtrate was neutralized with acetic acid (0.4ml). The resulting precipitate as collected by filtration was washed successively with ice-water, methanol and ether and dried at 70°C under reduced pressure to obtain the captioned compound (24mg).

IR(KBr): 3340, 2940, 1660, 1550, 1520, 1460, 1400, 1340, 1300, 1150, 1090, $820cm^{-1}$

$^1H$-NMR($Me_2SO-d_6/D_2O$)δ: 1.76-2.07(2H,m), 2.32(2H,t,J=7.2Hz), 2.93(2H,t,J=6.6Hz), 3.24(2H,t,J=6.6Hz), 4.24-4.31(1H,m), 5.85(1H,d,J=4Hz), 6.53(1H,s), 7.49(1H,d,J=4Hz)

The following compounds can be synthesized in a similar manner to the above Reference Examples or Examples.

(1) N-[4-[N-[2-(2,4-diamino-6,7-dihydro-5H-pyrrolo[2,3-d]pyrimidin-5-yl)ethyl]-N-ethylamino]benzoyl]-L-glutamic acid

(2) N-[4-[N-[2-(2,4-diamino-7H-pyrrolo[2,3-d]pyrimidin-5-yl)ethyl]-N-ethylamino]benzoyl]-L-glutamic acid

(3) N-[4-[N-[2-(2,4-diamino-7H-pyrrolo[2,3-d]pyrimidin-5-yl)ethyl]-N-propylamino]benzoyl]-L-glutamic acid

(4) N-[5-[N-[2-(2,4-diamino-6,7-dihydro-5H-pyrrolo-[2,3-d]pyrimidin-5-yl)ethyl]-N-methylamino]thiazole-2-carbonyl]-L-glutamic acid

(5) N-[5-[N-[2-(2,4-diamino-7H-pyrrolo[2,3-d]pyrimidin-5-yl)ethyl]-N-methylamino]thiazole-2-carbonyl]-L-glutamic acid

(6) N-[5-[N-[2-(2,4-diamino-6,7-dihydro-5H-pyrrolo[2,3-d]pyrimidin-5-yl)ethyl]-N-methylamino]-2-thenoyl]-L-glutamic acid

(7) N-[5-[N-[2-(2,4-diamino-7H-pyrrolo[2,3-d]pyrimidin-5-yl)ethyl]-N-methylamino]-2-thenoyl]-L-glutamic acid

(8) N-[5-[N-[2-(2-amino-4-hydroxy-6,7-dihydro-5H-pyrrolo[2,3-d]pyrimidin-5-yl)ethyl]-N-methylamino]-thiazole-2-carbonyl]-L-glutamic acid

(9) N-[5-[N-[2-(2-amino-4-hydroxy-7H-pyrrolo[2,3-d]pyrimidin-5-yl)ethyl]-N-methylamino]thiazole-2-carbonyl]-L-glutamic acid

(10) N-[5-[N-[2-(2-amino-4-hydroxy-6,7-dihydro-5H-pyrrolo[2,3-d]pyrimidin-5-yl)ethyl]-N-methylamino]-2-thenoyl]-L-glutamic acid

(11) N-[5-[N-[2-(2-amino-4-hydroxy-7H-pyrrolo[2,3-d]pyrimidin-5-yl)ethyl]-N-methylamino]-2-thenoyl]-L-glutamic acid

(12) N-[4-[N-[2-(2,4-diamino-7H-pyrrolo[2,3-d]pyrimidin-5-yl)ethyl]-N-methylamino]methylbenzoyl]-L-glutamic acid

(13) N-[5-[N-(tert-butoxycarbonyl)-N-[2-(2,4-diamino-6,7-dihydro-5H-pyrrolo[2,3-d]pyrimidin-5-yl)ethyl]amino]-2-thenoyl]-L-glutamic acid

(14) N-[5-[N-[2-(2,4-diamino-6,7-dihydro-5H-pyrrolo[2,3-d]pyrimidin-5-yl)ethyl]amino]-2-thenoyl]-L-glutamic acid

(15) N-[5-[N-(iso-propyloxycarbonyl)-N-[2-(2,4-diamino-6,7-dihydro-5H-pyrrolo[2,3-d]pyrimidin-5-yl)ethyl]amino]-2-thenoyl]-L-glutamic acid

(16) N-[5-[N-(iso-propyloxycarbonyl)-N-[2-(2,4-diamino-7H-pyrrolo[2,3-d]pyrimidin-5-yl)ethyl]amino]-2-thenoyl]-L-glutamic acid

(17) N-[4-[N-[2-(2,4-diamino-6,7-dihydro-5H-pyrrolo[2,3-d]pyrimidin-5-yl)ethyl]-N-(ethoxycarbonyl)amino]benzoyl]-L-glutamic acid

(18) N-[4-[N-(2-(2,4-diamino-6,7-dihydro-5H-pyrrolo[2,3-d]pyrimidin-5-yl)ethyl]-N-(methoxycarbonyl)amino]benzoyl]-L-glutamic acid

(19) N-[4-[N-[2-(2,4-diamino-7H-pyrrolo[2,3-d]pyrimidin-5-yl)ethyl]-N-(ethoxycarbonyl)amino]benzoyl]-L-glutamic acid

(20) N-[4-[N-[2-(2,4-diamino-7H-pyrrolo[2,3-d]pyrimidin-5-yl)ethyl]-N-(methoxycarbonyl)amino]benzoyl]-L-glutamic acid.

## Claims

### Claims for the following Contracting States : AT, BE, CH, DE, DK, FR, GB, IT, LI, LU, NL, SE

1. A compound of the formula (I)

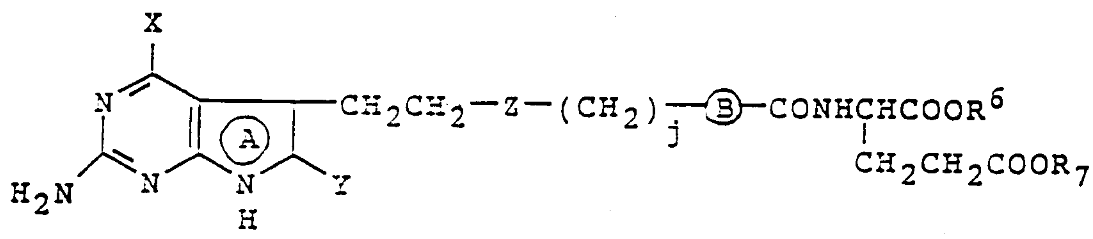

(I)

wherein the ring Ⓐ is a pyrrole ring which may be hydrogenated, X is an amino, hydroxyl or mercapto group, Y is a hydrogen atom or a hydroxyl group, Z is -O-, -S- or

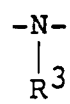

in which $R^3$ is a hydrogen atom, $C_{1-3}$ alkyl group or $C_{2-5}$ alkoxycarbonyl group, -Ⓑ- is phenyl-1,4-ylene, thiazol-2,5-ylene or thiophen-2,5-ylene group, $-COOR^6$ and $-COOR^7$ may be the same or different and are each a carboxyl group which may be esterified, wherein $R^6$ and $R^7$ are the same or different and are each hydrogen, an alkyl group with 1 to 5 carbon atoms or an optionally substituted benzyl or phenyl group, and j is 0 or 1, or its salt.

2. A compound of claim 1 wherein the moiety $-(CH_2)_j-$is a chemical bond, Z is -O-, -S- or

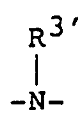

in which $R^{3'}$ is a $C_{1-3}$ alkyl group, -Ⓑ- is a phenyl-1,4-ylene or thiophen-2,5-ylene group, and $-COOR^6$ and $-COOR^7$ are a carboxyl group or a carboxyl group esterified with a $C_{1-5}$ alkyl group, or its salt.

3. A compound of claim 1 which is a compound of the formula:

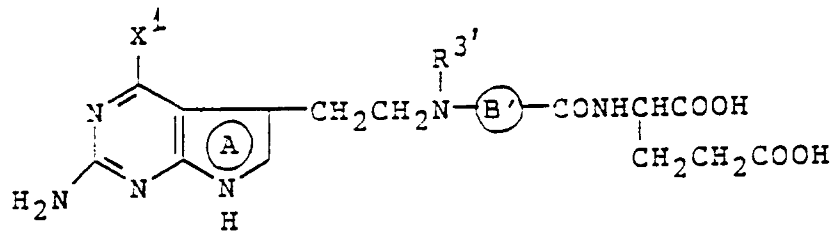

wherein the ring Ⓐ is a pyrrole ring which may be hydrogenated, $X^1$ is an amino or hydroxyl group, $R^{3'}$ is a $C_{1-3}$ alkyl group and -Ⓑ'- is phenyl-1,4-ylene, thiazol-2,5-ylene or thiophen-2,5-ylene group, or its pharmaceutically acceptable ester or salt.

4. A compound of claim 1 wherein the ring Ⓐ is a pyrroline ring, X is an amino group, Y is a hydrogen atom, the moiety $-(CH_2)_j-$ is a chemical bond, Z is an amino group or an amino group substituted by a $C_{1-3}$ alkyl, -Ⓑ- is a phenyl-1,4-ylene group, and $-COOR^6$ and $-COOR^7$ are a carboxyl group or an ethoxycarbonyl group, or its salt.

5. A compound of claim 1 which is N-[4-[N-[2-(2,4-diamino-6,7-dihydro-5H-pyrrolo[2,3-d] pyrimidin-5-yl)ethyl]-N-methylamino]benzoyl]-L-glutamic acid, or its diethyl ester or pharmaceutically acceptable salt.

6. A compound of the formula (VI)

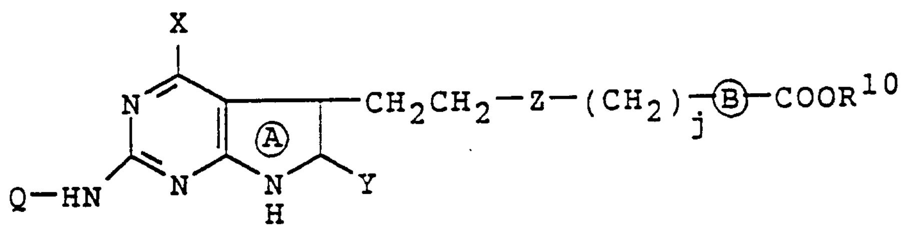 (VI)

wherein each of the ring Ⓐ, X, Y, Z, -Ⓑ- and j has the same meaning as defined in claim 1, Q is a hydrogen atom or an amino protecting group, and $-COOR^{10}$ is a carboxyl group which may be esterified, wherein $R^{10}$ has the same meaning as $R^6$ and $R^7$ respectively as defined in claim 1, or its salt.

7. A process for preparing a compound of the formula (I)

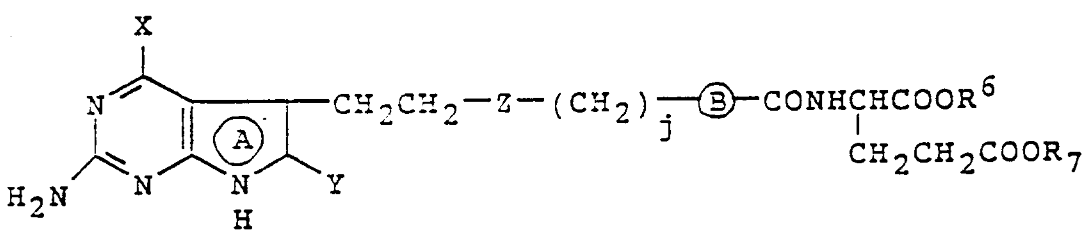 (I)

wherein the ring Ⓐ is a pyrrole ring which may be hydrogenated, X is an amino, hydroxyl or mercapto group, Y is a hydrogen atom or a hydroxyl group, Z is -O-, -S-or

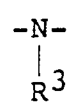

in which $R^3$ is a hydrogen atom, $C_{1-3}$ alkyl group or $C_{2-5}$ alkoxycarbonyl group, -Ⓑ- is phenyl-1,4-ylene, thiazol-2,5-ylene or thiophen-2,5-ylene group, -COOR$^6$ and -COOR$^7$ may be the same or different and are each a carboxyl group which may be esterified, wherein R$^6$ and R$^7$ are the same or different and are each hydrogen, an alkyl group with 1 to 5 carbon atoms or an optionally substituted benzyl or phenyl group, and j is 0 or 1, or its salt, which comprises reacting a compound of the formula (II)

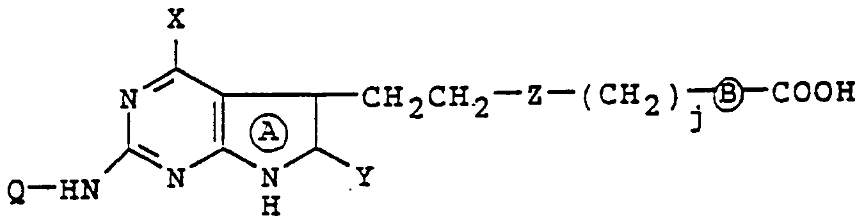

(II)

wherein each of the ring Ⓐ, X, Y, Z, -Ⓑ- and j has the same meaning as above, and Q is a hydrogen atom or an amino protecting group or its salt or reactive derivative at the carboxyl group, with a compound of the general formula (III)

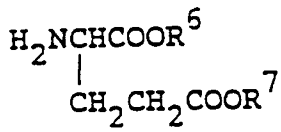

(III)

wherein each of -COOR$^6$ and -COOR$^7$ has the same meaning as above, and if necessary, subjecting the resultant product to removal reaction of the protecting group.

8. A process for preparing a compound of the formula

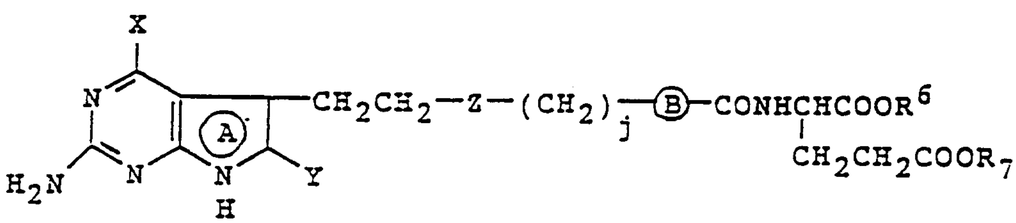

(I)

(I) wherein the ring Ⓐ is a pyrrole ring which may be hydrogenated, X is an amino, hydroxyl or mercapto group, Y is a hydrogen atom or a hydroxyl group, Z is -O-, -S- or

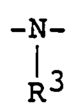

in which R$^3$ is a hydrogen atom, $C_{1-3}$ alkyl group or $C_{2-5}$ alkoxycarbonyl group, -Ⓑ- is phenyl-1,4-ylene, thiazol-2,5-ylene or thiophen-2,5-ylene group, -COOR$^6$ and -COOR$^7$ may be the same or different and are each a caboxyl group which may be esterified, wherein R$^6$ and R$^7$ are the same or different and are each hydrogen, an alkyl group with 1 to 5 carbon atoms or an optionally substituted benzyl or phenyl group, and j is 0 or 1, or its salt, which comprises reacting a compound of the formula (IV)

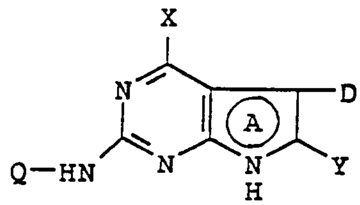

(IV)

wherein each of the ring Ⓐ, X and Y has the same meaning as above, Q is a hydrogen atom or an amino protecting group, and D is a group of the formula $-CH_2CH_2-L$ in which L is a leaving group, a group of the formula $-CH_2CH_2-ZH$ in which Z has the same meaning as above, a group of the formula

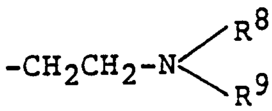

in which $R^8$ and $R^9$ may be the same or different and are each a hydrogen atom or a hydrocarbon radical optionally having substituent(s) or are combined to form a cyclic amino group together with the adjacent nitrogen atom, or a group of the formula $-CH_2CHO$, or its salt, with a compound of the formula (V)

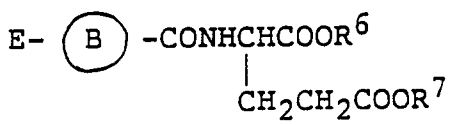

(V)

wherein each of Ⓑ, $-COOR^6$ and $-COOR^7$ has the same meaning as above, E is a group of the formula $L-(CH_2)_{m'}-$ in which L has the same meaning as above, and m' is 0 or 1, a group of OHC- or a group of the formula $HZ-(CH_2)_{m'}-$ in which Z and m' have the same meaning as above, and then subjecting the resultant product to removal reaction of the protecting group, if necessary.

**9.** An antitumor composition comprising an effective amount of a compound of the formula (I) in claim 1 or its pharmaceutically acceptable salt, and a pharmaceutically acceptable carrier or diluent.

**10.** Use of a compound of the formula (I) in claim 1 or its pharmaceutically acceptable salt in the preparation of an antitumor composition.

**Claims for the following Contracting States : ES, GR**

**1.** A process for preparing a compound of the formula (I)

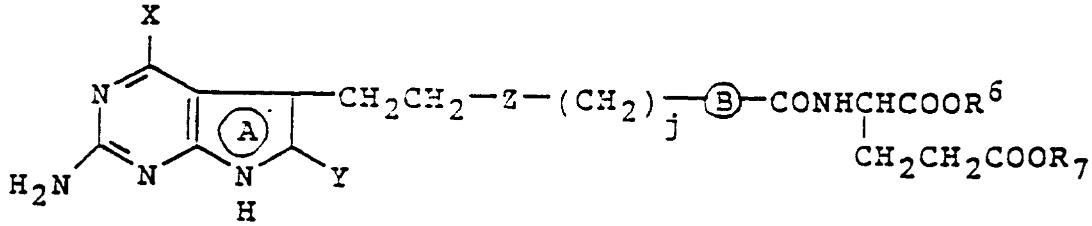

(I)

wherein the ring Ⓐ is a pyrrole ring which may be hydrogenated, X is an amino, hydroxyl or mercapto group, Y is a hydrogen atom or a hydroxyl group, Z is -O-, -S-or

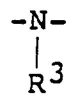

in which $R^3$ is a hydrogen atom, $C_{1-3}$ alkyl group or $C_{2-5}$ alkoxycarbonyl group, -Ⓑ- is phenyl-1,4-ylene, thiazol-2,5-ylene or thiophen-2,5-ylene group, -$COOR^6$ and -$COOR^7$ may be the same or different and are each a carboxyl group which may be esterified, wherein $R^6$ and $R^7$ are the same or different and are each hydrogen, an alkyl group with 1 to 5 carbon atoms or an optionally substituted benzyl or phenyl group, and j is 0 or 1, or its salt,
which comprises reacting a compound of the formula (II)

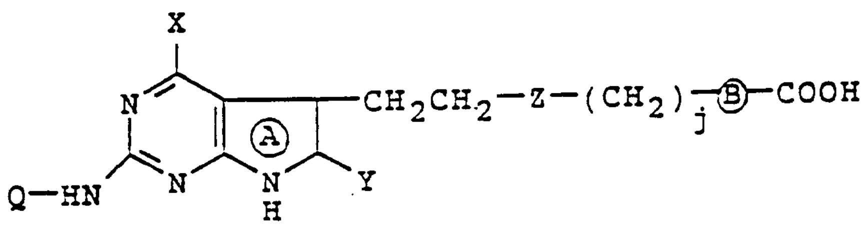

(II)

wherein each of the ring Ⓐ, X, Y, Z, -Ⓑ- and j has the same meaning as above, and Q is a hydrogen atom or an amino protecting group or its salt or reactive derivative at the carboxyl group, with a compound of the general formula (III)

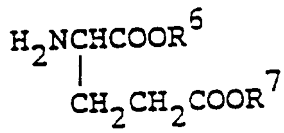

(III)

wherein each of -$COOR^6$ and -$COOR^7$ has the same meaning as above, and if necessary, subjecting the resultant product to removal reaction of the protecting group.

2. A process for preparing a compound of the formula

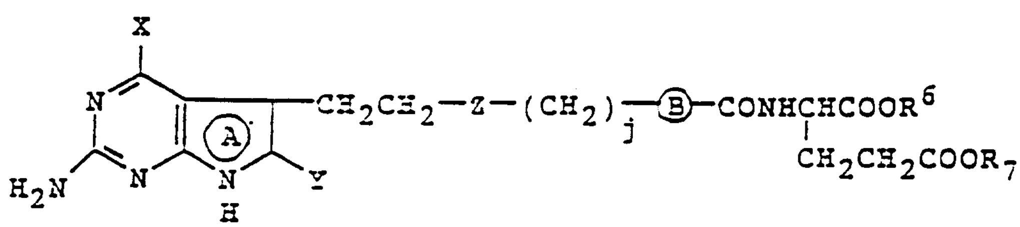

(I)

(I) wherein the ring Ⓐ is a pyrrole ring which may be hydrogenated, X is an amino, hydroxyl or mercapto group, Y is a hydrogen atom or a hydroxyl group, Z is -O-, -S- or

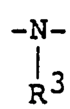

in which $R^3$ is a hydrogen atom, $C_{1-3}$ alkyl group or $C_{2-5}$ alkoxycarbonyl group, -Ⓑ- is phenyl-1,4-ylene, thiazol-2,5-ylene or thiophen-2,5-ylene group, -$COOR^6$ and -$COOR^7$ may be the same or different and are each a caboxyl group which may be esterified, wherein $R^6$ and $R^7$ are the same or different and are each hydrogen, an alkyl group

with 1 to 5 carbon atoms or an optionally substituted benzyl or phenyl group, and j is 0 or 1, or its salt, which comprises reacting a compound of the formula (IV)

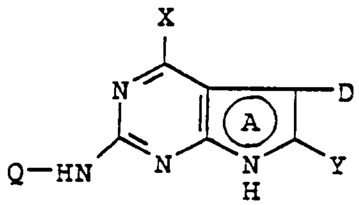

(IV)

wherein each of the ring Ⓐ, X and Y has the same meaning as above, Q is a hydrogen atom or an amino protecting group, and D is a group of the formula $-CH_2CH_2-L$ in which L is a leaving group, a group of the formula $-CH_2CH_2-ZH$ in which Z has the same meaning as above, a group of the formula

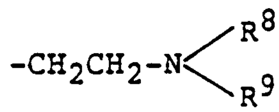

in which $R^8$ and $R^9$ may be the same or different and are each a hydrogen atom or a hydrocarbon radical optionally having substituent(s) or are combined to form a cyclic amino group together with the adjacent nitrogen atom, or a group of the formula $-CH_2CHO$, or its salt, with a compound of the formula (V)

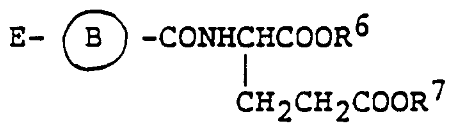

(V)

wherein each of Ⓑ, $-COOR^6$ and $-COOR^7$ has the same meaning as above, E is a group of the formula $L-(CH_2)_{m'}-$ in which L has the same meaning as above, and m' is 0 or 1, a group of OHC- or a group of the formula $HZ-(CH_2)_{m'}-$ in which Z and m' have the same meaning as above, and then subjecting the resultant product to removal reaction of the protecting group, if necessary.

**3.** A process of claim 1 or claim 2 wherein the moiety $-(CH_2)j-$ is a chemical bond, Z is -O-, -S- or

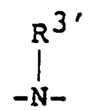

in which $R^{3'}$ is a $C_{1-3}$ alkyl group, -Ⓑ- is a phenyl-1,4-ylene or thiophen-2,5-ylene group, and $-COOR^6$ and $-COOR^7$ are a carboxyl group or a carboxyl group esterified with a $C_{1-5}$ alkyl group.

**4.** A process of claim 1 or claim 2 for preparing a compound of the formula:

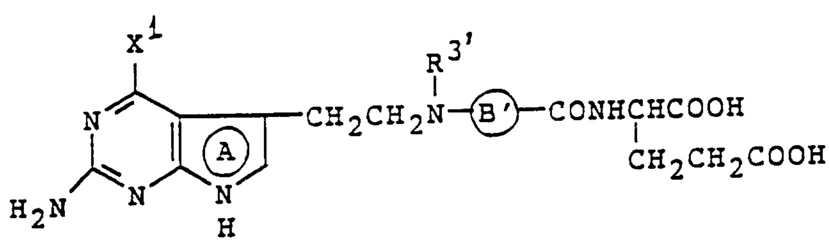

wherein the ring Ⓐ is a pyrrole ring which may be hydrogenated, $X^1$ is an amino or hydroxyl group, $R^{3'}$ is a $C_{1-3}$ alkyl group and -Ⓑ- is phenyl-1,4-ylene, thiazol-2,5-ylene or thiophen-2,5-ylene group, or its pharmaceutically acceptable ester or salt.

**5.** A process of claim 1 or claim 2 wherein the ring Ⓐ is a pyrroline ring, X is an amino group, Y is a hydrogen atom, the moiety $-(CH_2)_j-$ is a chemical bond, Z is an amino group or an amino group substituted by a $C_{1-3}$ alkyl, -Ⓑ- is a phenyl-1,4-ylene group, and $-COOR^6$ and $-COOR^7$ are a carboxyl group or an ethoxycarbonyl group.

**6.** A process of claim 1 or claim 2 for preparing N-[4-[N-[2-(2,4-diamino-6,7-dihydro-5H-pyrrolo[2,3-d] pyrimindin-5-yl)ethyl]-N-methylamino]benzoyl]-L-glutamic acid, or its diethyl ester or pharmaceutically acceptable salt.

**7.** A process for preparing a compound of the formula (VI)

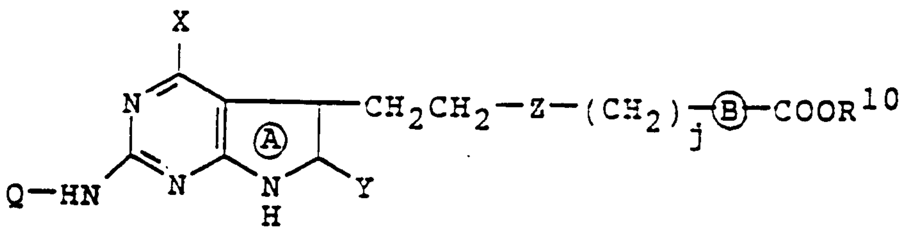

(VI)

wherein each of the ring Ⓐ, X, Y, Z, -Ⓑ- and j has the same meaning as defined in claim 1, Q is a hydrogen atom or an amino protecting group, and $-COOR^{10}$ is a carboxyl group which may be esterified, wherein $R^{10}$ has the same meaning as $R^6$ and $R^7$ respectively as defined in claim 1, or its salt, which comprises (1) introducing a removable functional group L to the active methylene part of a compound of the formula (VII)

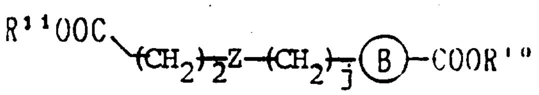

(VII)

wherein each of Z, Ⓑ, $R^{10}$ and j has the same meaning as defined above, $-COOR^{11}$ is a carboxyl group which may be esterified and $R^{11}$ has the same meaning as $R^6$ and $R^7$, respectively as defined in claim 1, to obtain a compound of the formula (VIII)

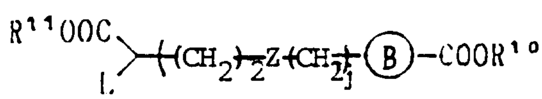

(VIII)

which is (2) subjected under a basic condition to the condensation reaction with malononitrile, $NC-CH_2COOR^{13}$, $NC-CH_2CSOR^{13}$ or $NC-CH_2CSSR^{13}$, wherein $R^{13}$ is hydrogen, a $C_{1-5}$ alkyl group, a benzyl group optionally having substituent(s) or a phenyl group optionally having substituent(s), to give a compound of the formula (IX)

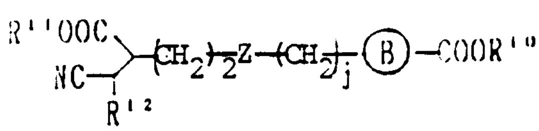

(IX)

wherein $R^{12}$ is cyano or a group of the formula $-COOR^{13}$, $-CSOR^{13}$ or $-CSSR^{13}$, wherein $R^{13}$ has the same meaning as defined above, which is (3) treated with guanidine, followed by ring closure/cyclization, and, if Y is OH, optionally followed by (4) deesterifying the ester residue $-COOR^{10}$ and (5) subjecting the product of the previous deesterifying step (4) to a reduction wherein the optional steps (4) and (5) may be carried out in a reverse order.

**8.** A process for preparing a compound of the formula (VI )

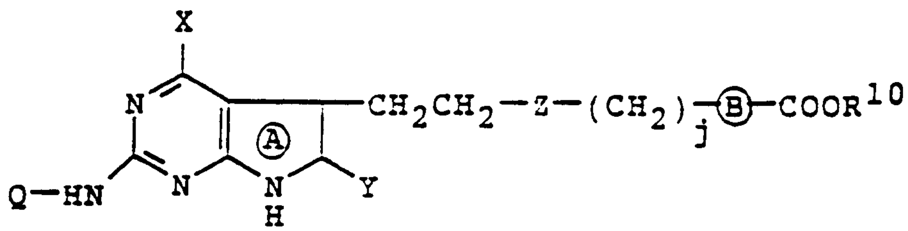

(VI )

wherein each of the ring Ⓐ, X, Z, -Ⓑ- and j has the same meaning as defined in claim 1, Y is a hydrogen atom, Q is a hydrogen atom or an amino protecting group, and $-COOR^{10}$ is a carboxyl group which may be esterified, wherein $R^{10}$ has the same meaning as $R^6$ and $R^7$ respectively as defined in claim 1, or its salt, which comprises (1) treating a compound of the formula (X)

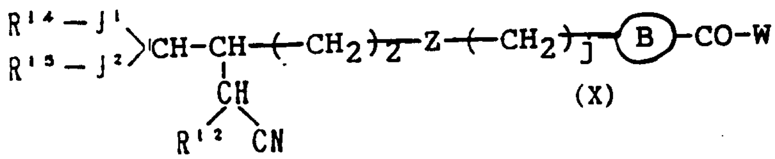

(X)

wherein j and -Ⓑ- have the same meaning as defined above, $R^{12}$ is cyano or a group of the formula $-COOR^{13}$, $-CSOR^{13}$ or $-CSSR^{13}$, wherein $R^{13}$ is hydrogen, a $C_{1-5}$ alkyl group, a benzyl group optionally having substituent(s) or a phenyl group optionally having substituent(s), W is a group of the formula $OR^{10}$, wherein $R^{10}$ has the same meaning as defined above, $J^1$ and $J^2$ may be the same or different and each of which is oxygen or sulfur atom, and $R^{14}$ and $R^{15}$ may be the same or different and are a hydrocarbon residue optionally having substituent(s); with guanidine to obtain a compound of the formula (XI)

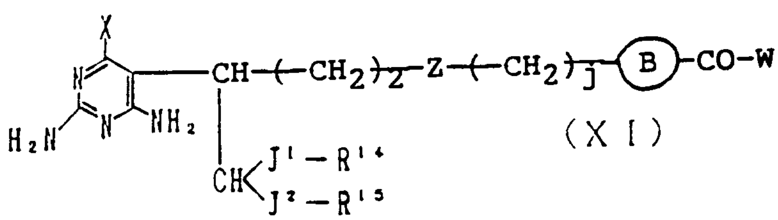

(X I)

wherein X has the same meaning as defined above,
(2) restoring the group

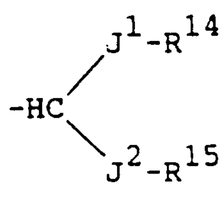

to a carbonyl group and optionally (3) converting the obtained pyrrole ring into a pyrroline ring through a catalytic reduction and optionally (4) converting an ester of the product of step (2) or (3) to the corresponding carboxylic acid by deesterification.

**9.** A process for preparing an antitumor composition comprising admixing an effective amount of a compound of the formula (I) in claim 1 or its pharmaceutically acceptable salt, and a pharmaceutically acceptable carrier or diluent.

**10.** Use of a compound of the formula (I) in claim 1 or its pharmaceutically acceptable salt in the preparation of an antitumor composition.

**Patentansprüche**

**Patentansprüche für folgende Vertragsstaaten : AT, BE, CH, DE, DK, FR, GB, IT, LI, LU, NL, SE**

**1.** Eine Verbindung der Formel I

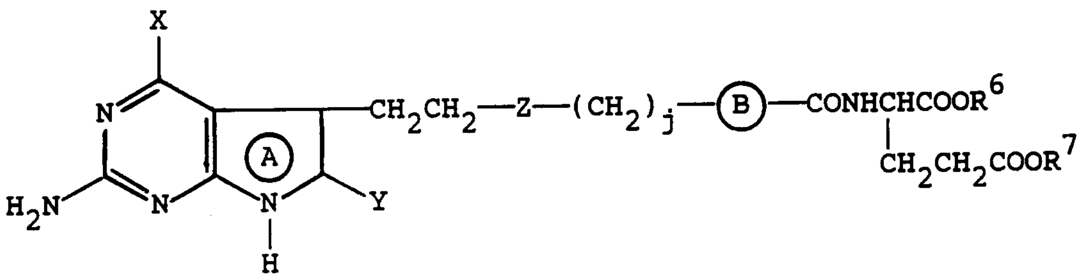

(I)

worin der Ring Ⓐ einen Pyrrolring bedeutet, der hydriert sein kann, X eine Amino-, Hydroxyl- oder Mercaptogruppe ist, Y ein Wasserstoffatom oder eine Hydroxylgruppe bedeutet, Z -O-, -S- oder

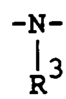

ist, worin $R^3$ ein Wasserstoffatom, eine $C_{1-3}$-Alkylgruppe oder eine $C_{2-5}$-Alkoxycarbonylgruppe bedeutet, -Ⓑ- eine Phenyl-1,4-ylen-, eine Thiazol-2,5-ylen- oder eine Thiophen-2,5-ylengruppe ist, $-COOR^6$ und $-COOR^7$ gleich oder verschieden sein können und jeder Rest eine Carboxylgruppe ist, die verestert sein kann, worin $R^6$ und $R^7$ gleich oder verschieden sind und jeder dieser Reste Wasserstoff, eine Alkylgruppe mit 1 bis 5 Kohlenstoffatomen oder eine gegebenenfalls substituierte Benzyl- oder Phenylgruppe bedeutet, und der Index j den Wert 0 oder 1 aufweist, oder deren Salz.

**2.** Eine Verbindung gemäß Anspruch 1, worin der Rest $-(CH_2)_j$-eine chemische Verbindungsstelle ist, Z -O-, -S- oder

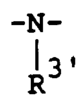

bedeutet, worin $R^{3'}$ eine $C_{1-3}$-Alkylgruppe ist, -Ⓑ- eine Phenyl-1,4-ylen- oder Thiophen-2,5-ylengruppe bedeutet und $-COOR^6$ und $-COOR^7$ eine Carboxylgruppe oder eine mit einer $C_{1-5}$-Alkylgruppe veresterte Carboxylgruppe sind, oder deren Salz.

**3.** Eine Verbindung gemäß Anspruch 1, welche eine Verbindung der nachstehenden Formel

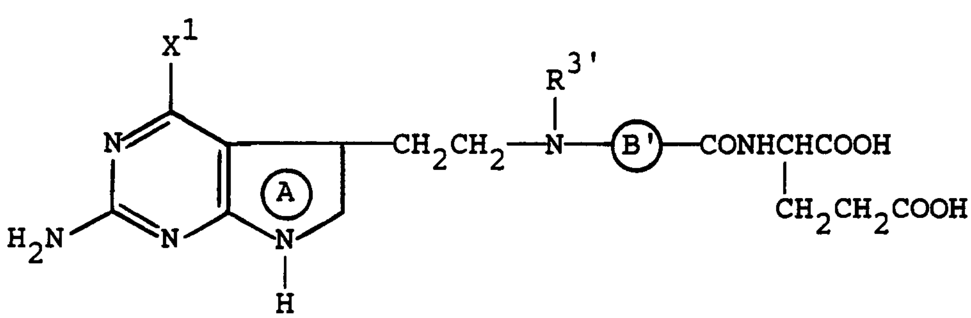

ist, worin der Ring Ⓐ ein Pyrrolring ist, der hydriert sein kann, $X^1$ eine Amino- oder Hydroxylgruppe bedeutet, $R^{3'}$ eine $C_{1-3}$-Alkylgruppe und -Ⓑ- eine Phenyl-1,4-ylen-, Thiazol-2,5-ylen- oder Thiophen-2,5-ylengruppe ist, oder deren pharmazeutisch verträglicher Ester oder deren Salz.

**4.** Eine Verbindung gemäß Anspruch 1, worin der Ring Ⓐ ein Pyrrolinring ist, X eine Aminogruppe bedeutet, Y ein Wasserstoffatom ist, der Rest $-(CH_2)_j-$ eine chemische Verbindungsstelle bedeutet, Z eine Aminogruppe oder eine durch ein $C_{1-3}$-Alkyl substituierte Aminogruppe ist, -Ⓑ- eine Phenyl-1,4-ylengruppe bedeutet und $-COOR^6$ und $-COOR^7$ eine Carboxylgruppe oder eine Ethoxycarbonylgruppe sind, oder deren Salz.

**5.** Eine Verbindung gemäß Anspruch 1, welche N-[4-[N-[2-(2,4-Diamino-6,7-dihydro-5H-pyrrolo[2,3-d]pyrimidin-5-yl)ethyl]-N-methylamino]benzoyl]-L-glutaminsäure, oder deren Diethylester oder deren pharmazeutisch geeignetes Salz ist.

**6.** Eine Verbindung der Formel VI

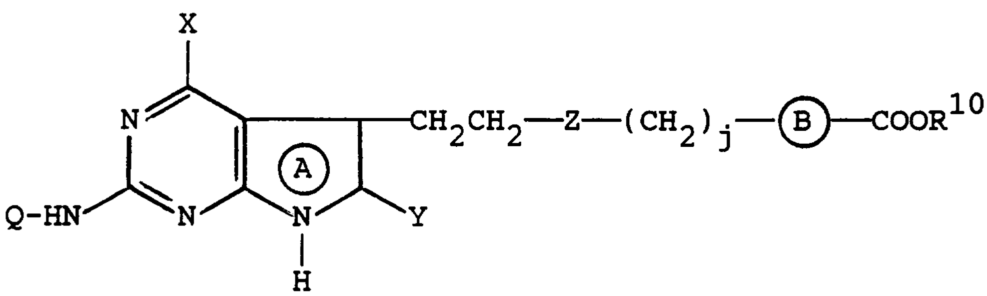

(VI)

worin jeder Rest des Rings Ⓐ, X, Y, Z, -Ⓑ- und des Index j jeweils die gleiche Bedeutung, wie in Anspruch 1 definiert, besitzt, Q ein Wasserstoffatom oder eine Amino-Schutzgruppe ist, und $-COOR^{10}$ eine Carboxylgruppe, die verestert sein kann, bedeutet, worin $R^{10}$ die gleiche Bedeutung wie $R^6$ bzw. $R^7$ hat, wie in Anspruch 1 definiert, oder deren Salz.

**7.** Ein Verfahren zur Herstellung einer Verbindung der Formel I

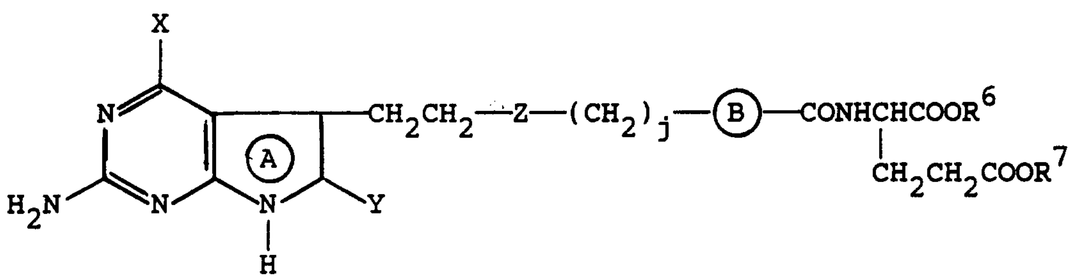

(I)

worin der Ring Ⓐ ein Pyrrolring ist, der hydriert sein kann, X eine Amino-, Hydroxyl- oder Mercaptogruppe ist, Y ein Wasserstoffatom oder eine Hydroxylgruppe bedeutet, Z -O-, -S- oder

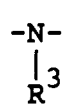

ist, worin $R^3$ ein Wasserstoffatom, eine $C_{1-3}$-Alkylgruppe oder eine $C_{2-5}$-Alkoxycarbonylgruppe bedeutet, -Ⓑ- eine Phenyl-1,4-ylen-, Thiazol-2,5-ylen- oder Thiophen-2,5-ylengruppe ist, $-COOR^6$ und $-COOR^7$ gleich oder verschieden sein können, und jeder der Reste eine Carboxylgruppe, die verestert sein kann, ist, worin $R^6$ und $R^7$ gleich oder verschieden sind, und jeweils Wasserstoff, eine Alkylgruppe mit 1 bis 5 Kohlenstoffatomen oder eine gegebenenfalls substituierte Benzyl- oder Phenylgruppe bedeuten, und der Index j den Wert 0 oder 1 aufweist, oder deren Salz,

welches das Umsetzen einer Verbindung der Formel II

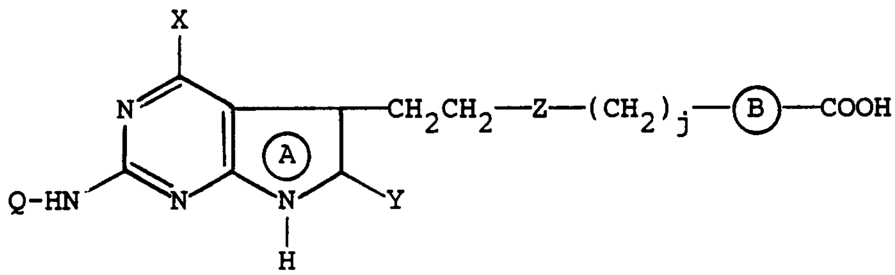

(II)

umfaßt, worin der Ring Ⓐ, X, Y, Z, -Ⓑ- und der Index j jeweils die gleiche Bedeutung wie oben besitzen, und Q ein Wasserstoffatom oder eine Amino-Schutzgruppe oder deren Salzes oder reaktiven Derivats an der Carboxylgruppe ist, mit einer Verbindung der allgemeinen Formel III

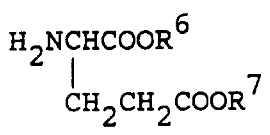

(III)

worin jeder der Reste $-COOR^6$ und $-COOR^7$ die gleiche Bedeutung wie oben besitzt, und, falls notwendig, Unterwerfen des resultierenden Produkts einer Entfernungsreaktion der Schutzgruppe.

**8.** Ein verfahren zur Herstellung einer Verbindung der Formel I

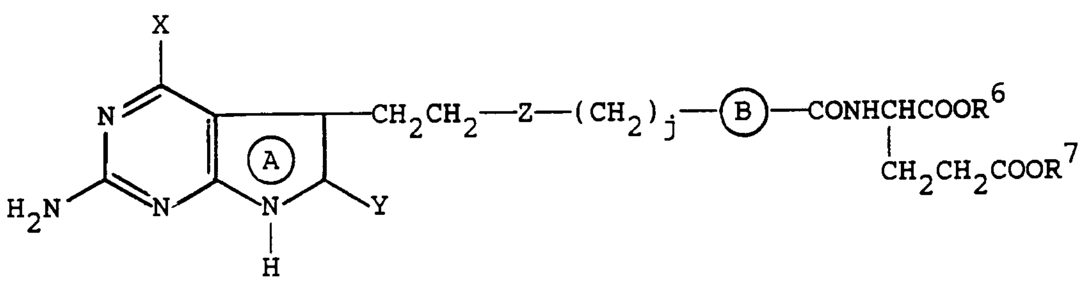

(I)

in welcher der Ring Ⓐ ein Pyrrolring ist, der hydriert sein kann, X eine Amino-, Hydroxyl- oder Mercaptogruppe bedeutet, Y ein Wasserstoffatom oder eine Hydroxylgruppe ist, Z -O-, -S- oder

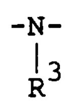

bedeutet, worin $R^3$ ein Wasserstoffatom, eine $C_{1-3}$-Alkylgruppe oder eine $C_{2-5}$-Alkoxycarbonylgruppe ist, -Ⓑ- eine Phenyl-1,4-ylen-, Thiazol-2,5-ylen- oder Thiophen-2,5-ylengruppe bedeutet, $-COOR^6$ und $-COOR^7$ gleich oder verschieden sein können, und jede der Gruppen eine Carboxylgruppe, die verestert sein kann, bedeutet, worin $R^6$ und $R^7$ gleich oder verschieden sind, und jeder Rest Wasserstoff, eine Alkylgruppe mit 1 bis 5 Kohlenstoffatomen oder eine gegebenenfalls substituierte Benzyl- oder Phenylgruppe ist, und der Index j 0 oder 1 ist, oder deren Salz, welches das Umsetzen einer Verbindung der Formel IV

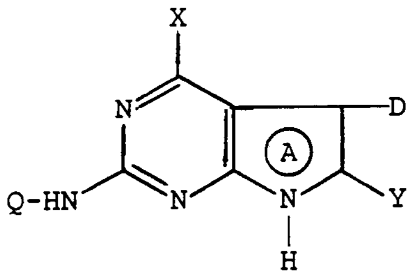

(IV)

umfaßt, worin jeder Rest des Rings Ⓐ, X und Y jeweils die gleiche Bedeutung wie oben besitzt, Q ein Wasserstoffatom oder eine Amino-Schutzgruppe ist, und D eine Gruppe der Formel $-CH_2CH_2-L$ bedeutet, in welcher L eine austretende Gruppe ist, eine Gruppe der Formel $-CH_2CH_2-ZH$, in welcher Z die gleiche Bedeutung wie oben besitzt, eine Gruppe der Formel

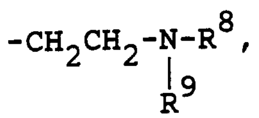

in welcher $R^8$ und $R^9$ gleich oder verschieden sein können, und jeder Rest ein Wasserstoffatom oder ein gegebenenfalls Substituent(en) aufweisender Kohlenwasserstoffrest ist, oder worin sie unter Bildung einer cyclischen Aminogruppe zusammen mit dem benachbarten Stickstoffatom kombiniert sind, oder eine Gruppe der Formel $-CH_2CHO$, oder deren Salz, mit einer Verbindung der Formel V

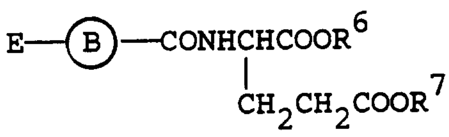

(V)

worin jeder Rest -Ⓑ-, $-COOR^6$ und $-COOR^7$ jeweils die gleiche Bedeutung wie oben besitzt, E eine Gruppe der Formel $L-(CH_2)_{m'}$-ist, in welcher L die gleiche Bedeutung wie oben aufweist, und m' 0 oder 1 ist, eine Gruppe OHC- oder eine Gruppe der Formel $HZ-(CH_2)_{m'}$-, in welcher Z und m' die gleiche Bedeutung wie oben besitzen, und anschließendes Unterwerfen des resultierenden Produkts der Entfernung der Schutzgruppe, falls erforderlich.

**9.** Eine Antitumor-Zusammensetzung, enthaltend eine wirksame Menge einer Verbindung der Formel I in Anspruch 1 oder deren pharmazeutisch verträgliches Salz, und einen pharmazeutisch verträglichen Träger oder ein Verdünnungsmittel.

**10.** Die Verwendung einer Verbindung der Formel I in Anspruch 1 oder ihres pharmazeutisch verträglichen Salzes in der Herstellung einer Antitumor-Zusammensetzung.

**Patentansprüche für folgende Vertragsstaaten : ES, GR**

1. Ein Verfahren zur Herstellung einer Verbindung der Formel I

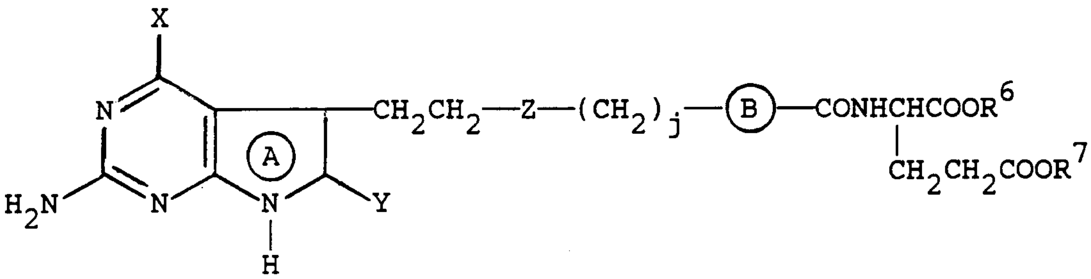

(I)

worin der Ring Ⓐ einen Pyrrolring bedeutet, der hydriert sein kann, X eine Amino-, Hydroxyl- oder Mercaptogruppe ist, Y ein Wasserstoffatom oder eine Hydroxylgruppe bedeutet, Z -O-, -S-oder

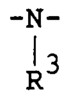

ist, worin $R^3$ ein Wasserstoffatom, eine $C_{1-3}$-Alkylgruppe oder eine $C_{2-5}$-Alkoxycarbonylgruppe bedeutet, -Ⓑ- eine Phenyl-1,4-ylen-, eine Thiazol-2,5-ylen- oder eine Thiophen-2,5-ylengruppe ist, $-COOR^6$ und $-COOR^7$ gleich oder verschieden sein können, und jeder Rest eine Carboxylgruppe ist, die verestert sein kann, worin $R^6$ und $R^7$ gleich oder verschieden sind, und jeder dieser Reste Wasserstoff, eine Alkylgruppe mit 1 bis 5 Kohlenstoffatomen oder eine gegebenenfalls substituierte Benzyl- oder Phenylgruppe bedeutet, und der Index j den Wert 0 oder 1 aufweist, oder deren Salz, welches das Umsetzen einer Verbindung der Formel II

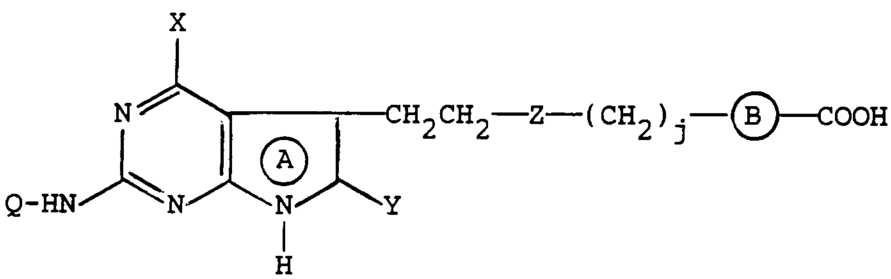

(II)

umfaßt, worin jeder Rest Ring Ⓐ, X, Y, Z, -Ⓑ- und der Index j jeweils die gleiche Bedeutung wie oben besitzt, und Q ein Wasserstoffatom oder eine Amino-Schutzgruppe oder deren Salz oder deren reaktives Derivat an der Carboxylgruppe ist, mit einer Verbindung der allgemeinen Formel III

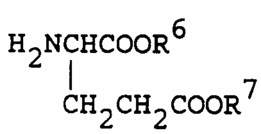

(III)

worin jeder der Reste $-COOR^6$ und $-COOR^7$ die gleiche Bedeutung wie oben besitzt, und, falls notwendig, Unterwerfen des resultierenden Produkts einer Entfernungsreaktion der Schutzgruppe.

**2.** Ein Verfahren zur Herstellung einer Verbindung der Formel I

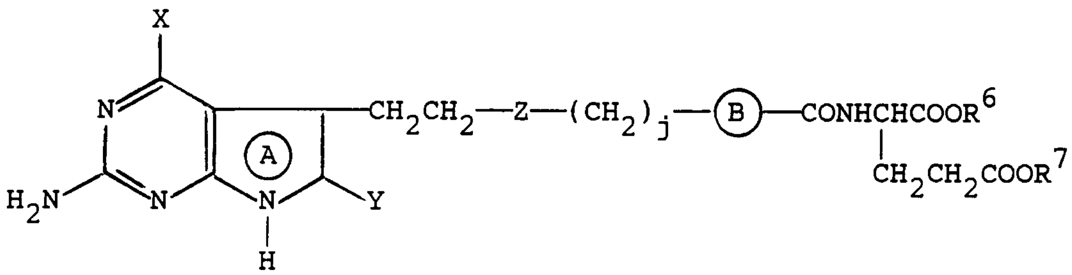

(I)

worin der Ring Ⓐ einen Pyrrolring bedeutet, der hydriert sein kann, X eine Amino-, Hydroxyl- oder Mercaptogruppe ist, Y ein Wasserstoffatom oder eine Hydroxylgruppe bedeutet, Z -O-, -S- oder

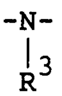

ist, worin $R^3$ ein Wasserstoffatom, eine $C_{1-3}$-Alkylgruppe oder eine $C_{2-5}$-Alkoxycarbonylgruppe bedeutet, -Ⓑ- eine Phenyl-1,4-ylen-, eine Thiazol-2,5-ylen- oder eine Thiophen-2,5-ylengruppe ist, $-COOR^6$ und $-COOR^7$ gleich oder verschieden sein können und jeder Rest eine Carboxylgruppe ist, die verestert sein kann, worin $R^6$ und $R^7$ gleich oder verschieden sind und jeder dieser Reste Wasserstoff, eine Alkylgruppe mit 1 bis 5 Kohlenstoffatomen oder eine gegebenenfalls substituierte Benzyl- oder Phenylgruppe bedeutet, und der Index j den Wert 0 oder 1 aufweist, oder deren Salz,
welches das Umsetzen einer Verbindung der Formel IV

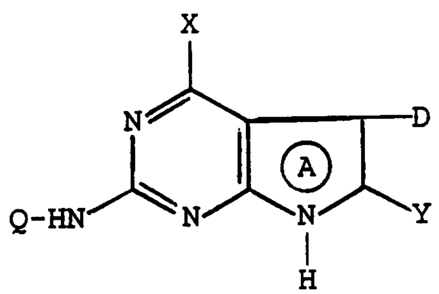

(IV)

umfaßt, worin jeder der Reste Ring Ⓐ, X und Y jeweils die gleiche Bedeutung wie oben aufweisen, Q ein Wasserstoffatom oder eine Amino-Schutzgruppe ist, und D eine Gruppe der Formel $-CH_2CH_2-L$ bedeutet, in welcher L eine austretende Gruppe ist, eine Gruppe der Formel $-CH_2CH_2-ZH$, in welcher Z die gleiche Bedeutung wie oben besitzt, eine Gruppe der Formel

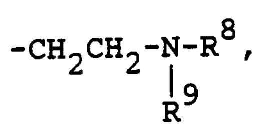

in welcher $R^8$ und $R^9$ gleich oder verschieden sein können, und jeder ein Wasserstoffatom oder ein Kohlenwasserstoffrest ist, gegebenenfalls mit Substituent(en), oder sie sind kombiniert unter Bildung einer- cyclischen Aminogruppe zusammen mit dem benachbarten Stickstoffatom, oder eine Gruppe der Formel $-CH_2CHO$, oder deren Salz, mit einer Verbindung der Formel V

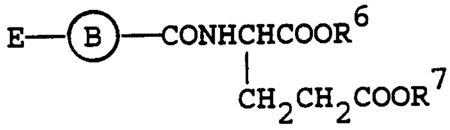

(V)

worin jeder Rest -Ⓑ-, $-COOR^6$ und $-COOR^7$ die gleiche Bedeutung wie oben besitzt, E eine Gruppe der Formel $L-(CH_2)_{m'}-$ ist, in welcher L die gleiche Bedeutung wie oben aufweist, und m' den Wert 0 oder 1 besitzt, eine Gruppe OHC- oder eine Gruppe der Formel $HZ-(CH_2)_{m'}-$, in welcher Z und m' die gleiche Bedeutung wie oben haben, und anschließend Unterwerfen des resultierenden Produkts einer Entfernungsreaktion der Schutzgruppe, falls notwendig.

3. Ein Verfahren gemäß Anspruch 1 oder Anspruch 2, worin der Rest $-(CH_2)_j-$ eine chemische Verbindungsstelle ist, Z -O-, -S-oder

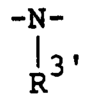

ist, worin $R^{3'}$ eine $C_{1-3}$-Alkylgruppe bedeutet, -Ⓑ-eine Phenyl-1,4-ylen- oder eine Thiophen-2,5-ylengruppe ist, und $-COOR^6$ und $-COOR^7$ eine Carboxylgruppe oder eine mit einer $C_{1-5}$-Alkylgruppe veresterte Carboxylgruppe bedeuten.

4. Ein Verfahren gemäß Anspruch 1 oder Anspruch 2 zur Herstellung einer Verbindung der nachstehenden Formel

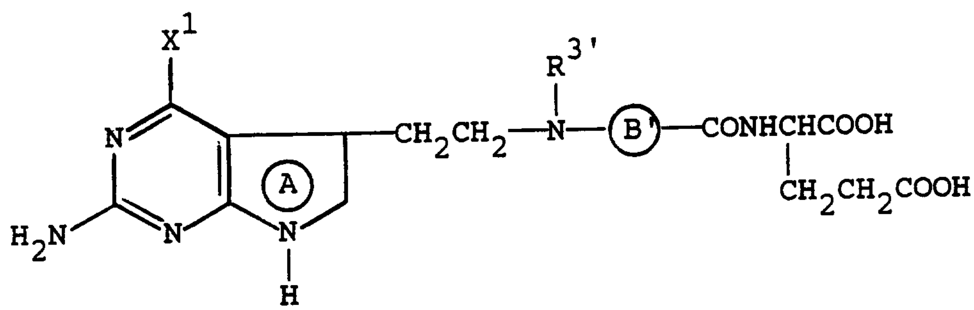

worin der Ring Ⓐ ein Pyrrolring ist, der hydriert sein kann, $X^1$ eine Amino- oder Hydroxylgruppe bedeutet, $R^{3'}$ eine $C_{1-3}$-Alkylgruppe und -Ⓑ- eine Phenyl-1,4-ylen-, eine Thiazol-2,5-ylen- oder eine Thiophen-2,5-ylengruppe ist, oder deren pharmazeutisch verträglichen Ester oder Salz.

5. Ein Verfahren gemäß Anspruch 1 oder Anspruch 2, worin der Ring Ⓐ ein Pyrrolinring ist, X eine Aminogruppe bedeutet, Y ein Wasserstoffatom ist, der Rest $-(CH_2)_j-$ eine chemische Verbindungsstelle bedeutet, Z eine Aminogruppe oder eine durch ein $C_{1-3}$-Alkyl substituierte Aminogruppe ist, -Ⓑ- eine Phenyl-1,4-ylengruppe bedeutet, und $-COOR^6$ und $-COOR^7$ eine Carboxylgruppe oder eine Ethoxycarbonylgruppe sind.

6. Ein Verfahren gemäß Anspruch 1 oder Anspruch 2 zur Herstellung von N-[4-[N-[2-(2,4-Diamino-6,7-dihydro-5H-pyrrolo[2,3-d]pyrimidin-5-yl)ethyl]-N-methylamino]benzoyl]-L-glutaminsäure, ihres Diethylesters oder eines pharmazeutisch verträglichen Salzes.

**7.** Ein Verfahren zur Herstellung einer Verbindung der Formel VI

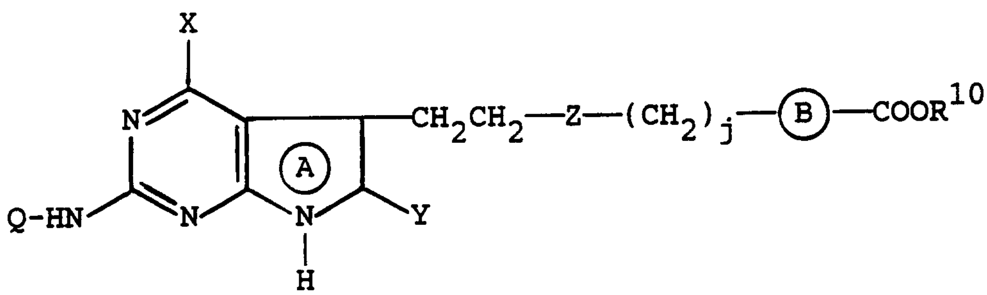

(VI)

worin jeder Rest von Ring Ⓐ, X, Y, Z, -Ⓑ- und Index j die gleiche Bedeutung wie in Anspruch 1 definiert, aufweist, Q ein Wasserstoffatom oder eine Amino-Schutzgruppe ist, und $-COOR^{10}$ eine Carboxylgruppe, die verestert sein kann, bedeutet, worin $R^{10}$ die gleiche Bedeutung wie $R^6$ bzw. $R^7$ besitzt, wie in Anspruch 1 definiert, oder deren Salz, welches umfaßt

(1) das Einführen einer entfernbaren funktionellen Gruppe L in den aktiven Methylenteil einer Verbindung der Formel VII

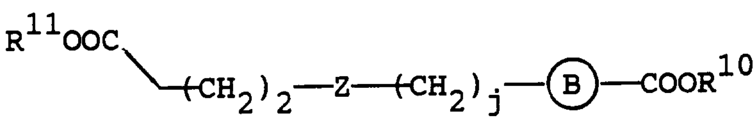

(VII)

worin jeder Rest Z, -Ⓑ-, $R^{10}$ und der Index j die gleiche Bedeutung wie oben definiert, aufweist, $-COOR^{11}$ eine Carboxylgruppe ist, die verestert sein kann, und $R^{11}$ die gleiche Bedeutung wie $R^6$ bzw. $R^7$ hat, wie in Anspruch 1 definiert, um eine Verbindung der Formel VIII

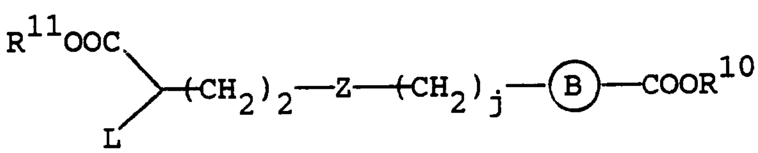

(VIII)

zu erhalten, welche

(2) unter einer basischen Bedingung der Kondensationsreaktion mit Malononitril, $NC\text{-}CH_2COOR^{13}$, $NC\text{-}CH_2CSOR^{13}$ oder $NC\text{-}CH_2CSSR^{13}$ unterworfen wird, worin $R^{13}$ Wasserstoff, eine $C_{1\text{-}5}$-Alkylgruppe, eine Benzylgruppe, gegebenenfalls Substituent(en) aufweisend oder eine Phenylgruppe, gegebenenfalls Substituent(en) aufweisend, ist, zur Lieferung einer Verbindung der Formel IX

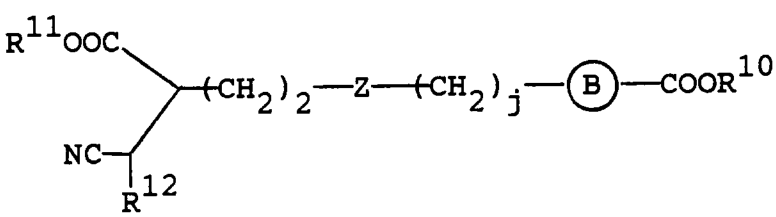

(IX)

worin $R^{12}$ Cyano oder eine Gruppe der Formel $-COOR^{13}$, $-CSOR^{13}$ oder $-CSSR^{13}$ ist, worin $R^{13}$ die gleiche Bedeutung hat, wie oben definiert, welche

(3) mit Guanidin behandelt wird, gefolgt von Ringschluß/Cyclisation, und, falls Y OH ist, gegebenenfalls gefolgt von

(4) Entesterung des Esterrestes $-COOR^{10}$ und

(5) Unterwerfen des Produkts der vorhergehenden Entesterungsstufe (4) einer Reduktion, worin die wahlweisen Stufen (4) und (5) in einer umgekehrten Reihenfolge ausgeführt sein können.

**8.** Ein Verfahren zur Herstellung einer Verbindung der Formel VI

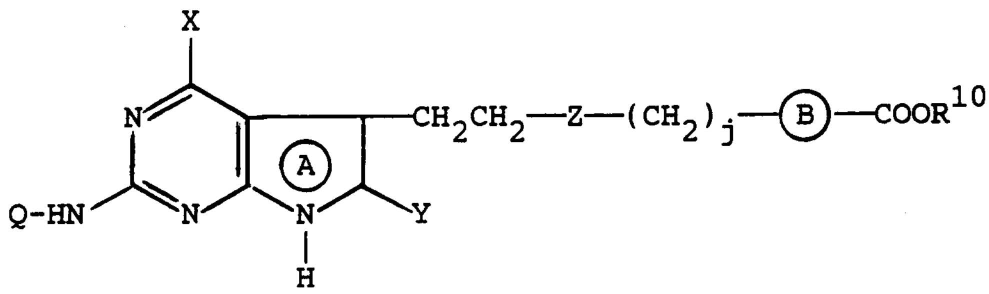

(VI)

worin jeder der Reste des Rings Ⓐ, X, Z, -Ⓑ- und der Index j die gleiche Bedeutung wie in Anspruch 1 aufweisen, Y ein Wasserstoffatom ist, Q ein Wasserstoffatom oder eine Amino-Schutzgruppe bedeutet, und $-COOR^{10}$ eine Carboxylgruppe, die verestert sein kann, ist, worin $R^{10}$ die gleiche Bedeutung wie $R^6$ bzw. $R^7$, wie in Anspruch 1 definiert, aufweist, oder deren Salz, welches umfaßt

(1) das Behandeln einer Verbindung der Formel X

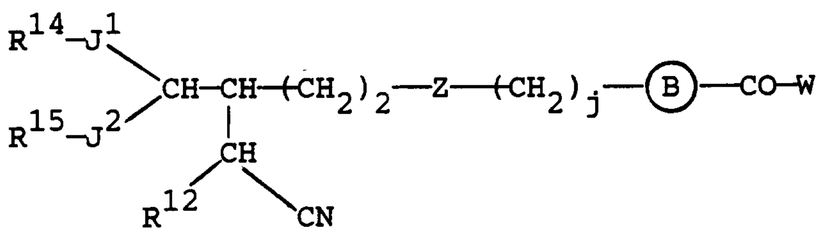

(X)

worin der Index j und -Ⓑ- die gleiche Bedeutung wie oben besitzen, $R^{12}$ Cyano oder eine Gruppe der Formel $-COOR^{13}$, $-CSOR^{13}$ oder $-CSSR^{13}$ ist, worin $R^{13}$ Wasserstoff, eine $C_{1-5}$-Alkylgruppe, eine Benzylgruppe, gegebenenfalls mit Substituent(en) oder eine Phenylgruppe, gegebenenfalls mit Substituent(en), bedeutet, W eine Gruppe der Formel $OR^{10}$ ist, worin $R^{10}$ die gleiche Bedeutung hat, wie oben definiert, $J^1$ und $J^2$ gleich oder verschieden sein können, und jeder Rest davon ein Sauerstoff- oder Schwefelatom ist, und $R^{14}$ und $R^{15}$ gleich oder verschieden sein können und ein Kohlenwasserstoffrest sind, gegebenenfalls mit Substituent(en); mit Guanidin zur Erzielung eine Verbindung der Formel XI

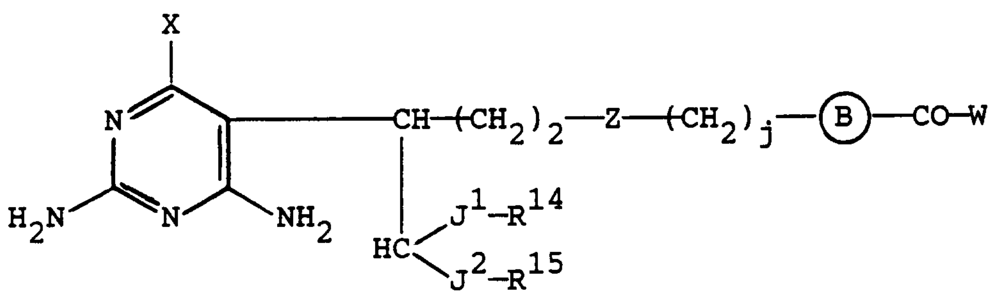

(XI)

worin X die gleiche Bedeutung, wie oben definiert, hat,
(2) Wiederherstellen der Gruppe

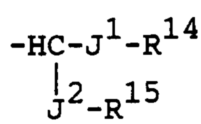

in eine Carbonylgruppe und gegebenenfalls

(3) Umwandeln des erhaltenen Pyrrolrings in einen Pyrrolinring durch eine katalytische Reduktion und gegebenenfalls

(4) Umwandeln eines Esters des Produkts von Stufe (2) oder (3) in die entsprechende Carbonsäure durch Entesterung.

**9.** Ein Verfahren zur Herstellung einer Antitumor-Zusammensetzung, umfassend das Mischen einer wirksamen Menge einer Verbindung der Formel I in Anspruch 1 oder ihres pharmazeutisch verträglichen Salzes, und eines pharmazeutisch verträglichen Trägers oder Verdünnungsmittels.

**10.** Die Verwendung einer Verbindung der Formel I in Anspruch 1 oder ihres pharmazeutisch verträglichen Salzes in der Herstellung einer Antitumor-Zusammensetzung.

**Revendications**

**Revendications pour les Etats contractants suivants : AT, BE, CH, DE, DK, FR, GB, IT, LI, LU, NL, SE**

**1.** Composé de formule (I) :

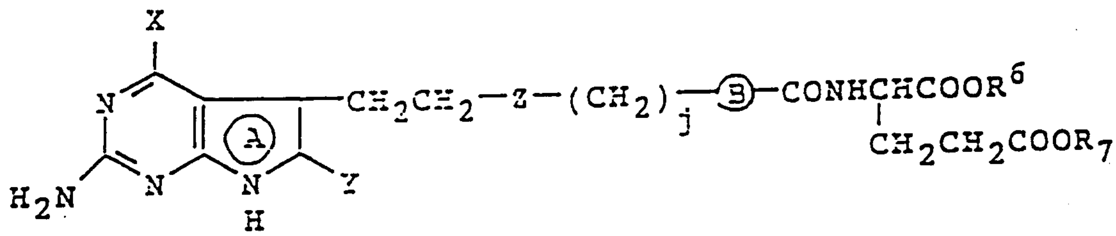

(I)

sachant que le cycle Ⓐ représente un cycle de type pyrrole qui peut être hydrogéné, X représente un groupe amino, hydroxyle ou mercapto, Y représente un atome d'hydrogène ou un groupe hydroxyle, Z représente -O-, -S- ou -N-$R^3$ dans lequel $R^3$ représente un atome d'hydrogène, un groupe alkyle en $C_{1-3}$ ou un groupe alcoxycarbonyle en $C_{2-5}$, -Ⓑ- représente un groupe phényl-1,4-ylène, thiazol-2,5-ylène ou thiophén-2,5-ylène, -$COOR^6$ et -$COOR^7$ peuvent être identiques ou différents et représentent chacun un groupe carboxyle qui peut être estérifié, dans lesquels $R^6$ et $R^7$ sont identiques ou différents et représentent chacun un atome d'hydrogène, un groupe alkyle comportant de 1 à 5 atomes de carbone ou un groupe phényle ou benzyle éventuellement substitué, et j vaut 0 ou 1, ou son sel.

**2.** Composé conforme à la revendication 1, dans lequel la partie $-(CH_2)_j-$ est une liaison chimique, Z représente -O-, -S- ou

$$-\overset{|}{N}-R^{3'}$$

dans lequel $R^{3'}$ représente un groupe alkyle en $C_{1-3}$, -Ⓑ- représente un groupe phényl-1,4-ylène ou thiophén-2,5-ylène, et -$COOR^6$ et -$COOR^7$ représentent un groupe carboxyle ou un groupe carboxyle estérifié avec un groupe alkyle en $C_{1-5}$, ou son sel.

**3.** Composé conforme à la revendication 1 qui est un composé de formule :

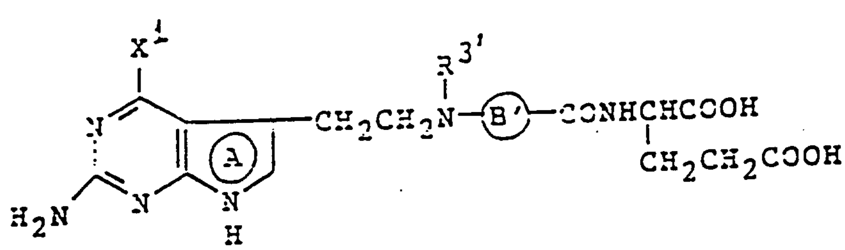

sachant que le cycle Ⓐ est un cycle de type pyrrole qui peut être hydrogéné, $X^1$ représente un groupe amino ou

hydroxyle, $R^{3'}$ représente un groupe alkyle en $C_{1-3}$ et -Ⓑ- représente un groupe phényl-1,4-ylène, thiazol-2,5-ylène ou thiophén-2,5-ylène, ou son sel ou ester pharmaceutiquement acceptable.

**4.** Composé conforme à la revendication 1, dans lequel le cycle A représente un cycle de type pyrroline, X représente un groupe amino, Y représente un atome d'hydrogène, la partie $-(CH_2)_j-$ est une liaison chimique, Z représente un groupe amino ou un groupe amino substitué par un groupe alkyle en $C_{1-3}$, - B - représente un groupe phényl-1,4-ylène, et $-COOR^6$ et $-COOR^7$ représentent un groupe carboxyle ou un groupe éthoxycarbonyle, ou son sel.

**5.** Composé conforme à la revendication 1, qui est l'acide N-[4-[N-[2-(2,4-diamino-6,7-dihydro-5H-pyrrolo[2,3-d]pyrimidin-5-yl)éthyl]-N-méthylamino]benzoyl]-L-glutamique ou son ester diéthylique ou sel pharmaceutiquement acceptable.

**6.** Composé de formule (VI) :

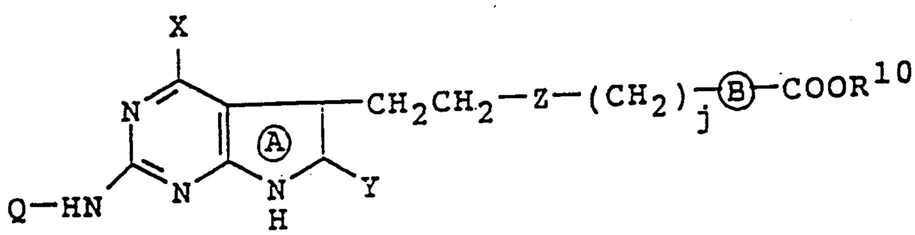

(VI)

sachant que chacun des cycle Ⓐ, X, Y, Z, -Ⓑ- et j a la même signification telle que définie dans la revendication 1, Q représente un atome d'hydrogène ou un groupe de protection de type amino, et $-COOR^{10}$ représente un groupe carboxyle qui peut être estérifié, dans lequel $R^{10}$ a la même signification que $R^6$ et $R^7$ respectivement, qui est définie dans la revendication 1, ou son sel.

**7.** Procédé qui consiste à préparer un composé de formule (I)

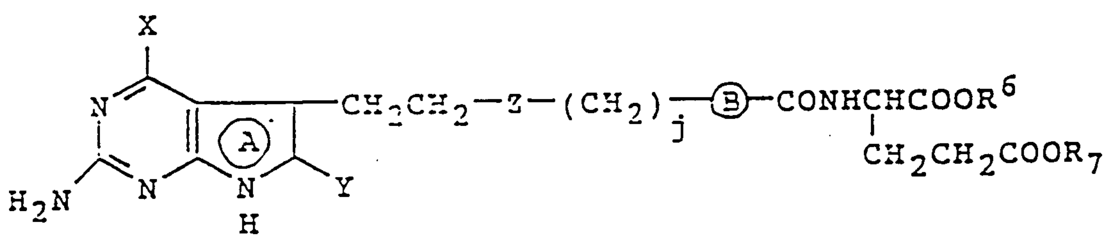

(I)

sachant que le cycle Ⓐ représente un cycle de type pyrrole qui peut être hydrogéné, X représente un groupe amino, un groupe hydroxyle ou mercapto, Y représente un atome d'hydrogène ou un groupe hydroxyle, Z représente -O-, -S- ou

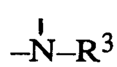

dans lequel $R^3$ représente un atome d'hydrogène, un groupe alkyle en $C_{1-3}$ ou un groupe alcoxycarbonyle en $C_{2-5}$, -Ⓑ- représente un groupe phényl-1,4-ylène, thiazol-2,5-ylène ou thiophén-2,5-ylène, $-COOR^6$ et $-COOR^7$ peuvent être identiques ou différents et représentent chacun un groupe carboxyle qui peut être estérifié, dans lequel $R^6$ et $R^7$ sont identiques ou différents et représentent chacun un atome d'hydrogène, un groupe alkyle comportant de 1 à 5 atomes de carbone ou un groupe phényle ou benzyle éventuellement substitué, et j vaut 0 ou 1, ou son sel, lequel procédé comprend le fait de faire réagir un composé de formule (II) :

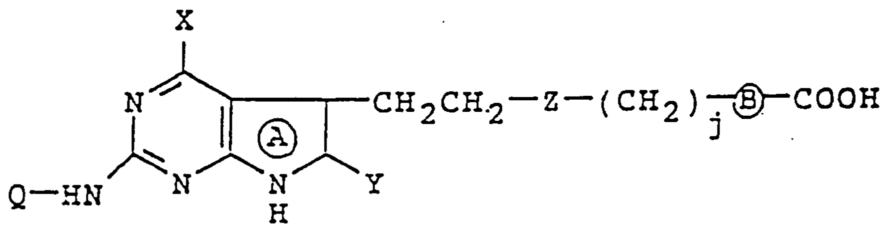 (II)

sachant que chacun des cycle Ⓐ, X, Y, Z, -Ⓑ- et j a la même signification que précédemment, Q représente un atome d'hydrogène ou un groupe de protection de type amino, ou son sel ou un dérivé activé au niveau du groupe carboxyle, avec un composé de formule générale (III) :

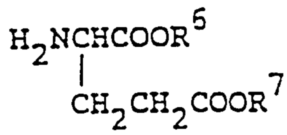 (III)

sachant que chacun des -COOR$^6$ et -COOR$^7$ a la même signification que précédemment, et si nécessaire, le fait de soumettre le produit résultant à une réaction d'élimination du groupe de protection.

**8.** Procédé qui consiste à préparer un composé de formule (I):

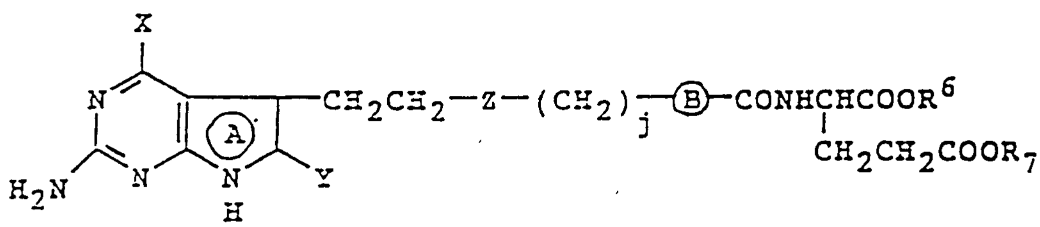 (I)

sachant que le cycle Ⓐ représente un cycle de type pyrrole qui peut être hydrogéné, X représente un groupe amino, hydroxyle ou mercapto, Y représente un atome d'hydrogène ou un groupe hydroxyle, Z représente -O-, -S- ou

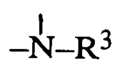

dans lequel R$^3$ représente un atome d'hydrogène, un groupe alkyle en $C_{1-3}$ ou un groupe alcoxycarbonyle en $C_{2-5}$, -Ⓑ- représente un groupe phényl-1,4-ylène, thiazol-2,5-ylène ou thiophén-2,5-ylène, -COOR$^6$ et -COOR$^7$ peuvent être identiques ou différents et représentent chacun un groupe carboxyle qui peut être estérifié, dans lequel R$^6$ et R$^7$ sont identiques ou différents et représentent chacun un atome d'hydrogène, un groupe alkyle comportant de 1 à 5 atomes de carbone ou un groupe phényle ou benzyle éventuellement substitué, et j vaut 0 ou 1, ou son sel, lequel procédé comprend le fait de faire réagir un composé de formule (IV) :

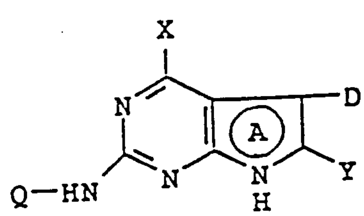 (IV)

sachant que chacun des cycle Ⓐ, X, Y, Z, -Ⓑ- et j a la même signification que précédemment, Q représente un atome d'hydrogène ou un groupe de protection de type amino, et D représente un groupe de formule $-CH_2CH_2-L$

dans laquelle L est un groupe partant, un groupe de formule $-CH_2CH_2-ZH$ dans laquelle Z a la même signification que précédemment, un groupe de formule

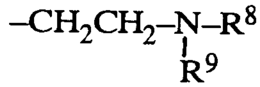

dans laquelle $R^8$ et $R^9$ peuvent être identiques ou différents et représentent chacun un atome d'hydrogène ou un radical hydrocarboné ayant éventuellement un ou des substituants ou sont combinés pour former ensemble un groupe amino cyclique avec l'atome d'azote adjacent, ou un groupe de formule (V) :

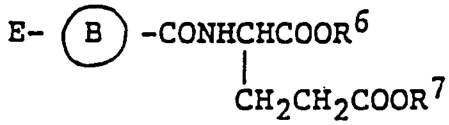

(V)

sachant que chacun des Ⓑ, $-COOR^6$ et $-COOR^7$ a la même signification que précédemment, E représente un groupe de formule $L-(CH_2)_{m'}$-dans laquelle L a la même signification que précédemment, et m' vaut 0 ou 1, un groupe de formule OHC- ou un groupe de formule $HZ-(CH_2)_{m'}$- dans laquelle Z et m' ont les mêmes significations que précédemment, et ensuite le fait de soumettre le produit résultant à une réaction d'élimination du groupe de protection, si nécessaire.

**9.** Composition antitumorale comprenant une quantité efficace d'un composé de formule (I) conforme à la revendication 1 ou son sel pharmaceutiquement acceptable, et un véhicule ou diluant pharmaceutiquement acceptable.

**10.** Utilisation d'un composé de formule (I) conforme à la revendication 1 ou son sel phamaceutiquement acceptable dans la préparation d'une composition antitumorale.

**Revendications pour les Etats contractants suivants : ES, GR**

**1.** Procédé qui consiste à préparer un composé de formule (I)

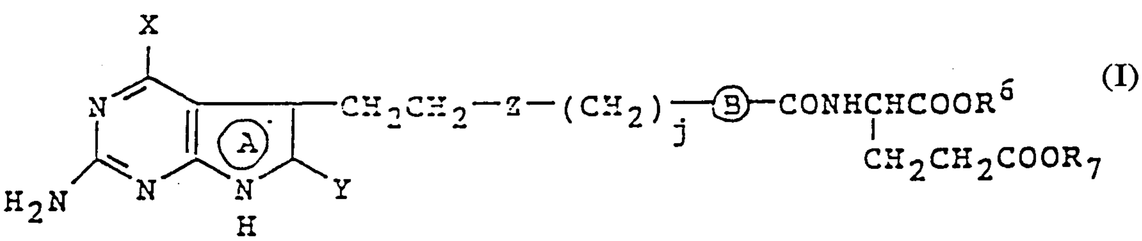

(I)

sachant que le cycle Ⓐ représente un cycle de type pyrrole qui peut être hydrogéné, X représente un groupe amino, un groupe hydroxyle ou mercapto, Y représente un atome d'hydrogène ou un groupe hydroxyle, Z représente -O-, -S- ou $-N-R^3$ dans lequel $R^3$ représente un atome d'hydrogène, un groupe alkyle en $C_{1-3}$ ou un groupe alcoxycarbonyle en $C_{2-5}$, -Ⓑ- représente un groupe phényl-1,4-ylène, thiazol-2,5-ylène ou thiophén-2,5-ylène, $-COOR^6$ et $-COOR^7$ peuvent être identiques ou différents et représentent chacun un groupe carboxyle qui peut être estérifié, dans lesquels $R^6$ et $R^7$ sont identiques ou différents et représentent chacun un atome d'hydrogène, un groupe alkyle comportant de 1 à 5 atomes de carbone ou un groupe phényle ou benzyle éventuellement substitué, et j vaut 0 ou 1, ou son sel, lequel procédé comprend le fait de faire réagir un composé de formule (II) :

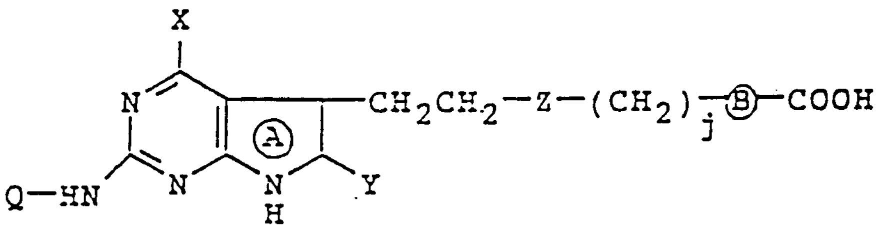

(II)

sachant que chacun des cycle Ⓐ, X, Y, Z, -Ⓑ- et j a la même signification que précédemment, Q représente un atome d'hydrogène ou un groupe de protection de type amino, ou son sel ou un dérivé activé au niveau du groupe carboxyle, avec un composé de formule générale (III) :

$$H_2NCHCOOR^6$$
$$CH_2CH_2COOR^7$$

(III)

sachant que chacun des $-COOR^6$ et $-COOR^7$ a la même signification que précédemment, et si nécessaire, le fait de soumettre le produit résultant à une réaction d'élimination du groupe de protection.

**2.** Procédé qui consiste à préparer un composé de formule (I) :

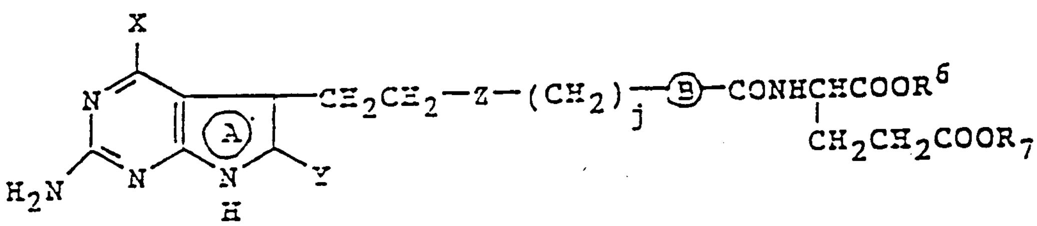

(I)

sachant que le cycle Ⓐ représente un cycle de type pyrrole qui peut être hydrogéné, X représente un groupe amino, hydroxyle ou mercapto, Y représente un atome d'hydrogène ou un groupe hydroxyle, Z représente -O-, -S- ou $-N-R^3$ dans lequel $R^3$ représente un atome d'hydrogène, un groupe alkyle en $C_{1-3}$ ou un groupe alcoxycarbonyle en $C_{2-5}$, -Ⓑ- représente un groupe phényl-1,4-ylène, thiazol-2,5-ylène ou thiophén-2,5-ylène, $-COOR^6$ et $-COOR^7$ peuvent être identiques ou différents et représentent chacun un groupe carboxyle qui peut être estérifié, dans lesquels $R^6$ et $R^7$ sont identiques ou différents et représentent chacun un atome d'hydrogène, un groupe alkyle comportant de 1 à 5 atomes de carbone ou un groupe phényle ou benzyle éventuellement substitué, et j vaut 0 ou 1, ou son sel,
lequel procédé comprend le fait de faire réagir un composé de formule (IV) :

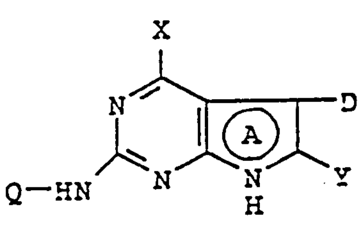

(IV)

sachant que chacun des cycle Ⓐ, X, Y, Z, -Ⓑ- et j a la même signification que précédemment, Q représente un atome d'hydrogène ou un groupe de protection de type amino, et D représente un groupe de formule $-CH_2-CH_2-L$ dans laquelle L est un groupe partant, un groupe de formule $-CH_2CH_2-ZH$ dans laquelle Z a la même signification que précédemment, un groupe de formule

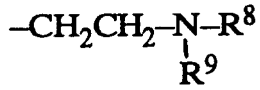

dans laquelle $R^8$ et $R^9$ peuvent être identiques ou différents et représentent chacun un atome d'hydrogène ou un radical hydrocarboné ayant éventuellement un ou des substituants ou sont combinés pour former ensemble un groupe amino cyclique avec l'atome d'azote adjacent, ou un groupe de formule (V) :

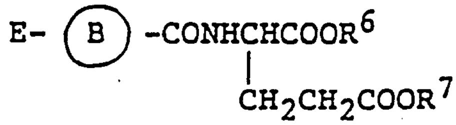

(V)

sachant que chacun des Ⓑ, $-COOR^6$ et $-COOR^7$ a la même signification que précédemment, E représente un groupe de formule $L-(CH_2)_{m'}-$ dans laquelle L a la même signification que précédemment, et m' vaut 0 ou 1, un groupe de formule OHC- ou un groupe de formule $HZ-(CH_2)_{m'}-$ dans laquelle Z et m' ont les mêmes significations que précédemment, et ensuite le fait de soumettre le produit résultant à une réaction d'élimination du groupe de protection, si nécessaire.

3. Procédé conforme à la revendication 1 ou 2, dans lequel la partie $-(CH_2)_j-$ est une liaison chimique, Z représente -O-, -S- ou $-N-R^{3'}$ dans lequel $R^{3'}$ représente un groupe alkyle en $C_{1-3}$, -Ⓑ- représente un groupe phényl-1,4-ylène ou thiophèn-2,5-ylène, et $-COOR^6$ et $-COOR^7$ représente un groupe carboxyle ou un groupe carboxyle estérifié avec un groupe alkyle en $C_{1-5}$.

4. Procédé conforme à la revendication 1 ou 2 qui consiste à préparer un composé de formule

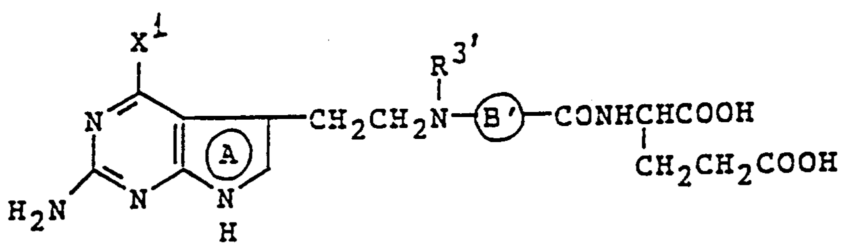

sachant que le cycle Ⓐ est un cycle de type pyrrole qui peut être hydrogéné, $X^1$ représente un groupe amino ou hydroxyle, $R^{3'}$ représente un groupe alkyle en $C_{1-3}$ et -Ⓑ'- représente un groupe phényl-1,4-ylène, thiazol-2,5-ylène ou thiophén-2,5-ylène, ou son sel ou ester pharmaceutiquement acceptable.

5. Procédé conforme à la revendication 1 ou 2, dans lequel le cycle Ⓐ représente un cycle de type pyrroline, X représente un groupe amino, Y représente un atome d'hydrogène, la partie $-(CH_2)_j-$ est une liaison chimique, Z représente un groupe amino ou un groupe amino substitué par un groupe alkyle en $C_{1-3}$, -Ⓑ- représente un groupe phényl-1,4-ylène, et $-COOR^6$ et $-COOR^7$ représentent un groupe carboxyle ou un groupe éthoxycarbonyle.

6. Procédé conforme à la revendication 1 ou 2 qui consiste à préparer l'acide N-[4-[N-[2-(2,4-diamino-6,7-dihydro-5H-pyrrolo[2,3-d]pyrimidin-5-yl)éthyl]-N-méthylamino]benzoyl]-L-glutamique ou son ester diéthylique ou sel pharmaceutiquement acceptable.

**7.** Procédé qui consiste à préparer un composé de formule (VI) :

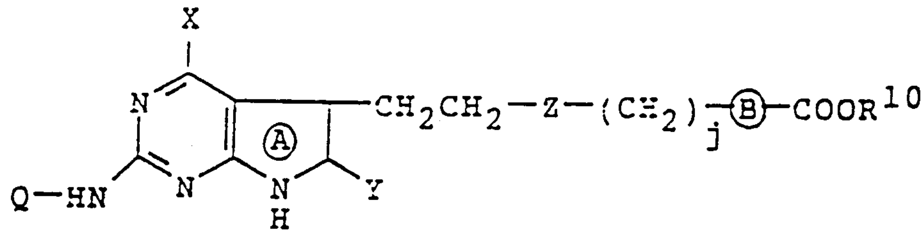

(VI)

dans laquelle chacun des cycle Ⓐ, X, Y, Z, -Ⓑ- et j a la même signification telle que définie dans la revendication 1, Q représente un atome d'hydrogène ou un groupe de protection de type amino, et $-COOR^{10}$ représente un groupe carboxyle qui peut être estérifié, dans lequel $R^{10}$ a la même signification que $R^6$ et $R^7$ respectivement, qui est définie dans la revendication 1, ou son sel, lequel procédé comprend (1) le fait d'introduire un groupe L fonctionnel, pouvant être élimineé, dans la partie méthylène active d'un composé de formule (VII) :

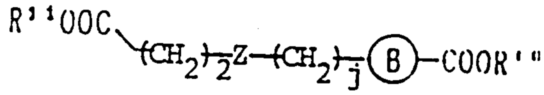

(VII)

sachant que chacun des symboles Z, Ⓑ, $R^{10}$ et j a la même signifcation que précédemment, $-COOR^{11}$ représente un groupe carboxyle qui peut être estérifié et $R^{11}$ a la même signification que $R^6$ et $R^7$, respectivement telle que définie dans la revendication 1, pour obtenir un composé de formule (VIII) :

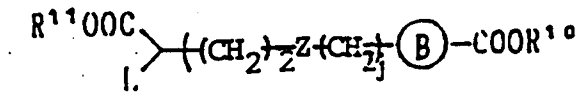

(VIII)

qui est (2) soumis dans des conditions basiques à une réaction de condensation avec un malononitrile, $NC-CH_2COOR^{13}$, $NC-CH_2CSOR^{13}$ ou $NC-CH_2CSSR^{13}$, dans lequel $R^{13}$ représente un atome d'hydrogène, un groupe alkyle en $C_{1-5}$, un groupe benzyle ayant éventuellement un ou des substituants ou un groupe phényle ayant éventuellement un ou des substituants, pour donner un composé de formule (IX) :

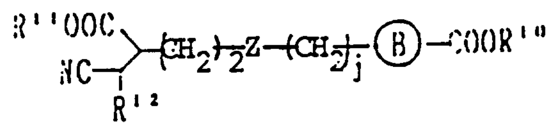

(IX)

sachant que $R^{12}$ représente un groupe cyano ou un groupe de formule $-COOR^{13}$, $-CSOR^{13}$ ou $-CSSR^{13}$, dans laquelle $R^{13}$ a la même signification que précédemment, qui est (3) traité avec de la guanidine, étape suivie par une cyclisation/fermeture de cycle, et, si Y représente OH, étape éventuellement suivie par (4) une désestérification du résidu d'ester $-COOR^{10}$, et (5) le fait de soumettre le produit de l'étape (4) précédente de désestérification à une réduction dans laquelle les étapes éventuelles (4) et (5) peuvent être effectuées dans un ordre inverse.

**8.** Procédé qui consiste à préparer un composé de formule (VI) :

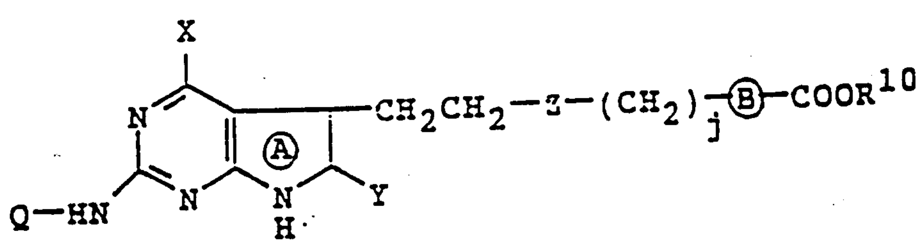

(VI)

dans laquelle chacun des cycle Ⓐ, X, Y, Z, -Ⓑ- et j a la même signification telle que définie dans la revendication 1, Y représente un atome d'hydrogène, Q représente un atome d'hydrogène ou un groupe de protection de type amino, et $-COOR^{10}$ représente un groupe carboxyle qui peut être estérifié, dans lequel $R^{10}$ a la même signification que $R^6$ et $R^7$ respectivement, qui est définie dans la revendication 1, ou son sel,
lequel procédé comprend (1) le fait de traiter un composé de formule (X) :

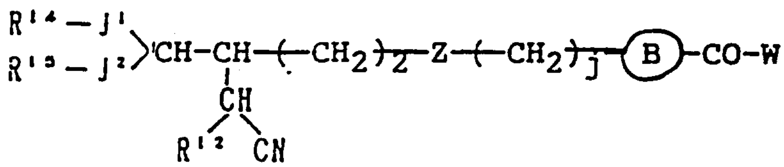

(X)

sachant que j et -Ⓑ- ont la même signification que précédemment, $R^{12}$ représente un groupe cyano ou un groupe de formule $-COOR^{13}$, $-CSOR^{13}$ ou $-CSSR^{13}$, dans laquelle $R^{13}$ représente un atome d'hydrogène, un groupe alkyle en $C_{1-5}$, un groupe benzyle ayant éventuellement un ou des substituants ou un groupe phényle ayant éventuellement un ou des substituants, W représente un groupe de formule $OR^{10}$, dans laquelle $R^{10}$ a la même signification que précédemment, $J^1$ et $J^2$ peuvent être identiques ou différents et chacun d'entre eux représente un atome d'oxygène ou un atome de soufre, et $R^{14}$ et $R^{15}$ peuvent être identiques ou différents et représentent chacun un résidu hydrocarboné ayant éventuellement un ou des substituants ; avec de la guanidine pour obtenir un composé de formule (XI) :

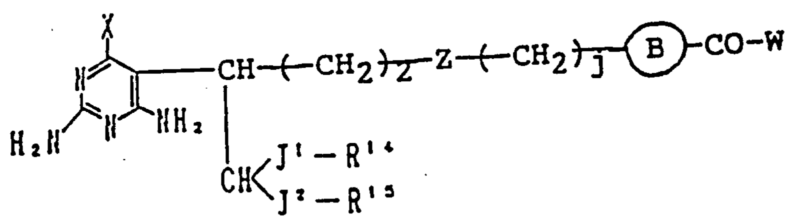

(XI)

sachant que X a la même signification que précédemment, (2) le fait de remettre le groupe

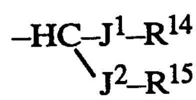

sous la forme d'un groupe carbonyle et éventuellement (3) le fait de transformer le cycle de type pyrrole obtenu en un cycle de type pyrroline à l'aide d'une réduction catalytique et éventuellement (4) le fait de transformer un ester du produit de l'étape (2) ou (3) en acide carboxylique correspondant par désestérification.

**9.** Procédé qui consiste à préparer une composition antitumorale comprenant le fait de mélanger une quantité efficace d'un composé de formule (I) de la revendication 1 ou son sel pharmaceutiquement acceptable, et un diluant ou véhicule pharmaceutiquement acceptable.

**10.** Utilisation d'un composé de formule (I) de la revendication (I) ou son sel pharmaceutiquement acceptable dans la préparation d'une composition antitumorale.